(19) Europäisches Patentamt
European Patent Office
Office européen des brevets

(11) Publication number: **0 010 941**
**B1**

(12) # EUROPEAN PATENT SPECIFICATION

(45) Date of publication of patent specification: 12.05.82

(21) Application number: 79302358.1

(22) Date of filing: 29.10.79

(51) Int. Cl.[3]: C 07 D 239/54, C 07 D 401/12, C 07 D 409/12, C 07 D 403/12, C 07 D 405/12, A 61 K 31/505

(54) **5-Fluorouracil derivatives, preparation thereof and their pharmaceutical compositions.**

(30) Priority: 30.10.78 GB 4242678
22.01.79 GB 7902195
19.03.79 GB 7909522
04.06.79 GB 7919439

(43) Date of publication of application:
14.05.80 Bulletin 80/10

(45) Publication of the grant of the patent:
12.05.82 Bulletin 82/19

(84) Designated Contracting States:
AT BE CH DE FR GB IT LU NL SE

(56) References cited:
DE - A - 2 455 423

CHEMICAL ABSTRACTS, vol. 88, no. 3, 16th January 1978, page 644, no. 22966p Columbus, Ohio, U.S.A.

(73) Proprietor: Fujisawa Pharmaceutical Co. Ltd.
3, Doshomachi 4-chome Higashi-ku
Osaka-shi, Osaka 541 (JP)

(72) Inventor: Takaya, Takao
No. 5-87, Suimeidai 1-chome
Kawanishi-shi, Hyogo 666-01 (JP)
Inventor: Tozuka, Zenzaburo
No. 16-13-121, Kamishinden 4-chome
Toyonaka-shi, Osaka 565 (JP)

(74) Representative: Shipley, Warwick Grenville Michael et al,
VENNER, SHIPLEY & CO. 368 City Road
London EC1V 2QA (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 010 941 B1

# 5-Fluorouracil derivatives, preparation thereof and their pharmaceutical compositions

This invention relates to 5-fluoroacil derivatives and to the pharmaceutically acceptable salts thereof.

More particularly, the present invention relates to 5-fluorouracil derivatives and to the pharmaceutically acceptable salts thereof which have antitumor activity, to processes for the preparation thereof and to pharmaceutical compositions comprising them.

Published Federal Republic of Germany Patent Specification No. 24 55 423 describes 5-fluorouracil derivatives of the formula:—

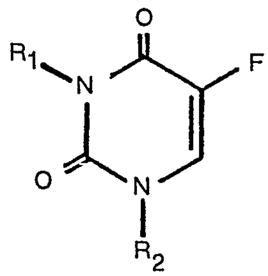

in which $R_1$ is a hydrogen atom or has the same meaning as $R_2$ and $R_2$ is an arylcarbonyl radical, a carbonyl group carrying a heterocyclic radical, an alkylsulphonyl radical, an arylsulphonyl radical, a sulphonyl group carrying a heterocyclic radical, a sulphonyl group carrying an alicyclic radical, an alkylcarbamoyl radical, an arylcarbamoyl radical or a carbamoyl group carrying an alicyclic radical. These compounds are said to possess an antitumor activity.

It is an object of this invention to provide 5-fluorouracil derivatives and pharmaceutically acceptable salts thereof which have a greatly improved antitumor activity.

Another object of the present invention is to provides processes for the preparation of 5-fluorouracil derivatives and of the pharmaceutically acceptable salts thereof.

A further object of the present invention is to provide pharmaceutical compositions comprising the 5-fluorouracil derivatives and/or the pharmaceutically acceptable salts thereof.

The 5-fluorouracil derivatives of the present invention are compounds of the formula:

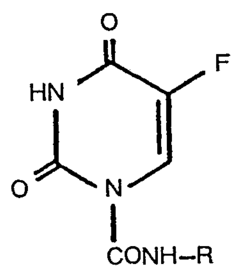

(i)

wherein R is a substituted or unsubstituted bridged alicyclic radical selected from substituted and unsubstituted norbornyl, norbornenyl, bicyclo[2.2.2]octyl, bicyclo[2.2.2]-oct-2-enyl, bicyclo-[3.1.1]heptyl and adamantyl, the substituent(s) of said bridged alicyclic radical being selected from ($C_1$—$C_6$alkyl, carboxy, esterified carboxy, ($C_1$—$C_6$)alkylidenedioxy, N,N-di-($C_1$—$C_6$)alkyl-carbamoyl, amino and protected amino; or is a substituted ($C_6$—$C_6$)alkyl radical, the substituent being selected from substituted and unsubstituted bridged alicyclic radicals selected from substituted and unsubstituted norbonyl and adamantyl, the substituent of said bridged alicyclic radical being selected from ($C_1$—$C_6$)alkyl and halogen; or is a substituted ($C_1$—$C_6$)alkyl radical, the substituent being selected from amino, protected amino, ($C_1$—$C_6$)alkoxy, acyl, ($C_1$—$C_6$)alkylthio, cyano($C_1$—$C_6$)alkylthio, phenylthio, pyridyl, thienyl, 1,2-dithiolanyl, 2,5-dioxypyrrolidinyl, carboxy and esterified carboxy, with the proviso that when the substituent of the substituted ($C_1$—$C_6$) alkyl radical is carboxy or esterified carboxy, the alkyl radical contains 3 to 6 carbon atoms, or is a trans-trans-penta-1,3-dienyl radical; or is a substituted ($C_2$—$C_6$)alkenyl radical, the substituent being selected from norbornenyl, ($C_1$—$C_6$)alkoxy substituted phenyl, furyl and thienyl; or is a tetrahydrothienyl radical; and the pharmaceutically acceptable salts thereof.

In the above and subsequent description of the invention, suitable examples and illustrations for the various definitions of the symbol R, which this invention includes within the scope thereof, are explained in detail as follows:

(1) Bridged alicyclic group which may be substituted:

Specific examples of substituents in the bridged alicyclic group include ($C_1$—$C_6$)alkyl (e.g. methyl, ethyl, propyl, isopropyl, butyl, isobutyl or tert.-butyl), in which the preferred number of carbon atoms is

up to 4; carboxy; esterified carboxy, such as ($C_1$—$C_6$)alkoxycarbonyl (e.g. methoxycarbonyl, ethoxycarbonyl, propoxycarbonyl, isopropoxycarbonyl, butoxycarbonyl, isobutoxycarbonyl or tert.-butoxycarbonyl); alkylidenedioxy (e.g. methylidenedioxy, ethylidenedioxy or propylidenedioxy); N,N-di($C_1$—$C_6$)alkylcarbamoyl (e.g. N,N-dimethylcarbamoyl, N,N-diethylcarbamoyl, N,N-dipropylcarbamoyl or N,N-diisopropylcarbamoyl); amino; protected amino; and halogen (fluorine, chlorine, bromine, iodine).

Specific examples of bridged alicyclic radicals include carboxynorbornyl, (e.g. 3-carboxy-2-norbornyl), ($C_1$—$C_6$)alkoxycarbonylnorbornyl (e.g. 3-methoxycarbonylnorborn-2-yl, 3-ethoxycarbonylnorborn-2-yl or 3-isopropoxycarbonylnorborn-2-yl), [N,N-di($C_1$—$C_6$)alkylcarbamoyl]norbornyl (e.g. (3-(N,N-diisopropylcarbamoyl)-2-norbornyl), aminonorbornyl (e.g. 3-amino-2-norbornyl), ($C_1$—$C_6$)alkoxycarbonylalkylidenedioxynorbornyl (e.g. 3-ethoxycarbonyl-5,6-isopropylidenedioxy-2-norbornyl), carboxynorbornenyl (e.g. 3-carboxy-5-norbornen-2-yl), ($C_1$—$C_6$)alkoxycarbonylnorbornenyl (e.g. 3-methoxycarbonyl-5-norbornen-2-yl, 3-ethoxycarbonyl-5-norbornen-2-yl, 3-isopropoxycarbonyl-5-norbornen-2-yl or 3-tert-.butoxycarbonyl-5-norbornen-2-yl), ($C_1$—$C_6$)alkoxycarbonylbicyclo[2.2.2]octyl (e.g. 3-ethoxycarbonylbicyclo[2.2.2]-oct-2-yl, haloadamantyl (e.g. 3-bromoadamantyl), bicyclo[3.1.1]heptyl substituted by ($C_1$—$C_6$)alkyl (e.g. pinanyl).

(2) Substituted ($C_1$—$C_6$)alkyl radical:

The alkyl moiety in the substituted ($C_1$—$C_6$)alkyl radical includes straight-chained and branched alkyl, such as methyl, ethyl, propyl, isopropyl, butyl, isobutyl, pentyl or hexyl, straight chained being preferred.

The substituents in the substituted ($C_1$—$C_6$)alkyl radicals are more particularly explained in the following:

The amino protective group in the protected amino group includes conventional N-protective groups, such as acyl, substituted or unsubstituted ar($C_1$—$C_6$)alkyl (e.g. benzyl, chlorobenzyl, methoxybenzyl, phenethyl, benzhydryl or trityl. The preferred acyl for the protective group is substituted or unsubstituted alkanoyl, such as ($C_1$—$C_6$)alkanoyl (e.g. formyl or acetyl), halo($C_1$—$C_6$)alkanoyl (e.g. chloroacetyl, trifluoroacetyl or dichloroacetyl; substituted or unsubstituted ($C_1$—$C_6$)alkoxycarbonyl, such as ($C_1$—$C_6$)alkoxycarbonyl (e.g. methoxycarbonyl or t.-butoxycarbonyl); halo($C_1$—$C_6$)alkoxycarbonyl (e.g. dichloroethoxycarbonyl or trichloroethoxycarbonyl); substituted or unsubstituted ar-($C_1$—$C_6$)alkoxycarbonyl (e.g. benzyloxycarbonyl, benzhydryloxycarbonyl, chlorobenzyloxycarbonyl, nitrobenzyloxycarbonyl or methoxybenzyloxycarbonyl).

($C_1$—$C_6$)Alkoxy includes straight-chained and branched radicals, such as methoxy, ethoxy, propoxy, isopropoxy, butoxy, isobutoxy, t.-butoxy, pentyloxy or hexyloxy, the ($C_1$—$C_4$)alkoxy radicals being preferred.

Acyl includes N-substituted carbamoyl, aliphatic acyl, acyl comprising an aromatic ring, (hereinafter referred to as aromatic acyl) and acyl comprising a heterocyclic ring (hereinafter referred to as heterocyclic acyl), preferred examples of which are as follows:

Preferred N-substituted carbamoyl includes N-monosubstituted carbamoyl and N,N-disubstituted carbamoyl, such as N-mono- and N,N-di($C_1$—$C_6$)alkylcarbamoyl (e.g. N-methylcarbamoyl, N-ethylcarbamoyl, N-propylcarbamoyl, N-isopropylcarbamoyl, N-butylcarbamoyl, N-isobutylcarbamoyl, N-pentylcarbamoyl, N,N-dimethylcarbamoyl, N,N-diethylcarbamoyl, N,N-dipropylcarbamoyl, N,N-diisopropylcarbamoyl, N,N-dibutylcarbamoyl or N,N-dipentylcarbamoyl), N-mono- and N,N-di($C_2$—$C_6$)alkenylcarbamoyl (e.g. N-allylcarbamoyl, N-(2-butenyl)-carbamoyl, N-methallylcarbamoyl, N,N-diallylcarbamoyl, N,N-(2-butenyl)-carbamoyl or N,N-dimethallylcarbamoyl; preferred aliphatic acyl radicals include ($C_1$—$C_6$)alkanoyl (e.g. formyl, acetyl, propionyl, butyryl, isobutyryl, pentanoyl or hexanoyl; preferred aromatic acyl radicals include aroyl (e.g. benzoyl, toluoyl or naphthoyl), aryloxycarbonyl (e.g. phenoxycarbonyl or naphthyloxycarbonyl) and aryloxy($C_1$—$C_6$)alkanoyl (e.g. phenoxyacetyl or phenoxypropionyl); heterocyclic acyl radicals include 3- to 7-membered heterocyclic carbonyl, in which heterocyclic ring contains 1 to 3 hetero atom(s) selected from nitrogen, oxygen and sulfur atom(s) (e.g. nicotinoyl, isonicotinoyl, thenoyl, furancarbonyl, prolyl, morpholinocarbamoyl, morpholinylcarbonyl or 4-methyl-1-piperazinocarbonyl), 3- to 7-membered heterocyclic lower alkanoyl, in which heterocyclic ring contains 1 to 4 hetero atom(s) selected from nitrogen, oxygen and sulfur atom(s) (e.g. thienylacetyl, morpholinoacetyl or tetrazylacetyl).

The alkyl moiety in the ($C_1$—$C_6$)-alkylthio and cyano($C_1$—$C_6$)-alkylthio radicals includes those illustrated hereinabove for ($C_1$—$C_6$)-alkyl in the substituted ($C_1$—$C_6$)-alkyl radical.

Particularly preferred ($C_1$—$C_6$)-alkylthio radicals include methylthio, ethylthio, propylthio, isopropylthio, butylthio, isobutylthio, *t*-butylthio, pentylthio or hexylthio, and preferred cyano-($C_1$—$C_6$)-alkylthio radicals include the corresponding cyano-($C_1$—$C_6$)-alkylthio, for example, cyanomethylthio.

Esterified carboxy includes ($C_1$—$C_6$)-alkoxycarbonyl (e.g. methoxycarbonyl, ethoxycarbonyl, propoxycarbonyl, isopropoxycarbonyl, butoxycarbonyl, *tert*-butoxycarbonyl or pentyloxycarbonyl).

(3) Substituted ($C_2$—$C_6$)-alkenyl radical:

The alkenyl moiety in the substituted alkenyl radical may be straight or branched (e.g. vinyl, allyl, isopropenyl, butenyl, isobutenyl, pentenyl or hexenyl).

The pharmaceutically acceptable salts of the compounds (I) are conventional non-toxic salts and include organic acid-addition salts (e.g. maleates, tartrates, methanesulfonates, benzenesulfonates or

toluenesulfonates), inorganic acid addition salts (e.g. hydrochlorides, hydrobromides, sulfates or phosphates,), salts with amino acids (e.g. arginine, aspartic acid, lysine or glutamic acid), inorganic base salts (e.g. alkali metal salts, such as sodium and potassium salts and alkaline earth metal salts, such as a calcium and magnesium salts), ammonium salts, salts with organic bases, for example amine salts (e.g. trimethylamine salts, triethylamine salts, ethanolamine salts, diethanolamine salts, triethanolamine salts, pyridine salts or dicyclohexylamine salts).

With regard to the stereochemistry of the new compounds (I) and of the related compounds (e.g. starting compounds or intermediate compounds) of this invention, it is to be understood that there may be one more stereoisomeric pair(s) of these compounds, such as optical and/or geometrical isomers, due to the asymmetric carbon atom(s) (e.g. of bridged alicyclic group) and/or double bond(s) (e.g. of alkenyl group) in their molecules and such stereo isomers are also included within the scope of this invention.

The processes for preparing the new compounds (I) of this invention will be explained in detail in the following:

Reacting 5-fluorouracil of the formula:—

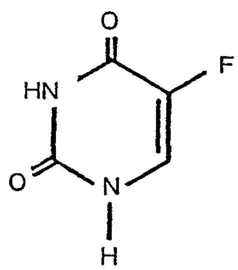

with an isocyanate of the formula:—

$$R_a–N=C=O$$

wherein $R_a$ is a substituted or unsubstituted bridged alicyclic radical selected from substituted and unsubstituted norbornyl, norbornenyl, bicyclo[2.2.2]octyl, bicyclo[2.2.2]oct-2-enyl, bicyclo[3.1.1]heptyl and adamantyl, the substituent(s) of said bridged alicyclic radical being selected from ($C_1$—$C_6$)alkyl, esterified carboxy, ($C_1$—$C_6$)alkylidenedioxy, N,N-di($C_1$—$C_{76}$)alkylcarbamoyl and protected amino; or is a substituted ($C_1$—$C_6$)alkyl radical, the substituent being selected from substituted and unsubstituted bridged alicyclic radicals selected from substituted and unsubstituted norbornyl, and adamantyl, the substituent of said bridged alicyclic radical being selected from ($C_1$—$C_6$)alkyl and halogen; or is a substituted ($C_1$—$C_6$)alkyl, the substituent being selected from protected amino, ($C_1$—$C_6$)alkoxy, acyl, ($C_1$—$C_6$alkylthio, cyano($C_1C_6$)alkylthio, phenylthio, pyridyl, thienyl, 1,1-dithiolanyl, 2,5-dioxo-pyrrolidinyl and esterified carboxy, with the proviso that, when the substituent of the substituted ($C_1$—$C_6$)alkyl radical is esterified carboxy, the alkyl radical contains 3 to 6 carbon atoms; or is trans,trans-penta-1,3-dienyl; or is a substituted ($C_2$—$C_6$)alkenyl radical, the substituent being selected from norbornenyl, ($C_1$—$C_6$alkoxy-substituted phenyl, furyl and thienyl; or is tetrahydrothienyl, to give a 5-fluorouracil derivative of the formula:—

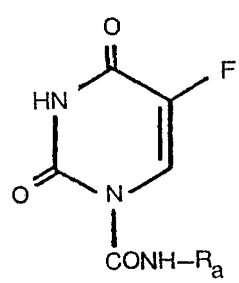

wherein $R_a$ has the same meaning as above.

This process is usually carried out in a conventional solvent, such as benzene, dimethyl sulfoxide, N,N-dimethylformamide, N,N-dimethylacetamide, tetrahydrofuran, acetonitrile, methylene chloride, ethylacetate, diethyl ether, methyl ethyl ketone, pyridine, or any other solvent which does not adversely influence to the reaction under an anhydrous condition. The reaction temperature is not critical and the reaction is preferably conducted at ambient temperature or with heating. The compounds (Ia) can be isolated from the reaction mixture and purified in a conventional manner.

This process may conveniently be carried out by adding 5-fluorouracil (II) directly to the reaction mixture containing the isocyanate (III), which can be prepared as illustrated in the following:

$R_a$—COOH (IV)

step (1) ↓ azide compound

$R_a$—$CON_3$ (VI)

step (2) ↓ Curtius rearrangement

$R_a$—N = C = O (III)

wherein $R_a$ is as defined above.

The first step of this process, i.e. the preparation of the acid azide (VI), is conventionally carried out by reacting the carboxylic acid (IV) or a reactive derivative thereof on the carboxy group with the azide compound.

Suitable reactive derivatives of the carboxylic acids (IV) include, for example, acid halides (e.g. acid chlorides, acid bromides), acid anhydrides, which include mixed acid anhydrides, such as mixed acid anhydrides with alkylcarbonic acids, which can be prepared by reacting the carboxylic acid (IV) with an alkyl halocarbonate (e.g. ethyl chlorocarbonate).

The azide compound may be hydrazoic acid or a salt thereof, such as an alkali metal salt (e.g. sodium azide, potassium azide); a phosphoryl azide of the formula (V), which will be illustrated hereinafter.

The reaction of the free form of carboxylic acid (IV) with an alkali metal salt of hydrazoic acid can be accelerated by a catalytic amount of a strong acid, such as sulfuric acid, and this first step can be preferably and conveniently carried out by reacting an acid halide or acid anhydride (preferably a mixed acid anhydride) derived from a carboxylic acid (IV) with an alkali metal salt of hydrazoic acid.

The reaction of the carboxylic acid (IV) with a phosphoryl azide of the formula (V) will be explained hereinafter.

The reaction of this step is usually carried out under hydrous conditions, i.e. in water or in an aqueous solution of water-miscible solvent, such as an alcohol (e.g. methanol or ethanol), dimethylformamide or ethyl acetate. The reaction is usually carried out with cooling or at ambient temperature.

The second step of this process, i.e. preparation of the isocyanate (III), is carried out by subjecting the acid azide (VI) to a so-called Curtius rearrangement. The Curtius rearrangement of this step is usually carried out under anhydrous conditions, with warming or heating, in a conventional solvent, such as benzene, dimethyl sulfoxide, N,N-dimethylacetamide, tetrahydrofuran, acetonitrile, methylene chloride, ethyl acetate, diethyl ether, methyl ethyl ketone, pyridine or any other solvent which does not adversely influence the reaction under anhydrous conditions.

If a phosphoryl azide of general formula (V) is used as the azide compound, the reaction is carried out under anhydrous conditions throughout the reaction so that the isocyanate (III) can be obtained by a one-pot reaction of a carboxylic acid (IV) and a phosphoryl azide (V), i.e. without isolating the intermediate of general formula (VI), as shown by the following reaction scheme and explained in detail in the following:

$$R_a\text{—COOH} + N_3\text{—}\overset{\overset{\displaystyle O}{\|}}{P}(OR_p)_2\text{(V)/Base} \rightarrow \quad \text{(IV)}$$

(warming or heating)

$$[R_a\text{—}CON_3\ \text{(VI)}] \rightarrow R_a\text{—NCO} \quad \text{(III)}$$

wherein $R_a$ is as defined above and $R_p$ is ($C_1$—$C_4$)-alkyl or aryl.

In the above definition $R_p$, the alkyl radical may be straight-chained or branched, such as methyl, ethyl, propyl, isopropyl or butyl, and preferred aryl which may be phenyl.

The isocyanate (III) is prepared by reacting an acid of the general formula (IV) with a phosphoryl azide of general formula (V) in the presence of a base.

The preferred phosphoryl azides (V) include di($C_1$—$C_6$)-alkylphosphoryl azides (e.g. dimethyl-phosphoryl azide or diethylphosphoryl azide) and diaryl-phosphoryl azides (e.g. diphenyl phosphoryl azide). These phosphoryl azides can be prepared by reacting the corresponding phosphoryl halide (e.g. dimethyl-phosphoryl chloride, diethylphosphoryl chloride or diphenylphosphoryl chloride) with sodium azide according to methods described in the literature (for example, J. Org. Chem., *27*, page 4255; and J. Amer. Chem. Soc., *94*, page 6203).

Preferred bases include organic tertiary amines, such as trialkylamines (e.g. trimethylamine, triethylamine, etc.), dialkylarylamines (e.g. N,N-dimethylaniline, etc.) and the like and nitrogen

heterocyclic compounds (e.g. pyridine, picoline, N-methylpyrrolidine, N-methylpiperidine or N-methylmorpholine).

The reaction of this step is usually carried out with warming or heating in a conventional solvent, as exemplified in the afore-mentioned second step, the reaction being carried out under anhydrous conditions.

It is to be understood that the reaction may be carried out by first preparing the acid azide of general formula (VI) and then subjecting it to a Curtius rearrangement under the described reaction conditions to give the isocyanate (III).

The isocyanate (III) produced according to the above methods can be isolated from the reaction mixture by conventional methods. In this regard, it is to be noted that the reaction mixture *per se* comprising the isocyanate (III) and, in some cases, the acid azide (VI) can also be used directly in Process 1 as it is, i.e. without isolation of the isocyanate, and if a reaction mixture *per se* comprising the acid azide (VI) is also used in the above Process 1, the acid azide may be rearranged to give the isocyanate (III) under the reaction conditions of Process 1 and the latter (III) may be reacted with 5-fluorouracil (II) to give the 5-fluorouracil derivatives (I).

Particulars of the methods for the preparation of the isocyanate (III) as explained above will be exemplified in the working Examples hereinafter.

The carboxylic acids (IV) to be used in this process include known and novel compounds.

The preparation of the known compounds are referred to in the following bibliography, ie. BEILSTEIN (4. Auflage, Gesamtregister) Sachregister für Band 17 U. 18 F—Z, page 1413—1418 (1977). Chemische Berichte, *101*, 564—573 1968, Chemical Abstracts, *65*, 16975d.

Some representative particulars of the novel acids can be prepared by the methods described in the working Examples and the other can also be prepared in a similar manner. Some of their preparations will generally be illustrated as follows.

Reference (1)

Preparation of cyano-($C_1$—$C_6$)-alkylthio-($C_1$—$C_6$)-alkanoic acids [$R_a$—COOH, wherein $R_a$ is a cyano-($C_1$—$C_6$)-alkylthio ($C_1$—$C_6$)-alkyl radical]: a cyano-($C_1$—$C_6$)-alkylthio-($C_1$—$C_6$)-alkanoic acid can be prepared by reacting a cyano-($C_1$—$C_6$)-alkyl halide [e.g. a cyano-($C_1$—$C_6$)-alkyl chloride or cyano-($C_1$—$C_6$)-alkyl bromide] with a mercapto-($C_1$—$C_6$)-alkanoic acid. This reaction is preferably carried out in the presence of a conventional alkaline catalyst. In particular, a cyanoethylthio-($C_1$—$C_6$)-alkanoic acid can be prepared by reacting acrylonitrile with a mercapto-($C_1$—$C_6$)-alkanoic acid (e.g. thioglycolic acid), as shown by the following equation:

$$NC-CH=CH_2+HS\cdot CH_2COOH \rightarrow NCCH_2CH_2SCH_2COOH$$

Reference (2)

Mono-($C_1$—$C_6$)-alkyl esters of 5-norbornene-2,3-dicarboxylic acid ($R_a$—COOH, wherein $R_a$ is ($C_1$—$C_6$)-alkoxycarbonylnorbornenyl): a mono-($C_1$—$C_6$)-alkyl ester of 5-norbornen-2,3-dicarboxylic acid can be prepared by reacting a 5-norbornen-2,3-dicarboxylic anhydride with a lower alkanol. This reaction is preferably carried out with heating. Particular reference is made to Examples Nos. 23, 40, 41 and 44.

Reference (3)

Mono-($C_1$—$C_6$)-alkyl esters of norbornane-2,3-dicarboxylic acid [$R_a$—COOH, wherein $R_a$ is ($C_1$—$C_6$) alkoxycarbonylnorbornyl]: a mono-($C_1$—$C_6$) alkyl ester of norbornane-2,3-dicarboxylic acid may be prepared by hydrogenating a mono-($C_1$—$C_6$)-alkylester of 5-norbornene-2,3-dicarboxylic acid in a conventional manner. Particular reference is made to Examples Nos. 24, 35, 36 and 37.

Reference (4)

3-[N,N-di($_1$—$C_6$)alkylcarbamoyl]-5-norbornen-2-carboxylic acids [$R_a$—COOH, wherein $R_a$ is [N,N-di($C_1$—$C_6$)alkylcarbamoyl]-norbornenyl]]: a 3-[N,N-di($C_1$—$C_6$)alkylcarbamoyl]-5-norbornene-2-carboxylic acid may be prepared by reacting 5-norbornene-2,3-dicarboxylic anhydride with a di($C_1$—$C_6$)-alkyl amine. This reaction is preferably carried out with heating. Particular reference is made to Example No. 39.

Reference (5)

3-[N,N-di($C_1$—$C_6$)alkylcarbamoyl]-5-norbornane-2-carboxylic acid [$R_a$—COOH, wherein $R_a$ is: [N,N-di($C_1$—$C_6$)alkylcarbamoyl]-norbornyl]]: a 3-[N,N-di($C_1$—$C_6$)alkylcarbamoyl]-5-norbornane-2-carboxylic acid may be prepared by hydrogenating a 3-[N,N-di($C_1$—$C_6$)-alkylcarbamoyl]-5-norbornen-2-carboxylic acid in a conventional manner. Particular reference is made to Example No. 39.

Reference (6)

3-($C_1$—$C_6$)alkoxycarbonylbicyclo[2.2.2]-octene-2-carboxylic acids [$R_a$—COOH, wherein $R_a$ is ($C_1$—$C_6$)-alkoxycarbonylbicyclo[2.2.2]-octenyl]: a 3-$C_1$—$C_6$)alkoxycarbonylbicyclo[2.2.2]octene-2-carboxylic acid may be prepared from D,L-5-bicyclo-[2.2.2]octene-2,3-dicarboxylic acid anhydride by substantially the same method as that used for preparing 3-($C_1$—$C_6$)-alkoxycarbonylnorbornene-2,3-dicarboxylic acids. Particular reference is made to Example No. 49.

Reference (7)

3-($C_1$—$C_6$)alkoxycarbonylbicyclo-[2.2.2]octane-2-carboxylic acids [$R_a$—COOH, wherein $R_a$ is ($C_1$—$C_6$)-akloxycarbonylbicyclo[2.2.2]-octyl]: a 3-($C_1$—$C_6$)-alkoxycarbonylbicyclo[2.2.2]octane-2-carboxylic acid may be prepared from a 3-($C_1$—$C_6$)-alkoxycarbonylbicyclo[2.2.2]octene-2-carboxylic acid by substantially the same method as is used for preparing D,L-3-($C_1$—$C_6$)alkoxycarbonyl-norbornane-2-carboxylic acid. Particular reference is made to Example No. 49.

Hydrolysing a compound of the formula:

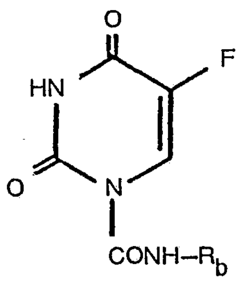

wherein $R_b$ is a bridged alicyclic radical containing a protected amino group, the bridged alicyclic radical being selected from norbornyl, norbornenyl, bicyclo[2.2.2]octyl, bicyclo[2.2.2]oct-2-enyl, bicyclo[3.1.1]heptyl and adamantyl; or is a substituted ($C_1$—$C_6$)alkyl radical, the substituent being a protected amino group; to give a 5-fluorouracil derivative of the formula:—

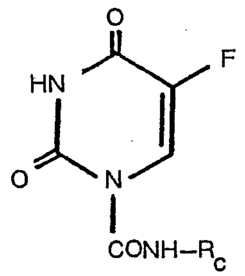

wherein $R_c$ is a bridged alicyclic radical containing an amino group, the bridged alicyclic radical being selected from norbornyl, norbornenyl, bicyclo[2.2.2]octyl, bicyclo[2.2.2]oct-2-enyl, bicyclo[3.1.1]heptyl and adamantyl; or is a substituted ($C_1$—$C_6$)alkyl radical, the substituent being an amino group.

Regarding the definitions of $R_b$ and $R_c$, the particulars thereof are the same as the corresponding particulars explained and illustrated hereinabove for the corresponding definitions of R and are to be referred to. Particulars of the protected amino group in the definition of $R_b$ are the same as those of the protected amino group explained hereinabove for the substituents of both of the bridged alicylic radical and the substituted alkyl radical R.

The hydrolysis reaction of this process may be carried out in a conventional manner, preferably under acidic conditions.

Examples of acids which can be used for the acidic hydrolysis include organic or inorganic acids, such as formic acid, trifluoroacetic acid, benzenesulfonic acid, *p*-toluenesulfonic acid, hydrochloric acid, hydrobromic acid, sulfuric acid and acidic cation exchange resins. The acid is preferably one which can easily be removed from the reaction mixture in a conventional manner, such as neutralization or distillation under reduced pressure, for example, formic acid, trifluoroacetic acid or hydrochloric acid. The appropriate acid for the reaction can be selected having regard to the chemical properties of the starting compound and of the product, as well as the nature of the amino protective group to be eliminated. The acidic hydrolysis can be carried out in the presence or absence of a solvent.

The solvent used may be a conventional organic solvent which does not adversely influence this reaction, water or a mixture thereof. Particularly when the hydrolysis is carried out with trifluoroacetic acid, the reaction may be accelerated by the addition of anisole.

The reaction temperature is not critical but is preferably selected having regard to the chemical properties of the compound (Ib) and the product (Ic), as well as the nature of the amino protective group and the method employed, the reaction preferably being carried out under mild conditions, such as with cooling, at ambient temperature or at a slightly elevated temperature.

Process 3

Hydrolysing a compound of the formula:—

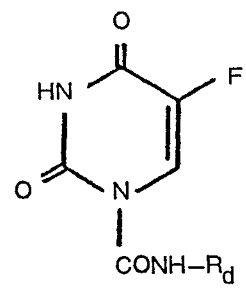

wherein $R_d$ is a bridged alicyclic radical containing an esterified carboxy group, the bridged alicyclic radical being selected from norbornyl, norbornenyl, bicyclo[2.2.2]octyl, bicyclo[2.2.2]oct-2-enyl, bicyclo[3.1.1]heptyl and adamantyl; or is a substituted ($C_1$—$C_6$)alkyl radical, the substituent being an esterified carboxy group, to give a 5-fluorouracil derivative of the formula:—

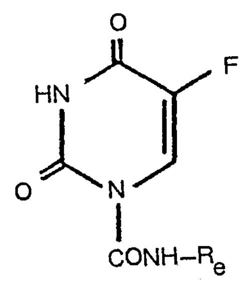

wherein $R_e$ is a bridged alicyclic radical containing a carboxy group, the bridge alicyclic radical being selected from norbornyl, norbornenyl, bicyclo[2.2.2]octyl, bicyclo[2.2.2]oct-2-enyl, bicyclo[3.1.1]heptyl and adamantyl; or is a substituted ($C_1$—$C_6$)alkyl radical, the substituent being a carboxy group.

Regarding the definitions of $R_d$ and $R_e$, the particulars thereof are the same as the corresponding particulars explained and illustrated hereinabove for the corresponding definitions of R and are to be referred to. Particulars of the esterified carboxy group in the definitions for $R_d$ are the same as those of the esterified carboxy group explained hereinabove for the substituents of both the bridged alicyclic radical and the substituted alkyl radical R.

The hydrolysis of this process may be conducted by a conventional acidic hydrolysis.

Examples of acids to be used in this acidic hydrolysis include organic or inorganic acids, such as formic acid, trifluoroacetic acid, benzenesulfonic acid, *p*-toluenesulfonic acid, hydrochloric acid or cation exchange resins. The acid is preferably one which can easily be removed from the reaction product in a conventional manner, such as neutralization or distillation under reduced pressure, for example, formic acid, trifluoroacetic acid or hydrochloric acid. The appropriate acid for the reaction can be selected having regard to the chemical properties of the starting compound and of the product, as well as the nature of the alkoxycarbonyl group to be hydrolyzed. The acidic hydrolysis can be conducted in the presence or absence of a solvent.

The solvent used may be a conventional organic solvent which does not adversely influence this reaction, water or a mixture thereof. Particularly when the hydrolysis is carried out with trifluoroacetic acid, the reaction may be accelerated by the addition of anisole.

The reaction temperature is not critical but is preferably selected having regard to the chemical properties of the compound (Id) and the product (Ie), as well as the nature of alkoxycarbonyl group and the method employed, the reaction preferably being carried out under mild conditions, such as with cooling, at ambient temperature or at a slight elevated temperature.

The object compounds (I) of this invention and pharmaceutically acceptable salts thereof exhibit strong antitumor activity, and are useful as an antitumor agent for therapeutical treatment in human being and animals.

For therapeutical administration, the 5-fluorouracil derivatives (I) or their pharmaceutically acceptable salts of this invention may be used in the form of pharmaceutical preparation which comprises said compound, as an active ingredient, in admixture with a pharmaceutically acceptable carrier such as an organic or inorganic solid or liquid excipient suitable for the oral or parentical administration. If desired, there may be included auxiliary substances, stabilizing agents, wetting or emulsifying agents, buffers and other commonly used additives in addition to the above exemplified

excipient. The pharmaceutical preparation may be prepared in solid form as capsule, tablet, dragee, granule or suppository, or in liquid form such as solution, suspension or emulsion.

While the dosage of the compound of this invention may vary depending upon the age and/or the conditions of a patient, an average single dose of about 100 mg., 200 mg., 400 mg. and 1600 mg. of the compounds according to this invention may be effective for treating of tumors in human being and animals. In general amounts between about 200 mg. and about 4,000 mg. or even more may be administered to the patient per day.

Test data on antitumor activity of some representative compounds of this invention are shown below in order to show the utility of this invention.

Test method:

Male and female $BDF_1$ mice, aged more than 6 weeks, weighing more than 18 g. in male and more than 17 g. in female, were used in this experiment. Lymphocytic Leukemia (P388 or L 1210 was transferred every 6 or 7 days in DBA/2 mice by intraperitoneal inoculation of ascites cells. Test compounds were dissolved in aqueous methyl cellulose solution. In this experiment, after 24 hours of the inoculation of leukemia cells to the test mice, the test compound was administered orally in doses of 10, 32, 100, 180, 320 and 560 mg./kg. respectively in each medicated group (aqueous methyl cellulose only in the control group) once a day for 4 days. Antitumor activity of the test compounds was evaluated by the increase in life-span over control (ILS = T/C × 100—100) in leukemias, wherein T is median survival time (MST) of the medicated group, and C is median survival time of control group.

Test result:

The test results are shown in the following tables (Fig. 1—Fig. 3).

The data shows that objective compounds have strong anti-tumor activity and little or no toxic effect.

FIG. 1
Antitumor activity of test compounds against Lymphocytic Leukemia P388 by oral administration

| Test compounds (No. of Examples) | No. of mice | Daily dose (mg./kg.) | Toxic death | MST (days) | ILS (%) | Weight change (g.) day 1 to day 4 |
|---|---|---|---|---|---|---|
| 11 | 7 | 180 | 0 | 16.0 | 46 | –0.2 |
| 17 | 7 | 180 | 0 | 14.0 | 27 | –0.6 |
| 26 | 7 | 180 | 0 | 15.0 | 36 | –1.0 |
| Control | 16 | 0 | 0 | 11.0 | — | +1.2 |
| 22 | 8 | 180 | 0 | 12.5 | 32 | –0.3 |
| 24 | 8 | 180 | 0 | 16.0 | 68 | +0.3 |
| Control | 10 | 0 | 0 | 9.5 | — | +2.2 |
| 29 | 10 | 100 | 0 | 15.0 | 50 | –0.2 |
| 30 | 10 | 100 | 0 | 14.0 | 40 | 0 |
| 33 | 10 | 100 | 0 | 13.0 | 30 | –0.4 |
| 35 | 10 | 100 | 0 | 14.0 | 40 | +0.2 |
| Control | 10 | 0 | 0 | 10.0 | — | +1.3 |

FIG. 2

Antitumor activity of test compounds against Lymphocytic Leukemia P388 and Lymphocytic Leukemia L 1210 by oral administration

| Test Compounds (No. of examples) | Daily dose (mg./kg.) | P388 | | L 1210 | |
|---|---|---|---|---|---|
| | | ILS (%) | Index* | ILS (%) | Index* |
| 1 | 560 | 20 | + | 47 | + |
| | 320 | 40 | + | 47 | + |
| | | 35 | + | | |
| | | 10 | – | | |
| | 180 | 50 | ++ | | |
| | | 35 | + | | |
| | 100 | 50 | ++ | 14 | – |
| | | 40 | + | | |
| | | 30 | + | | |
| | 32 | 20 | + | | |
| | 10 | 10 | – | | |
| 2 | 560 | 10 | – | | |
| | 320 | 25 | + | 89 | ++ |
| | | 10 | – | | |
| | 180 | 44 | + | | |
| | | 25 | + | | |
| | 100 | 55 | ++ | 26 | + |
| | | 20 | + | | |
| | 32 | 20 | + | 5 | – |
| | 10 | 10 | – | | |
| 15 | 560 | 0 | – | 31 | + |
| | 320 | 40 | + | 17 | – |
| | | 40 | + | | |
| | 180 | 33 | + | | |
| | | 25 | + | | |
| | 100 | 10 | – | 1 | – |
| | | 10 | – | | |

FIG. 2 — Continued
Antitumor activity of test compounds against Lymphocytic Leukemia P388 and Lymphocytic Leukemia L 1210 by oral administration

| Test Compounds (No. of examples) | Daily dose (mg./kg.) | P388 | | L 1210 | |
|---|---|---|---|---|---|
| | | ILS (%) | Index* | ILS (%) | Index* |
| | 560 | 0 | – | | |
| | 320 | 10 | – | 60 | ++ |
| | 180 | 68 | ++ | | |
| 24 | 100 | 40 | + | 17 | – |
| | 32 | 15 | – | 2 | – |
| | 10 | 10 | – | | |

* Index <20 –, ≧20 +, ≧50 ++

FIG. 3

Antitumor activity of test compounds against Lymphocytic Leukemia P388 by oral administration

| Test compounds (No. of Examples) | Daily dose (mg.kg.) | ILS (%) | Weight change (g.) day 1 to day 4 |
|---|---|---|---|
| 3 | 180 | 44 | –0.1 |
| 34 | 100 | 27 | +0.8 |
| 36 | 100 | 33 | –0.5 |
| 37 | 100 | 23 | +0.8 |
| 40 | 100 | 32 | +0.6 |
| 41 | 100 | 52 | –0.7 |
| 42 | 100 | 24 | –0.4 |
| 43 | 100 | 27 | +0.6 |
| 44 | 100 | 48 | –0.8 |
| 45 | 100 | 38 | –0.5 |
| 46 | 100 | 27 | –1.6 |
| 49 | 100 | 36 | –1.3 |

The following Examples are given for the purpose of illustrating the present invention in more detail.

Example 1

1-[N-(1-Adamantylmethyl)carbamoyl]-5-fluorouracil

A solution of diphenylphosphoryl azide [$N_3 \cdot PO(OC_6H_5)_2$] (5.50 g) in dry benzene (10 ml) and a solution of triethylamine (2.02 g) in dry benzene (10 ml) were added to a solution of 2-(1-

adamantyl)acetic acid (3.88 g) in dry benzene (10 ml) with stirring at ambient temperature. The clear solution was stirred for 30 minutes at 80°C to give a solution comprising 1-adamantylmethyl isocyanate (IR: 2270 $cm^{-1}$).

To the above solution of 1-adamantylmethyl isocyanate was added a solution of 5-fluorouracil (2.60 g) in N,N-dimethylacetamide (20 ml) at 80°C and stirred for further 3 hours at the same temperature. The resultant mixture was diluted with ethyl acetate (200 ml), washed with water (each 30 ml., 3 times), dried over magnesium sulfate, treated with activated charcoal, filtered and evaporated in vacuo. The residue was recrystallized from diethyl ether to give 1-[N-(1-adamantylmethyl)carbamoyl]-5-fluorouracil (1.85 g), as colorless crystalline powder.

m.p. 175—176°C.

N.M.R. ($CDCl_3$) (ppm):

1.40 to 1.80 (12H, m), 1.80 to 2.2 (3H, m), 3.67 (2H, d, J=6Hz), 8.47 (1H, d, J=7Hz), 9.0 to 9.4 (1H, broad), 9.5 to 9.9 (1H, broad).

## Example 2

1-[N-(2-Norbornylmethyl)carbamoyl]-5-fluorouracil

The reaction of 2-norbornylacetic acid and diphenylphosphoryl azide in a solution of triethylamine and benzene provided a solution comprising 2-norbornylmethyl isocyanate (I.R.: 2270 $cm^{-1}$), which was reacted with 5-fluorouracil to provide 1-[N-(2-norbornylmethyl)carbamoyl]-5-fluorouracil substantially in the similar method to that of Example 1.

m.p. 165—165.5°C (recrystallized from ethyl acetate).

N.M.R. (DMSO-$d_6$) (ppm):

0.83 to 1.90 (8H, m), 2.0 to 2.30 (2H, m), 2.9 to 3.50 (3H, m), 8.37 (1H, d, J=8Hz), 9.18 (1H, t, J=6Hz), 11.4 (1H, broad).

## Example 3

1-[N-(2-Ethoxyethyl)carbamoyl]-5-fluorouracil

A solution of diphenylphosphoryl azide [$N_3 \cdot PO(OC_6H_5)_2$] (5.50 g.) in dry benzene (10 ml.) and a solution of triethylamine (2.02 g.) in dry benzene (10 ml.) were added to a solution of 3-ethoxypropionic acid (2.36 g.) in dry benzene (10 ml.) with stirring at ambient temperature. The clear solution was stirred for 30 minutes at 80°C to give a solution comprising 2-ethoxyethyl isocyanate.

To the above solution of 2-ethoxyethyl isocyanate was added a solution of 5-fluorouracil (2.60 g.) in N,N-dimethylacetamide (20 ml.) at 80°C and stirred for further 3 hours at the same temperature. The resultant mixture was diluted with ethyl acetate (200 ml.), washed with saturated sodium chloride aqueous solution (each 30 ml., 2 times), dried over magnesium sulfate, treated with activated charcoal, filtered and evaporated in vacuo. The residue was recrystallized from ethyl acetate to give 1-[N-(2-ethoxyethyl)carbamoyl]-5-fluorouracil (1.82 g.).

m.p. 134—135°C (recrystallized from ethyl acetate).

N.M.R. ($CDCl_3$) (ppm):

1.23 (3H, t, J=7Hz), 3.40 to 3.87 (6H, m), 8.45 (1H, d, J=7Hz), 9.27 (1H, broad), 9.63 (1H, broad).

## Example 4

1-[N-(2-acetylethyl)carbamoyl]-5-fluorouracil

A solution of diphenylphosphoryl azide [$N_3 \cdot PO(OC_6H_5)_2$] (5.25 g.) in dry benzene (10 ml.) and a solution of triethylamine (2.0 g.) in dry benzene (10 ml.) were added to a solution of 3-acetylpropionic acid (2.32 g.) in dry benzene (10 ml.) with stirring at ambient temperature. The clear solution was stirred for 30 minutes at 50°C to give a solution comprising 2-acetylethyl isocyanate.

To the above solution of 2-acetylethyl isocyanate was added a solution of 5-fluorouracil (2.60 g.) in N,N-dimethylacetamide (15 ml.) at 80°C and stirred for further 5 hours at the same temperature. The resultant mixture was diluted with ethyl acetate (200 ml.), washed with water (each 30 ml., 3 times), dried over magnesium sulfate, treated with activated charcoal, filtered and evaporated in vacuo. The residue was recrystallized from ethyl acetate to give 1-[N-(2-acetylethyl)carbamoyl]-5-fluorouracil (439 mg.).

m.p. 158—159°C (recrystallized from ethyl acetate).

N.M.R. (DMSO-$d_6$) (ppm):

2.13 (3H, s), 2.73 (2H, t, J=6Hz), 3.45 (2H, q, J=6Hz), 8.37 (1H, d, J=8Hz), 9.2 (1H, t, J=6Hz), 12.0 to 12.5 (1H, broad).

## Example 5

1-[N-[2-(N′,N′-Diethylcarbamoyl)ethyl]carbamoyl]-5-fluorouracil

The reaction of 3-(N,N-diethylcarbamoyl)propionic acid and diphenylphosphoryl azide in a solution of triethylamine and benzene provided a solution comprising 2-(N′,N′-diethylcarbamoyl)ethyl isocyanate (I.R.: 2290 $cm^{-1}$), which was reacted with 5-fluorouracil to provide 1-[N-[2-(N′,N′-

diethylcarbamoyl)ethyl]carbamoyl]-5-fluorouracil substantially in the similar method to that of Example 4.

m.p. 146—147°C (crystallized from ethyl acetate).

N.M.R. ($DMSO-d_6$) (ppm):

0.87 to 1.30 (6H, m), 2.5 to 2.8 (2H, m), 3.06 to 3.76 (6H, m), 8.43 (1H, d, J=8Hz), 9.37 (1H, t, J=6Hz)

Example 6

1-[N-[2-(N',N'-Dipropylcarbamoyl)ethyl]carbamoyl]-5-fluorouracil

The reaction of 3-(N,N-dipropylcarbamoyl)propionic acid and diphenylphosphoryl azide in a solution of triethylamine and benzene provided a solution comprising 2-(N',N'-dipropylcarbamoyl)ethyl isocyanate (I.R.: 2280 $cm^{-1}$), which was reacted with 5-fluorouracil to provide 1-[N-[2-(N',N'-dipropylcarbamoyl)ethyl]carbamoyl]-5-fluorouracil substantially in the similar method to that of Example 4.

m.p. 175—176°C (recrystallized from ethyl acetate).

N.M.R. ($DMSO-d_6$) (ppm):

0.67 to 1.10 (6H, m), 1.2 to 1.9 (2H, m), 3.0 to 3.83 (6H, m), 8.37 (1H, d, J=8Hz), 9.33 (1H, t, J=6Hz).

Example 7

1-[N-[3-(N',N'-Dipropylcarbamoyl)propyl]carbamoyl]-5-fluorouracil

The reaction of 4-(N,N-dipropylcarbamoyl)butylic acid and diphenylphosphoryl azide in a solution of triethylamine and benzene provided a solution comprising 3-(N',N'-dipropylcarbamoyl)propyl isocyanate, was reacted with 5-fluorouracil to provide 1-[N-[3-(N',N'-dipropylcarbamoyl)propyl]carbamoyl]-5-fluorouracil substantially in the similar method to that of Example 4.

m.p. 146—146.5°C (recrystallized from ethyl acetate).

N.M.R. ($DMSO-d_6$) (ppm):

0.6 to 1.0 (6H, m), 1.1 to 2.0 (6H, m), 2.25 (2H, d), 2.95 to 3.5 (6H, m), 8.67 (1H, d, J=8Hz), 9.07 (1H, t, J=6Hz), 12.25 (1H, s).

Example 8

1-[N-[2-(N',N'-Diallylcarbamoyl)ethyl]carbamoyl]-5-fluorouracil

The reaction of 3-(N,N-diallylcarbamoyl)propionic acid and diphenylphosphoryl azide in a solution of triethyl amine and benzene provided a solution comprising 2-(N',N'-diallylcarbamoyl)ethyl isocyanate (I.R.: 2275 $cm^{-1}$), which was reacted with 5-fluorouracil to provide 1-[N-[2-(N',N'-diallylcarbamoyl)ethyl]carbamoyl]-5-fluorouracil substantially in the similar method to that of Example 4.

m.p. 126—127°C (recrystallized from ethyl acetate).

N.M.R. ($DMSO-d_6$) (ppm):

2.40 to 2.72 (2H, m), 3.36 to 3.64 (2H, m), 3.90 (4H, d, J=5Hz), 4.92 to 5.3 (4H, m), 5.5 to 6.05 (2H, m), 8.36 (1H, d, J=8Hz), 9.36 (1H, t, J=6Hz).

Example 9

1-[N-(2-Morpholinocarbonylethyl)carbamoyl]-5-fluorouracil

The reaction of 3-morpholinocarbonylpropionic acid and diphenylphosphoryl azide in a solution of triethyl amine and benzene provided a solution comprising 2-morpholinocarbonylethyl isocyanate, which was reacted with 5-fluorouracil to provide 1-[N-(2-morpholinocarbonylethyl)carbamoyl]-5-fluorouracil substantially in the similar method to that of Example 4.

m.p. 178—180°C (recrystallized from ethyl acetate).

2.3 to 2.8 (2H, m), 3.1 to 3.7 (10H, m), 8.38 (1H, d, J=8Hz), 9.36 (1H, t, J=6Hz).

Example 10

1-[N-(2-Propylthioethyl)carbamoyl]-5-fluorouracil

A solution of diphenylphosphoryl azide [$N_3 \cdot PO(OC_6H_5)_2$] (8.25 g.) in dry benzene (10 ml.) and a solution of triethylamine (3.03 g.) in dry benzene (10 ml.) were added to a solution of 3-propylthiopropionic acid (4.44 g.) in dry benzene (10 ml.) with stirring at ambient temperature. The clear solution was stirred for 30 minutes at 70—80°C to give a solution comprising 2-propylthioethyl isocyanate (I.R.: 2260 $cm^{-1}$).

To the above solution of 2-propylthioethyl isocyanate was added a solution of 5-fluorouracil (2.60 g.) in N,N-dimethylacetamide (20 ml.) at 80°C and stirred for further 8 hours at the same temperature. The resultant mixture was diluted with ethyl acetate (200 ml.), washed with water (each 30 ml., 3 times), dried over magnesium sulfate, treated with activated charcoal, filtered and evaporated in vacuo. The residue was recrystallized from ethyl acetate to give 1-[N-(2-propylthioethyl)carbamoyl]-5-fluorouracil.

m.p. 112—114°C (recrystallized from ethyl acetate).

N.M.R. (DMSO-$d_6$) (ppm):

0.95 (3H, t, J=8Hz), 1.56 (2H, sex, J=8Hz), 2.4 to 2.8 (4H, m), 3.36 to 3.64 (2H, m), 8.40 (1H, d, J=8Hz), 9.36 (1H, t, J=6Hz).

Example 11

1-[N-[2-(2-Cyanoethylthio)ethyl]carbamoyl]-5-fluorouracil

The mixture of acrylonitrile (5.31 g.) and 3-mercaptopropionic acid (10.6 g.) was stirred for 1 day at ambient temperature and distilled for 1 day at ambient temperature and distilled under reduced pressure to give colorless liquid of 3-(2-cyanoethylthio)propionic acid (7.5 g.). B.p. 124—126°C/10—13 mm Hg.

I.R. (Nujol): 2250, 1720 $cm^{-1}$.

The reaction of 3-(2-cyanoethylthio)propionic acid and diphenylphosphoryl azide in a solution of triethylamine and benzene provided a solution comprising 2-(2-cyanoethylthio)ethyl isocyanate (I.R.: 2250 $cm^{-1}$), which was reacted with 5-fluorouracil to provide 1-[N-[2-(2-cyanoethylthio)ethyl]-carbamoyl]-5-fluorouracil substantially in the similar method to that of Example 10.

m.p. 121—123°C (recrystallized from ethyl acetate).

N.M.R. (DMSO-$d_6$) (ppm):

2.07 (2H, t, J=7Hz), 2.6 to 3.1 (2H, m), 3.1 to 3.8 (4H, m), 8.4 (1H, d, J=8Hz), 9.33 (1H, t, J=6Hz).

Example 12

1-[N-(2-Phenylthioethyl)carbamoyl]-5-fluorouracil

The reaction of 3-phenylthiopropionic acid and diphenylphosphoryl azide in a solution of triethyl amine and benzene provided a solution comprising 2-phenylthioethyl isocyanate (I.R.: 2260 $cm^{-1}$), which was reacted with 5-fluorouracil to provide 1-[N-(2-phenylthioethyl)carbamoyl]-5-fluorouracil substantially in the similar method to that of Example 10.

m.p. 130—133°C (recrystallized from ethyl acetate).

N.M.R. (DMSO-$d_6$) (ppm):

2.7 to 3.7 (4H, m), 7.1 to 7.45 (5H, m), 8.38 (1H, d, J=8Hz), 6.25 [nh]*a), 9.45 [h]*b) (1H, t, J=6Hz; t, J=6Hz), 11.7—12.4 (1H, broad)

*a) [nh]; Non-hydrogen-bonded,

*b) [h]; hydrogen-bonded

Example 13

1-[N-(4-Pyridylmethyl)carbamoyl]-5-fluorouracil

Diphenylphosphoryl azide (5.5 g.) was added to a solution of 4-pyridyl acetic acid (2.74 g.) with stirring at ambient temperature. The clear solution was stirred for 30 minutes at 80°C to give a solution comprising 4-pyridylmethyl isocyanate (I.R.: 2280 $cm^{-1}$).

To the above solution of 4-pyridylmethyl isocyanate was added 5-fluorouracil (2.60 g.) at 80°C and stirred for 4 hours at the same temperature. The resultant mixture was evaporated in vacuo. The residue was washed with diethyl ether (3 times) and crystallized. The crystals were filtered and washed with a small amount of ethanol. To the washed crystals a small amount of pyridine was added, and the mixture was warmed to be dissolved and cooled to be recrystallized. The crystals were washed successively with ethyl acetate and diethyl ether to give 1-[N-(4-pyridylmethyl)carbamoyl]-5-fluorouracil (1.75 g.)

m.p. 195—200°C (dec.) (recrystallized from pyridine).

N.M.R. (DMSO-$d_6$) (ppm):

4.5 (2H, d, J=6Hz), 7.2 to 7.85 (2H, m), 8.33 (1H, d, J=8Hz), 8.4 to 8.65 (2H, m), 9.57 (1H, t, J=6Hz), 11.2 to 13.0 (1H, broad)

Example 14

1-[N-(2-Thienylmethyl)carbamoyl]-5-fluorouracil

The reaction of 2-thienylacetic acid and diphenylphosphoryl azide in a solution of triethyl amine and benzene provided a solution comprising 2-thienylmethyl isocyanate, which was reacted with 5-fluorouracil to provide 1-[N-(2-thienylmethyl)-carbamoyl]-5-fluorouracil substantially in the similar method to that of Example 13.

m.p. 131—132°C (recrystallized from ethyl acetate).

N.M.R. (DMSO-$d_6$) (ppm):

4.38 (1H, d, J=6Hz), 4.65 (1H, d, J=6Hz), 6.9 to 7.5 (3H, m), 8.42 (1H, d, J=7Hz), 9.66 (1H, t, J=6Hz), 12.0 to 12.5 (1H, broad)

Example 15

1-[N-[4-(1,2-Dithiolan-3-yl)-butyl]carbamoyl]-5-fluorouracil

The reaction of 5-(1,2-dithiolan-3-yl)pentanoic acid and diphenylphosphoryl azide in a solution of triethyl amine and benzene provided a solution comprising 4-(1,2-dithiolan-3-yl)butyl isocyanate (I.R.: 2270 $cm^{-1}$), which was reacted with 5-fluorouracil to provide 1-[N-[4-(1,2-dithiolan-3-yl)-butyl]carbamoyl]-5-fluorouracil substantially in the similar method to that of Example 13.

m.p. 126°C (recrystallized from diethyl ether).

N.M.R. ($CDCl_3$) (ppm):

1.4 to 3.9 (13H, m), 8.52 (1H, d, J=7Hz), 9.10 (1H, broad).

Example 16

1-[N-[5-(2,5-dioxopyrrolidin-1-yl)pentyl]carbamoyl]-5-fluorouracil

The reaction of 6-[(2,5-dioxopyrrolidin-1-yl)-hexanoic acid and diphenylphosphoryl azide in a solution of triethyl amine and benzene provided a solution comprising 5-(2,5-dioxopyrrolidin-1-yl)-pentyl isocyanate, which was reacted with 5-fluorouracil to provide 1-[N-[5-(2,5-dioxopyrrolidin-1-yl)-pentyl]carbamoyl]-5-fluorouracil substantially in the similar method to that of Example 13.

m.p. 142—142.5°C (recrystallized from ethyl acetate).

N.M.R. (DMSO-$d_6$) (ppm):

1.2 to 1.8 (6H, m), 2.63 (4H, s), 3.1 to 3.6 (4H, m), 8.40 (1H, d, J=8Hz), 9.10 (1H, t, J=6Hz).

Example 17

1-[N-[2-(*trans*-2-Furyl)ethenyl]carbamoyl]-5-fluorouracil

A solution of diphenylphosphoryl azide [$N_3 \cdot PO(OC_6H_5)_2$] (5.50 g.) in dry benzene (10 ml.) and a solution of triethyl amine (2.0 g.) in dry benzene (10 ml.) were added to a solution of *trans*-3-furylacrylic acid (2.76 g.) in dry benzene (10 ml.) with stirring at ambient temperature. The clear solution was stirred for 30 minutes at 80°C to give a solution comprising 2-(2-furyl)ethenyl isocyanate (I.R.: 2150 $cm^{-1}$).

To the above solution of 2-(2-furyl)ethenyl isocyanate was added a solution of 5-fluorouracil (2.60 g.) in N,N-dimethylacetamide (15 ml.) at 80°C and stirred for further 3 hours at the same temperature. The resultant mixture was diluted with ethyl acetate (200 ml.), washed with water (3 times), dried over magnesium sulfate, treated with activated charcoal, filtered and evaporated in vacuo. The residue was recrystallized from ethyl acetate to give 1-[N-[2-(*trans*-2-furyl)ethenyl]carbamoyl]-5-fluorouracil.

m.p. 215—217°C (dec.) (recrystallized from ethyl acetate).

N.M.R. (DMSO-$d_6$) (ppm):

5.95 (1H, d, J=15Hz), 6.05 to 7.6 (4H, m), 7.7 (1H, d, J=7Hz), 9.0 [h]*b), 8.4 [nh]*a) (1H, d, J=11Hz; d, J=8Hz), 10.7 to 11.7 (1H, broad)

*a) [nh]; Non-hydrogen-bonded,

*b) [h]; Hydrogen-bonded

Example 18

1-[N-[*trans*-2-(2,6-Dimethoxyphenyl)ethenyl]carbamoyl]-5-fluorouracil

The reaction of 3-(2,6-methoxyphenyl)acrylic acid and diphenylphosphoryl azide in a solution of triethyl amine and benzene provided a solution comprising: 2-(2,6-dimethoxyphenyl)ethyenyl Isocyanate (I.R.: 2150 $cm^{-1}$), which was reacted with 5-fluorouracil to provide 1-[N-[*trans*-2-(2,6-dimethoxyphenyl)ethenyl]carbamoyl]-5-fluorouracil substantially in the similar method to that of Example 17.

m.p. 193—195°C (recrystallized from ethyl acetate).

N.M.R. (DMSO-$d_6$) (ppm):

3.70 (3H, s), 3.78 (3H, s), 6.18 (1H, d, J=14Hz), 6.5 to 7.1 (3H, m), 7.15 to 7.57 (1H, m), 7.77 (1H, d, J=7Hz), 8.9 [h]*b), 8.37 [nh]*a) (1H, d, J=11Hz; d, J=8Hz), 10.8 to 11.7 (1H, broad).

*a) [nh]; Non-hydrogen-bonded

*b) [h]; Hydrogen-bonded

Example 19

1-[N-(*trans, trans*-1,3-Pentadienyl)-carbamoyl]-5-fluorouracil

A solution of diphenylphosphoryl azide [$N_3 \cdot PO(OC_6H_5)_2$] (5.50 g.) in dry benzene (10 ml.) and a solution of triethylamine (2.02 g.) in dry benzene (10 ml.) were added to a solution of 2,4-hexadienoic acid (2.24 g.) in dry benzene (10 ml.) with stirring at ambient temperature. The clear solution was stirred for 3 hours at 50°C to give a solution comprising *trans, trans*-1,3-pentadienyl isocyanate.

To the above solution of *trans, trans*-1,3-pentadienyl isocyanate was added a solution of 5-fluorouracil (2.6 g.) in N,N-dimethylacetamide (15 ml.) at 80°C and stirred for 3 hours at the same temperature. The resultant mixture was diluted with ethyl acetate (100 ml.), washed with water (4 times), dried over magnesium sulfate, filtered and evaporated in vacuo. The residue was recrystallized from diethyl ether to give 1-[N-(*trans, trans*-1,3-pentadienyl)carbamoyl]-5-fluorouracil.

m.p. 163°C (recrystallized from diethyl ether).

N.M.R. (DMSO-$d_6$) (ppm):

1.5 to 1.83 (3H, m), 5.13 to 7.0 (4H, m), 7.73 (1H, d, J=6Hz), 8.37 [nh]*a), 10.86 [h]*b) (1H, d, J=8Hz; d, J=10Hz), 11.0 to 11.9 (1H, broad).

*a) [nh]; Non-hydrogen-bonded

*b) [h]; Hydrogen-bonded

## Example 20

1-[N-(3-*t*-Butoxycarbonylaminopropyl)carbamoyl]-5-fluorouracil

A solution of diphenyl phosphoryl azide [$N_3$·PO($OC_6H_5$)$_2$] (5.50 g.) in dry benzene (10 ml.) and a solution of triethylamine (2.02 g.) in dry benzene (10 ml.) were added to a solution of 4-*t*-butoxycarbonylaminobutylic acid (4.06 g.) in dry benzene (10 ml.) with stirring at ambient temperature. The clear solution was stirred for 30 minutes at 80°C to give a solution comprising 3-*t*-butoxycarbonylaminopropyl isocyanate (I.R.: 2280 $cm^{-1}$).

To the above solution of 3-*t*-butoxycarbonylaminopropyl isocyanate was added a solution of 5-fluorouracil (2.60 g.) in N,N-dimethylacetamide (20 ml.) at 80°C and stirred for further 3 hours at the same temperature. The resultant mixture was diluted with ethyl acetate (200 ml.), washed with water (each 30 ml., 3 times), dried over magnesium sulfate, treated with activated charcoal, filtered and evaporated in vacuo.

The residue was recrystallized from ethyl acetate to give 1-[N-(3-*t*-butoxycarbonylaminopropyl)-carbamoyl]-5-fluorouracil.

I.R. (Nujol):

3250, 3100, 2930, 2850, 1760, 1720, 1680, 1490, 1430, 1310, 1280, 1240, 1200, 1175, 1125, 1070, 910, 860, 810, 770, 740, 720 $cm^{-1}$

## Example 21

1-[N-(5-*t*-Butoxycarbonylamino)pentyl)carbamoyl]-5-fluorouracil

The reaction of 6-*t*-butoxycarbonylaminohexanoic acid and diphenylphosphoryl azide in a solution of triethyl amine and benzene provided a solution comprising 5-*t*-butoxycarbonylaminopentyl isocyanate, which was reacted with 5-fluorouracil to provide 1-[N-(5-*t*-butoxycarbonylaminopentyl)-carbamoyl]-5-fluorouracil substantially in the similar method to that of Example 20.

I.R. (Nujol):

3200, 3100, 3050, 2930, 2850, 1760, 1720, 1680, 1490, 1430, 1395, 1310, 1280, 1240, 1200, 1175, 1125, 1070, 910, 860, 810, 770, 760, 720 $cm^{-1}$

## Example 22

1-[N-[2-(*trans*-2-Thienyl)ethenyl]carbamoyl]-5-fluorouracil

The reaction of *trans*-3-(2-thienyl)acrylic acid and diphenylphosphoryl azide in a solution of triethyl amine and benzene provided a solution comprising 2-(2-thienyl)ethenyl isocyanate, which was reacted with 5-fluorouracil to provide 1-[N-[2-(*trans*-2-thienyl)ethenyl]carbamoyl]-5-fluorouracil substantially in the similar method to that of Example 17.

m.p. 163—166°C (dec.) (recrystallized from ethyl acetate).

N.M.R. (DMSO-$d_6$) (ppm):

6.28 (1H, d, J=14Hz), 6.6 to 7.5 (4H, m), 7.75 (1H, d, J=6Hz), 8.41 [nh]*a), 9.0 [h]*b), (1H, d, J=7Hz; d, J=11Hz), 10.5 to 12.0 (1H, broad).

*a) [nh]; Non-hydrogen-bonded

*b) [h]; Hydrogen-bonded

## Example 23

D,L-1-[N-(3-Methoxycarbonyl-5-norbornen-2-yl)carbamoyl]-5-fluorouracil

A mixture of 5-norbornen-2,3-dicarboxylic anhydride (24.6 g.) and methanol (68 g.) was heated to reflux for 8 hours and evaporated to dryness under reduced pressure. The oily residue was made solidified and dried to give D,L-5-norbornen-2,3-dicarboxylic acid monomethyl ester (29.0 g.), as powders.

N.M.R. ($CDCl_3$) (ppm):

1.33 to 1.66 (2H, m), 3.0 to 3.43 (4H, m), 3.57 (3H, s), 6.1 to 6.5 (2H, m), 10.12 (1H, s).

The reaction of D,L-5-norbornen-2,3-dicarboxylic acid monomethyl ester and diphenylphosphoryl azide in a solution of triethyl amine and benzene provided a solution comprising 3-methoxycarbonyl-5-norbornen-2-yl isocyanate, which was reacted with 5-fluorouracil to provide D,L-1-[N-3-(methoxycarbonyl-5-norbornen-2-yl)carbamoyl]-5-fluorouracil substantially in the similar method to that of Example 1.

m.p. 144—145°C (crystallized from ethyl acetate).

N.M.R. (DMSO-$d_6$) (ppm):

1.47 (2H, broad s), 3.12 (2H, broad s), 3.23 to 3.43 (1H, m), 3.5 (3H, s), 4.6 to 5.1 (1H, m), 6.07 to 6.6 (2H, m), 8.4 (1H, d, J=8Hz), 9.09 (1H, d, J=9Hz), 11.5 to 12.8 (1H, broad)

### Example 24

D,L-1-[N-(2-Methoxycarbonylnorborn-3-yl)carbamoyl]-5-fluorouracil

D,L-5-Norbornen-2,3-dicarboxylic acid monomethyl ester which was prepared in Example 23 (24.0 g.) was dissolved in ethanol (300 ml.), and hydrogenated over 10% palladium charcoal catalyst under hydrogen atmosphere of ordinary pressure at ambient temperature. The reaction mixture was filtrated and the filtrate was concentrated in vacuo. The oily residue was made solidified and dried to give D,L-norbornan-2,3-dicarboxylic acid monomethyl ester (24.3 g.), as powders.

I.R. (Nujol):

1730, 1700, 1435, 1420, 1360, 1330, 1310, 1300, 1290, 1255, 1240, 1210, 1190, 1120, 1080, 900, 740 $cm^{-1}$.

N.M.R. ($DMSO-d_6$) (ppm):

1.1 to 2.1 (6H, m), 2.33 to 2.7 (2H), 3.0 (2H, broad s), 3.53 (3H, s)

The reaction of D,L-norbornan-2,3-dicarboxylic acid monomethyl ester and diphenylphosphoryl azide in a solution of triethyl amine and benzene provided a solution comprising D,L-2-methoxycarbonylnorborn-3-yl isocyanate (I.R.: 2300 $cm^{-1}$), which was reacted with 5-fluorouracil to provide D,L-1-[N-(2-methoxycarbonylnorborn-3-yl)carbamoyl]-5-fluorouracil substantially in the similar method to that of Example 1.

m.p. 164—165°C (crystallized from ethyl acetate).

N.M.R. ($DMSO-d_6$) (ppm):

1.5 (6H, broad s), 2.33 to 2.67 (2H), 2.9 to 3.4 (1H, m), 3.6 (3H, s), 4.07 to 4.6 (1H, m), 8.4 (1H, d, J=8Hz), 10.07 (1H, d, J=8Hz), 12.3 (1H, broad s).

### Example 25

1-[N-(3-Aminopropyl)carbamoyl]-5-fluorouracil hydrochloride

Hydrogen chloride gas was introduced to the solution of 1-[N-[3-(*t*-butoxycarbonylamino)-propyl]-carbamoyl]-5-fluorouracil (5.1 g.) which was prepared in Example 20 in ethanol (30 ml.) until precipitates were appeared for 1 hour with stirring under cooling with ice. The reaction mixture was further stirred for 1 hour at ambient temperature, and diethyl ether (100 ml.) was added therein. The resultant crystalline precipitates were collected by filtration and were dried over phosphoric anhydride to give 1-[N-(3-aminopropyl)carbamoyl]-5-fluorouracil hydrochloride (1.5 g.).

m.p. 173—174°C (crystallized from diethyl ether).

N.M.R. ($DMSO-d_6$) (ppm):

1.43 to 2.17 (2H, m), 2.53 to 3.57 (4H, m), 8.27 (1H, d, J=8Hz), 9.1 (1H, t, J=6Hz), 12.27 (1H, broad).

### Example 26

1-[N-(5-Aminopentyl)carbamoyl]-5-fluorouracil hydrochloride

1-[N-(5-Aminopentyl)carbamoyl]-5-fluorouracil hydrochloride was prepared in substantially the similar method to that of Example 25 from 1-[N-[5-(*t*-butoxycarbonylamino)pentyl]carbamoyl]-5-fluorouracil which was prepared in Example 21.

m.p. 171—172°C (dec.) (crystallized from diethyl ether).

N.M.R. ($DMSO-d_6$) (ppm):

1.5 to 2.0 (6H, m), 2.5 to 3.1 (2H, m), 3.1 to 3.6 (2H, m), 8.4 (1H, d, J=8Hz), 9.15 (1H, t, J=6Hz), 12.37 (1H, broad).

### Example 27

1-[N-(2-Tetrahydrothienyl)carbamoyl]-5-fluorouracil

Thionyl chloride (16.5 g.) was added to 2-tetrahydrothiophenecarboxylic acid (12.2 g.) at ambient temperature and heated for 2 hours at 80°C. The reaction mixture was evaporated to dryness under reduced pressure and the residual oil was fractionated by distillation in vacuo to give 2-tetrahydrothiophenecarboxylic acid chloride (9.79 g.), (b.p. 46°C/1 mmHg).

N.M.R. ($CDCl_3$) (ppm):

1.8 to 2.6 (4H, m), 2.85 to 3.2 (2H, m), 4.15 to 4.4 (1H, m).

A solution of sodium azide (4.14 g.) in water (16 ml.) was added dropwise to a solution of 2-tetrahydrothiophenecarboxylic acid chloride (9.60 g.) in diethyl ether (24 ml.) with stirring under ice-cooling and stirred for further 15 minutes at the same temperature. After the mixture was stirred for 1 hour at ambient temperature, nitrogen gas was introduced to remove off diethyl ether. The resultant mixture was extracted with diethyl ether (50 ml.). The extracts were washed with water, dried over magnesium sulfate and concentrated in vacuo to give oily 2-tetrahydrothiophenecarboxylic acid azide (8.63 g.).

I.R. (firm):

2950, 2860, 2150, 1710, 1680, 1590, 1310, 1160—1240, 1080, 900 $cm^{-1}$.

A solution of 2-tetrahydrothiophenecarboxylic acid azide (8.5 g.) in benzene (20 ml.) was stirred at 70°C for 4 hours under nitrogen atmosphere. The reaction mixture was evaporated to dryness under reduced pressure to give oily 2-tetrahydrothienyl isocyanate (7.3 g.).

I.R. (firm):

2950, 2880, 2250, 1680, 1590, 1440, 1310, 1190—1240, 900 $cm^{-1}$.

A solution of 5-fluorouracil (2.6 g.) in N,N-dimethylacetoamide (10 ml.) was added to oily 2-tetrahydrothiophenyl isocyanate (3.1 g.). The mixture was stirred at 50°C for 8 hours and concentrated in vacuo. The residue was treated with water (80 ml.) to give crystalline precipitates, which were collected by filtration and dried over phosphoric anhydride to give 1-[N-(2-tetrahydrothienyl)-carbamoyl]-5-fluorouracil (3.68 g.); pale yellow crystalline powder.

m.p. 148°C (recrystallized from diethyl ether).

N.M.R. (DMSO-$d_6$) (ppm):

1.8 to 2.2 (4H, m), 2.7 to 3.2 (2H, m), 5.32 to 5.55 (1H, m), 8.30 (1H, d, J=8Hz), 9.40 (1H, d, J=8Hz), 12.35 (1H, broad s).

Example 28

1-[N-(4-Chlorophenyl)carbamoyl]-5-fluorouracil

A solution of 5-fluorouracil (2.6 g.) in pyridine (25 ml.) was added to 4-chlorophenyl isocyanate (3.07 g.) and the solution was stirred at 80°C for 4 hours. The resultant solution was allowed to stand overnight in a refrigerator. The crystalline precipitates were collected by filtration, washed successively with ethyl acetate, diethyl ether, and then dried to give 1-[N-(4-chlorophenyl)carbamoyl)-5-fluorouracil (3.30 g); colorless crystalline powder.

m.p. over 260°C (recrystallized from pyridine).

N.M.R. (DMSO-$d_6$) (ppm):

7.2—7.7 (4H, m), 7.73 (1H, d, J=6Hz), 8.80 (1H, t, J=7Hz), 10.5 to 11.67 (1H, broad).

Example 29

1-[N-(2-Norbornyl)carbamoyl]-5-fluorouracil

The reaction of 2-norbornanecarboxlic acid and diphenylphosphoryl azide in a solution of triethylamine and benzene provided a solution comprising 2-norbornyl isocyanate (I.R.: 2270 $cm^{-1}$), which was reacted with 5-fluorouracil to provide 1-[N-(2-norbornyl)carbamoyl]-5-fluorouracil substantially in the similar method to that of Example 1.

m.p. 252—255°C (recrystallized from ethyl acetate).

N.M.R. (DMSO-$d_6$) (ppm):

1.0 to 1.67 (8H, broad s), 1.80 to 2.43 (2H, m), 3.33 to 3.73 (1H, m), 8.43 (1H, d, J=8Hz), 9.33 (1H, d, J=7Hz).

Example 30

1-[N-[3-(2-Thienyl)propyl]carbamoyl]-5-fluorouracil

Diphenyl phosphoryl azide [$N_3 \cdot PO(OC_6H_5)_2$] (9.08 g.) was added dropwise to a solution of 4-(2-thienyl)butyric acid (5.53 g.) in dry pyridine (25 ml.) and stirred for one hour at 100°C to give a solution comprising 3-(2-thienyl)propyl isocyanate (I.R.: 2260 $cm^{-1}$).

5-Flourouracil (3.30 g.) was added to the solution of 3-(2-thienyl)propyl isocyanate and stirred at 100°C for 2 hours. The resultant mixture was evaporated to dryness under reduced pressure. The residue was dissolved in ethyl acetate, washed with water, dried over magnesium sulfate, filtered and evaporated under reduced pressure. The residue was recrystallized from ethanol to give colorless plate 1-[N-[3-(2-thienyl)propyl]carbamoyl]-5-fluorouracil (2.3 g.). M.p. 131—133°C.

N.M.R. (DMSO-$d_6$) (ppm):

1.90 (2H, quintet, J=7Hz) 2.87 (2H, t, J=7Hz), 3.37 (2H, quartet, J=7Hz), 6.83 to 7.07 (2H, m), 7.20 to 7.40 (1H, m), 8.40 (1H, d, J=8Hz), 9.18 (1H t, J=6Hz), 12.22 (1H, s)

Example 31

1-[N-(3-*tert*-Butoxycarbonylpropyl)carbamoyl]-5-fluorouracil

Glutaric anhydride (11.41 g.) was added portionwise to a solution of potassium *tert*-butoxide (14.02 g.) in tetrahydrofuran (100 ml.) at ambient temperature. After the exothermic reaction was ceased, the reaction mixture was stirred at 40°C for 15 minutes. Tetrahydrofuran was distilled off under reduced pressure. The residue was dissolved in water. After ethyl acetate was added, the solution was adjusted to pH 9 with aqueous potassium carbonate solution under ice-cooling. The separated water layer was washed with ethyl acetate, treated with activated charcoal, overlapped with ethyl acetate, and then adjusted to pH 4.0 with 10% hydrochloric acid under ice-cooling. The ethyl acetate layer was dried over magnesium sulfate, treated with activated charcoal, filtered and concentrated under reduced pressure to give glutaric acid mono-*tert*-butyl ester (10 g.).

N.M.R. ($CDCl_3$) (ppm):

1.43 (9H, s), 1.67 to 2.67 (6H, m)

Diphenylphosphoryl azide (12.25 g.) was added dropwise to a solution of glutaric acid mono-*tert*-butyl ester (8.38 g.) in dry pyridine (37 ml.) and stirred at 90°C for 40 minutes to give 3-*tert*-butoxycarbonylpropyl isocyanate (I.R.: 2270 $cm^{-1}$).

5-Fluorouracil (4.83 g.) was added to the solution and stirred at 90°C for 3 hours. The resultant

mixture was evaporated under reduced pressure. The residue was dissolved in ethyl acetate and washed with dilute hydrochloric acid and water. Ethyl acetate layer was dried over magnesium sulfate, treated with activated charcoal, filtered and concentrated under reduced pressure to give the crystals of 1-[N-(3-*tert*-butoxycarbonylpropyl)carbamoyl-5-fluorouracil. Recrystallization from ethanol gave the pure compound (411 mg.).

N.M.R. (DMSO-$d_6$) (ppm):

1.43 (9H, s), 1.57 to 2.33 (4H, m,), 3.57 (2H, m), 8.68 (1H, d, J=8Hz), 11.00 (1H, broad), 14.40 (1H, broad)

Example 32

1-[N-(3-Carboxypropyl)carbamoyl]-5-fluorouracil

1-[N-(3-*tert*-Butoxycarbonylpropyl)carbamoyl]-5-fluorouracil (400 mg.) obtained in Example 31 was suspended in anisole (0.4 ml.). Trifluoroacetic acid (1.6 ml.) was added to the suspension under ice-cooling and stirred for 40 minutes at ambient temperature. The resultant solution was concentrated under reduced pressure to give residue, which was washed successively with ethanol, water, ethanol and diethyl ether to give the crystals of 1-[N-(3-carboxypropyl)carbamoyl]-5-fluorouracil (228 mg.). M.p. 146—147°C.

N.M.R. (DMSO-$d_6$):

1.73 (2H, quintet, J=7Hz), 2.20 (2H, d, J=7Hz), 3.30 (2H, quartet, J=7Hz), 8.30 (1H, d, J=8Hz), 9.07 (1H, t, J=6Hz), 12.20 (1H, broad).

Example 33

1-[N-(1-Adamantyl)carbamoyl]-5-fluorouracil

Diphenylphosphoryl azide (9.08 g.) was added dropwise to a solution of 1-adamantanecarboxylic acid (5.4 g.) in dry pyridine (25 ml.) and stirred at 70°C for one hour to give 1-adamantyl isocyanate [I.R.: 2250 $cm^{-1}$].

5-Fluorouracil (2.6 g.) was added to the solution and stirred at 100°C for 7 hours. The resultant mixture was evaporated to dryness under reduced pressure. The residue was dissolved in ethyl acetate, chromatographed on silica gel, and eluted with ethyl acetate to give 1-[N-(1-adamantyl)carbamoyl]-5-fluorouracil (0.85 g.). M.p. above 260°C.

N.M.R. ($CDCl_3$) (ppm):

1.5 to 2.3 (15H, m), 8.47 (1H, d, J=8Hz), 9.0 (1H, broad s.), 9.47 (1H, broad s.)

Example 34

1-[N-[2-(2-Norbornyl)ethyl]carbamoyl]-5-fluorouracil

Diphenylphosphoryl azide (6.05 g.) was added dropwise to a solution of 3-(2-norbornyl)propionic acid (3.36 g.) in dry pyridine (20 ml.) and stirred at 80°C for 30 minutes to give 2-(2-norbornyl)ethyl isocyanate (I.R. 2260 $cm^{-1}$).

5-Fluorouracil (1.3 g.) was added to the solution and stirred at 100°C for 4 hours. The resultant mixture was evaporated under reduced pressure. The residue was dissolved in ethyl acetate and washed with water. The ethyl acetate layer was dried over magnesium sulfate, filtered and concentrated. The residue was recrystallized from ethanol to give cristals of 1-[N-[2-(2-norbornyl)ethyl]carbamoyl]-5-fluorouracil (0.6 g.). M.p. 128—130°C

N.M.R. ($CDCl_3$) (ppm):

0.9 to 2.6 (15H, m), 8.46 (1H, d, J=7Hz), 9.0 (1H, d, J=7Hz), 9.78 (1H, s)

Example 35

D,L-1-[N-(3-Ethoxycarbonylnorborn-2-yl)- carbamoyl]-5-fluorouracil

5-Norbornen-2,3-dicarboxylic anhydride was treated with ethanol to provide D,L-5-norbornene-2,3-dicarboxylic acid monoethyl ester substantially in the similar manner described for the preparation of D,L-5-norbornene-2,3-dicarboxylic acid monomethyl ester described in Example 23.

N.M.R. ($CDCl_3$) (ppm):

1.20 (3H, t, J=8Hz), 1.40 (2H, quartet, J=8Hz), 3.30 (2H, s), 4.04 (2H, quartet, J=8Hz), 6.24 (2H, m), 16.32 (1H, s)

D,L-Norbornane-2,3-dicarboxylic acid monoethyl ester was prepared from D,L-5-norbornene-2,3-dicarboxylic acid monoethyl ester substantially in the similar manner to that of D,L-norbornane-2,3-dicarboxylic acid monomethyl ester described in Example 24.

N.M.R. ($CDCl_3$) (ppm):

1.10 to 1.93 (9H, m), 2.60 (2H, s), 3.00 (2H, s), 4.10 (2H, quartet, J=7Hz), 10.83 (1H, s)

Diphenylphosphoryl azide (10.11 g.) was added dropwise to a solution of D,L-norbornane-2,3-dicarboxylic acid monoethyl ester (7.09 g.) in dry pyridine (25 ml.) and stirred at 80°C for 35 minutes to give D,L-3-ethoxycarbonylnorborn-2-yl isocyanate (I.R.: 2280 $cm^{-1}$).

5-Fluorouracil (3.25 g.) was added to the solution and stirred at 80°C for 4 hours. The resultant mixture was evaporated under reduced pressure. The residue was dissolved in ethyl acetate and washed with dilute hydrochloric acid and water. Ethyl acetate layer was dried over magnesium sulfate,

filtered and evaporated to give residue, which was recrystallized from ethanol to give colorless crystals of D,L-1-[N-(3-ethoxycarbonylnorborn-2-yl)carbamoyl]-5-fluorouracil (2.91 g.). M.p. 160—161°C.

N.M.R. (DMSO-$d_6$) (ppm):

1.13 (3H, t, J=7Hz), 1.48 (7H, broad s), 3.10 (2H, m), 4.06 (2H, quintet, J=7Hz), 4.37 (1H, dd, J=8Hz, J=8Hz), 8.38 (1H, d, J=7Hz), 10.0 (1H, d, J=8Hz), 12.2 (1H, s).

Example 36

D, L-1-[N-(3-Isopropoxycarbonylnorborn-2-yl)carbamoyl]-5-fluorouracil

5-Norbornene-2,3-dicarboxylic anhydride (20.0 g.) was added to a solution of sodium isopropoxide in isopropanol which was prepared from sodium (2.78 g.) and isopropanol (100 ml.). The solution was refluxed for 2 hours, and evaporated under reduced pressure. The residue was dissolved in water and overlapped with ethyl acetate. The mixture was adjusted to pH 1 with 10% hydrochloric acid under ice-cooling. The ethyl acetate layer was dried over magnesium sulfate, treated with activated charcoal, filtered, and concentrated to dryness to give crystalline powders of D,L-5-norbornene-2,3-dicarboxylic acid monopropyl ester (20.49 g.).

N.M.R. (DMSO-$d_6$) (ppm):

1.20 (6H, d, J=8Hz), 1.43 (2H, m), 3.03 (2H, broad s), 3.13 to 3.20 (2H, s), 4.92 (1H, quintet, J=8Hz), 6.17 (2H, m).

D,L-Norbornane-2,3-dicarboxylic acid monoisopropyl ester was obtained by catalytic reduction of D,L-5-norbornene-2,3-dicarboxylic acid monoisopropyl ester substantially in the similar manner to that of D,L-norbornane-2,3-dicarboxylic acid monomethyl ester described in Example 24.

N.M.R. (DMSO-$d_6$) (ppm):

1.13 to 1.80 (12H, m), 2.53 (3H, m), 2.97 (1H, quartet, J=5Hz), 4.87 (1H, quintet, J=8Hz).

Diphenylphosphoryl azide (13.48 g.) was added dropwise to a solution of D,L-norbornane-2,3-dicarboxylic acid monoisopropyl ester (11.08 g.) in dry pyridine (41 ml.) and stirred at 100°C for one hour to give D,L-3-isopropoxycarbonylnorborn-2-yl isocyanate (I.R.: 2260 $cm^{-1}$).

5-Fluorouracil (5.31 g.) was added to the solution and stirred at 100°C for 4 hours. The resultant mixture was evaporated under reduced pressure. The residue was dissolved in ethyl acetate and washed with dilute hydrochloric acid and water. Ethyl acetate layer was dried over magnesium sulfate, treated with activated charcoal, filtered and concentrated under reduced pressure. The residue obtained was recrystallized from ethanol to give D,L-1-[N-(3-isopropoxycarbonylnorborn-2-yl)-5-fluorouracil (275 mg.). M.p. 135—136°C.

N.M.R. ($CDCl_3$) (ppm):

1.23 (6H, d, J=8Hz), 1.37 to 2.07 (7H, m), 2.50 (2H, broad s), 4.45 (1H, m), 5.03 (1H, q, J=8Hz), 8.47 (1H, d, J=7Hz), 10.97 (1H, d, J=7Hz).

Example 37

D,L-1-[N-(3-*tert*-Butoxycarbonyl-2-norbornyl)carbamoyl] -5-fluorouracil

Potassium *tert*-butoxide (50.0 g.) was dissolved in *tert*-butyl alcohol (200 ml.) under heating. To the solution was added portionwise 5-norbornene-2,3-dicarboxylic anhydride (58.53 g.). The mixture was refluxed and evaporated under reduced pressure. The residue was dissolved in aqueous sodium bicarbonate solution, overlapped with ethyl acetate and adjusted to pH 4.0 with 10% hydrochloric acid. The ethyl acetate layer was washed with water, dried over magnesium sulfate, treated with activated charcoal, filtered and concentrated under reduced pressure to give crystals D,L-3-*tert*-butoxycarbonyl-5-norbornene-2-carboxylic acid (72.80 g.).

N.M.R. (DMSO-$d_6$) (ppm):

1.45 (9H, s), 2.43 (2H, m), 3.05 (2H, m), 3.17 (1H, s), 3.20 (1H, s), 6.13 (1H, m), 6.40 (1H, m), 13.97 (1H, broad s).

D,L-3-*tert*-Butoxycarbonyl-norbornane-2-carboxylic acid was obtained by the catalytic reduction of D,L-3-*tert*-butoxycarbonyl-5-norbornene-2-carboxylic acid substantially in the similar manner to that of D,L-norbornane-2,3-dicarboxylic acid monomethyl ester described in Example 24.

N.M.R. (DMSO-$d_6$) (ppm):

1.13 to 1.77 (15H, m), 2.50 (3H, m), 2.97 (1H, m)

Diphenylphosphoryl azide (45.73 g.) was added dropwise to a solution of D,L-3-*tert*-butoxycarbonylnorbornane-2-carboxylic acid (36.30 g.) in dry pyridine (126 ml.) and stirred at 100°C for 40 minutes to give D,L-3-*tert*-butoxycarbonyl-2-norbornyl isocyanate (I.R.: 2270 $cm^{-1}$).

5-Fluorouracil (16.39 g.) was added to the above solution and stirred for 100°C for 4 hours. The resultant mixture was treated substantially in the similar manner to that of D,L-1-[N-(3-ethoxycarbonyl-2-norbornyl)]-5-fluorouracil described in Example 35 to give D,L-1-[N-(3-*tert*-butoxycarbonyl-2-norbornyl)carbamoyl]-5-fluorouracil (10.35 g.). M.p. 161°C.

N.M.R. (DMSO-$d_6$) (ppm):

1.40 (14H, m), 2.03 (1H, m), 2.40 (1H, m), 3.33 (2H, m), 4.28 (1H, m), 8.46 (1H, d, J=7Hz), 11.13 (1H, d, J=7Hz).

Example 38

D,L-1-[N-(3-Carboxy-2-norbornyl)carbamoyl]-5-fluorouracil

D,L-1-[N-(3-*tert*-butoxycarbonyl-2-norbornyl)carbamoyl]-5-fluorouracil (2.0 g.) prepared in Example 37 was suspended in anisole (2 ml.). Trifluoroacetic acid (8 ml.) was added to the suspension under ice-cooling and stirred for 30 minutes at ambient temperature. The resultant solution was concentrated under reduced pressure, added with diethyl ether and filtered. The filtrate was washed with diethyl ether and recrystallized with ethanol to give D,L-1-[N-(3-carboxy-2-norbonyl)carbamoyl]-5-fluorouracil. M.p. 161°C.

N.M.R. (DMSO-$d_6$) (ppm):

1.17 to 1.67 (5H, m), 2.05 (1H, m), 2.43 (1H, m), 4.30 (1H, m), 8.47 (1H, d, J=8Hz), 11.10 (1H, d, J=7Hz), 14.10 (2H, broad).

Example 39

D,L-1-[N-[3-(N,N -Diisopropylcarbamoyl)-2-norbornyl]carbamoyl]-5-fluorouracil

The mixture of 5-norbornene-2,3-dicarboxylic anhydride (20.0 g.) and diisopropylamine (70 ml.) was refluxed for 40 minutes and evaporated under reduced pressure to remove the excess diisopropylamine. The residue was dissolved in aqueous sodium bicarbonate solution and then to aqueous potassium carbonate solution. The alkaline solution was acidified with 10% hydrochloric acid to pH 1.5 under ice-cooling to give the precipitates, which were filtered, washed with water and dried over phosphoric pentaoxide to give crystalline D,L-3-(N,N-diisopropylcarbamoyl)-5-norbornene-2-carboxylic acid (26.67 g.).

N.M.R. (DMSO-$d_6$) (ppm):

1.00 to 1.32 (14H, m), 2.90 to 3.00 (2H, broad doublet), 3.28 (2H, s), 3.33 (1H, quintet, J=7Hz), 4.06 (1H, quintet, J=7Hz), 5.95 (1H, m), 6.27 (1H, m)

D,L-3-(N,N-diisopropylcarbamoyl)-2-norbornanecarboxylic acid was prepared from D,L-3-(N,N-diisopropylcarbamoyl)-5-norbornene-2-carboxylic acid substantially in the similar manner to that of D,L-norbornane-2,3-dicarboxylic acid monomethyl ester described in Example 24.

N.M.R. (DMSO-$d_6$) (ppm):

1.05 to 1.33 (16H, m), 1.77 (2H, m), 2.33 (2H, broad s), 2.73 (1H, d), 3.27 (2H, m), 4.00 (1H, quintet, J=6Hz).

Diphenylphosphoryl azide (11.32 g.) was added dropwise to a solution of D,L-3-(N,N-diisopropylcarbamoyl)-2-norbornanecarboxylic acid (10.0 g.) in dry pyridine (32 ml.) and stirred at 80°C for 35 minutes to give D,L-3-(N,N-diisopropylcarbamoyl)-2-norbornylisocyanate (I.R.: 2270 $cm^{-1}$).

5-Fluorouracil (4.05 g.) was added to the solution and stirred at 80°C for 3 hours. The resultant mixture was evaporated under reduced pressure. The residue was dissolved in ethyl acetate. The precipitates partially formed during washing with dilute hydrochloric acid were filtered and washed with ethanol and diethyl ether. The ethyl acetate layer was washed with water, dried over magnesium sulfate, treated with activated charcoal, filtered and evaporated under reduced pressure to give crystals. The residue and the precipitates obtained above were combined and recrystallized from ethanol to give crystals of D,L-1-[N-[3-(N,N-diisopropylcarbamoyl)-2-norbornyl]carbamoyl]-5-fluorouracil (754 mg.). M.p. 201—202°C.

N.M.R. (DMSO-$d_6$) (ppm):

1.06 to 1.71 (20H, m), 2.47 (2H, m), 3.20 to 3.63 (2H, m), 4.07 to 4.47 (2H, m), 8.60 (1H, d, J=8Hz), 12.67 (1H, d, J=8Hz), 14.17 (1H, broad s)

Example 40

D,L-1-[N-(3-Ethoxycarbonyl-5-norbornen-2-yl)carbamoyl]-5-fluorouracil

Diphenylphosphoryl azide (14.4 g.) was added dropwise to a solution to D,L-5-norbornene-2,3-dicarboxylic acid monoethyl ester (10.0 g.) in dry pyridine (40 ml.) and stirred at 80°C for 50 minutes to give 3-ethoxycarbonyl -5-norbornen-2-yl isocyanate (I.R.: 2260 $cm^{-1}$).

5-Fluorouracil (6.19 g.) was added to the solution and stirred at 80°C for 2 hours. The resultant mixture was evaporated under reduced pressure. The residue was dissolved in ethyl acetate and washed with dilute hydrochloric acid and water. The ethyl acetate layer was dried over magnesium sulfate, treated with activated charcoal, filtered and evaporated. The residue was washed with isopropyl ether, triturated with ethanol and crystallized from ethanol to give D,L-1-[N-(3-ethoxycarbonyl-5-norbornen-2-yl)carbamoyl]-5-fluorouracil (1.67 g.) M.p. 150—152°C.

N.M.R (DMSO-$d_6$) (ppm):

1.08 (3H, t, J=7Hz), 1.46 (2H, m), 3.08 (2H, broad s), 3.36 (1H, dd, J=8Hz), J=3Hz), 3.96 (2h, quintet, J=7Hz), 4.80 (1H, double triplet, J=8Hz, J=3Hz), 6.12 (1H, m), 6.48 (1H, m), 8.40 (1H, d, J=7Hz), 9.08 (1H, d, J=8Hz).

Example 41

D,L-1-[N-(3-*tert*-Butoxycarbonyl-5-norbornen-2-yl)carbamoyl]-5-fluorouracil

Diphenylphosphoryl azide (37.47 g.) was added dropwise to a solution of D,L-3-*tert*-butoxycarbonyl-5-norbornene-2-carboxylic acid (32.46 g.) prepared in Example 37 in dry pyridine (115

ml.) and stirred at 100°C for 40 minutes to give 3-*tert*-butoxycarbonyl-5-norbornen-2-yl isocyanate (I.R.: 2260 $cm^{-1}$).

5-Fluorouracil (14.74 g.) was added to the solution and stirred at 100°C for 4 hours. The resultant mixture was evaporated under reduced pressure. The residue was dissolved in ethyl acetate and washed with dilute hydrochloric acid and water, dried over magnesium sulfate, treated with activated charcoal, filtered and concentrated under reduced pressure. The residue was washed with petroleum ether and triturated with diethyl ether. The precipitates were crystallized from ethanol to give D,L-1-N-(3-*tert*-butoxycarbonyl-5-norbornen-2-yl)carbamoyl]-5-fluorouracil (1.55 g.). M.p. 146°C.

N.M.R. (DMSO-$d_6$) (ppm):

1.4 to 1.93 (11H, m), 3.00 (2H, broad s), 3.27 (1H, s), 4.53 (1H, quintet, J=4Hz), 6.17 (1H, m), 6.50 (1H, m), 8.33 (1H, d, J=7Hz), 8.93 (1H, d, J=8Hz), 12.20 (1H, broad s)

Example 42

D,L-1-[N-(3-carboxy-5-norbornen-2-yl)carbamoyl]-5-fluorouracil

D,L-1-[N-(3-carboxy5-norbornen-2-yl)carbamoyl]-5-fluorouracil was prepared from D,L-1-[N-(3-*tert*-butoxycarbonyl-5-norbornen-2-yl)carbamoyl]-5-fluorouracil prepared in Example 41 substantially in the similar manner to that of 1-[N-(3-carboxypropyl)carbamoyl]-5-fluorouracil described in Example 32. M.p. 169°C.

N.M.R. (DMSO-$d_6$ (ppm):

1.33 to 1.97 (3H, m), 3.03 (2H, s), 4.57 (1H, quintet, J=4Hz), 6.13 (1H, m), 6.50 (1H, m), 8.33 (1H, d, J=7Hz), 8.93 (1H, d, J=8Hz), 12.20 (1H, d, J=4Hz).

Example 43

1-[N-(2-(5-Norbornen-2-yl)ethenyl]carbamoyl]-5-fluorouracil

Diphenylphosphoryl azide (12.1 g.) was added dropwise to a solution of 3-(5-norbornen-2-yl)acrylic acid (6.57 g.) in dry pyridine (30 ml.) and stirred at 80°C for 30 minutes to give 2-(5-norbornen-2-yl)ethenyl isocyanate (I.R.: 2260 $cm^{-1}$).

5-Fluorouracil (2.6 g.) was added to the solution and stirred at 100°C for 4 hours. The resultant mixture was evaporated under reduced pressure. The residue was dissolved in ethyl acetate and washed with water. The ethyl acetate layer was dried over magnesium sulfate, filtered and evaporated under reduced pressure. The residue was crystallized from ethanol to give 1-[N-[2-(5-norbornen-2-yl)ethenylcarbamoyl]-5-fluorouracil (0.3 g.). M.p. 130—132°C.

N.M.R. (DMSO-$d_6$) (ppm):

1.0 to 2.9 (7H, m), 5.5 to 6.3 (4H, m), 7.71 [nh]*a), 9.30 [h]*b) (1H, d, J=6Hz; d, J=8Hz), 8.35 (1H, d, J=7Hz) *a) [nh]; non-hydrogen-bonded *b) [h]; hydrogen-bonded.

Example 44

D,L-1-[N-(3-Isopropoxycarbonyl-5-norbornen-2-yl)carbamoyl]5-fluorouracil

Diphenylphosphoryl azide (24.5 g.) was added dropwise to a solution of D,L-5-norbornene-2,3-dicarboxylic acid monoisopropyl ester (20.0 g.) prepared in Example 36 in dry pyridine (100 ml.) and stirred for 40 minutes at 100°C to give a solution of D,L-3-isopropoxycarbonyl-5-norbornen-2-yl isocyanate [I.R: 2260 $cm^{-1}$].

5-Fluorouracil (12.8 g.) was added to the solution of D,L-3-isopropoxycarbonyl-5-norbornen-2-yl isocyanate and stirred at 100°C for 2 hours. The resultant mixture was evaporated to dryness under reduced pressure. The residue was dissolved in ethyl acetate, washed with dilute hydrochloric acid and water, dried over magnesium sulfate, treated with activated charcoal, filtered and concentrated to dryness under reduced pressure. The residue was dissolved in ethyl acetate, chromatographed on silica gel, and eluted with ethyl acetate, to give crystalline powders. Recrystallization from ethanol gave colorless crystals of D,L-1-[N-(3-isopropoxycarbonyl-5-norbornen-2-yl)carbamoyl]-5-fluorouracil (375 mg.). M.p. 148°C.

N.M.R. (DMSO-$d_6$) (ppm):

1.00(3H, d, J=7Hz), 1.13(3H, d, J=7 Hz), 1.43(2H, broad s.), 3.07 to 3.43(3H, m), 4.57 to 4.97(2H, m), 6.13(1H, m), 6.47(1H, m), 8.40(1H, d, J=7Hz), 9.07 (1H, d, J=9Hz), 12.27(1H, broad).

Example 45

D,L-1-[N-(3-Ethoxycarbonyl-5,6-isopropylidenedioxy-2-norbornyl)carbamoyl]-5-fluorouracil

D,L-5-Norbornene-2,3-dicarboxylic acid monoethyl ester (18.0 g.) was dissolved in a solution of potassium hydroxide (12.0 g.) in water (130 ml.) at —3°C. Potassium permanganate (14.9 g.) was portionwise added to the solution during 25 minutes below —3°C. To the solution was added a mixture of sulfur dioxide (12 g.) and 50% sulfuric acid (40 g.). The mixture was heated to the boiling point, cooled to the room temperature. After filtration of the precipitates formed, the filtrate was overlayed with ethyl acetate, adjusted with 10% hydrochloric acid to pH 1. The ethyl acetate layer was dried over magnesium sulfate, treated with activated charcoal, filtered and then concentrated to dryness under

reduced pressure to give oily D,L-5,6-dihydroxynorbornane-2,3-dicarboxylic acid monoethyl ester (11.2 g.).

N.M.R. ($D_2O$) (ppm):

1.23 (3H, t, J=7Hz), 1.37 (1H, m), 1.90 (1H, m), 2.50 (2H, m), 3.17 ((2H, m), 4.10 (2H, quintet, J=7Hz).

A solution of D,L-5,6-dihydroxynorbornane-2,3-dicarboxylic acid monoethyl ester (11.2 g.p, 2,2-dimethoxypropane (4.78 g) and p-toluenesulfonic acid (5 mg.) in dry N,N-dimethylformamide was refluxed for 2 hours under reduced pressure at 60—65°C. The resultant mixture was evaporated under reduced pressure. The residue was dissolved in aqueous sodium bicarbonate solution under ice-cooling, washed with ethyl acetate, treated with activated charcoal and then filtered. The solution was overlayed with ethyl acetate, adjusted to pH 3.5 with 10% hydrochloric acid under ice-cooling. The ethyl acetate layer was washed with saturated aqueous sodium chloride solution, dried over magnesium sulfate, treated with activated charcoal, filtered and concentrated woth dryness under reduced pressure to give oily D,L-5,6-isopropylidenedioxynorbornane-2,3-dicarboxylic acid monoethyl ester (9.29 g.).

N.M.R. ($CDCl_3$) (ppm):

1.10 to 1.43 (10H, m), 1.87 (1H, broad s), 2.60 (2H, broad s), 3.07 ((2H, m), 4.10 (2H, quintet, J=7Hz), 4.57 (2H, m).

Diphenylphosphoryl azide (6.85 g.) was added to a solution of D,L,-5,6-isopropylidenedioxy-norbornane-2,3,-dicarboxylic acid monoethyl ester in dry pyridine (22 ml.). The solution was stirred for 45 minutes at 90°C to give a solution comprising D,L-5,6-isopropylidenedioxy-3-ethoxycarbonyl-2-norbornyl isocyanate [I.R.; 2270 $cm^{-1}$].

5-Fluorouracil (2.71 g.) was added to the solution of D,L-5,6-isopropylidenedioxy-3-ethoxycarbonyl-2-norbornyl isocyanate and stirred at 90°C for 2 hours. The resultant solution was evaporated under reduced pressure. The residue was dissolved in ethyl acetate, washed with dilute hydrochloric acid and water, dried over magnesium sulfate, treated with activated charcoal, filtered and evaporated under reduced pressure. The residue was triturated with petroleum ether, to which ethanol was added, and filtered. The obtained powders were recrystallized from ethanol to give crystalline 1-[N-(3-ethoxycarbonyl-5,6-isopropylidenedioxy-2-norbornyl)carbamoyl]-5-fluorouracil (1.50 g.). M.p. 173—175°C.

N.M.R. ($CDCl_3$) (ppm):

1.13 to 1.43 (10H, m), 1.80 to 2.00 (1H, m), 2.73 (2H, broad s), 3.10 (double doublet, J=1 1Hz, J=5Hz), 4.00 to 4.60 (5H, m), 8.47 (1H, d, J=7Hz), 9.33 (1H, broad s), 10.10 (1 H, d, J=8Hz).

## Example 46

1-[N-(3-Methyladamantan-1-ylmethyl)carbamoyl] -5-fluorouracil

Diphenylphosphoryl azide (6.41 g.) was added dropwise to a solution of 3-methyladamantan-1-ylacetic acid (4.85 g.) in dry pyridine (20 ml.) and stirred at 100°C for 40 minutes to give 3-methyl-adamantan-1-ylmethyl isocyanate [I.R.: 2260 $cm^{-1}$]. 5-Fluorouracil (2.52 g.) was added to the solution and stirred at 100°C for 2 hours. The resultant mixture was evaporated under reduced pressure. The residue was dissolved in ethyl acetate and washed with dilute hydrochloric acid and water. Ethyl acetate layer was dried over magnesium sulfate, filtered and evaporated to give residue, which was recrystallized from ethanol to give colorless crystals of 1-[N-(3-methyladamantan-1-ylmethyl)carbamoyl]-5-fluorouracil. (984 mg.) M.p. 172°C.

N.M.R. (DMSO-$d_6$) (ppm):

0.77 (3H, s) 1.20 to 1.53 (12H, m), 2.00 (2H, broad s), 3.00 (2H, d, J=6Hz), 8.33 (1H, d, J=8Hz), 9.13 (2H, t, J=6Hz), 12.27 (1H, broad).

## Example 47

(+)-1-[N-(3-Pinanyl)carbamoyl]-5-fluorouracil

The reaction of (+)-pinane-3-carboxylic acid and diphenyl phosphoryl azide in dry pyridine provided a solution comprising(+)-3-pinanyl isocyanate [I.R.: 2260 $cm^{-1}$], which was reacted with 5-fluorouracil to provide (+)-1-[N-(3-pynanyl)carbamoyl]-5-fluorouracil substantially in the similar method to that of Example 45. M.p. 189°C.

N.M.R. (DMSO-$d_6$) (ppm):

1.00 to 1.24 (9H, m), 1.56 to 2.04 (5H, m), 2.32 to 2.64 (2H, m), 4.12 (1H, m), 8.36 (1H, d, J=8Hz), 9.12 (1H, d, J=10Hz).

## Example 48

1-[N-(3-Bromoadamantan-1-ylmethyl)carbamoyl] -5-fluorouracil

The reaction of 3-bromoadamantan-1-ylacetic acid and diphenyl phosphoryl azide in dry pyridine provided a solution comprising 3-bromoadamantan-1-ylmethyl isocyanate [I.R.: 2270 $cm^{-1}$], which was reacted with 5-fluorouracil to provide 1-[N-(3-bromoadamantan-1-ylmethyl)carbamoyl]-5-fluorouracil substantially by the similar method to that of Example 45. M.p. 185—186°C.

N.M.R. ($DMSO-d_6$) (ppm):

1.53 to 1.66(6H, m), 2.13(2H, s), 2.27(6H, s), 3.12(2H, d, J=6Hz), 8.43(1H, d, J=8Hz), 9.20(1H, t, J=6Hz), 12.37(1H, broad).

## Example 49

D,L-1-[N-(3-Ethoxycarbonylbicyclo[2.2.2]oct-2-yl)carbamoyl]-5-fluorouracil

Sodium (1.29 g.) was dissolved in ethanol (80 ml.) under heating. To the solution was added portionwise D,L-bicyclo[2.2.2]oct-2-ene-5,6-dicarboxylic anhydride (10.0 g.). The mixture was refluxed for 7 hours and evaporated under reduced pressure. The residue was dissolved in aqueous sodium bicarbonate solution, overlapped with ethyl acetate and adjusted to pH 3.0 with 10% hydrochloric acid. The ethyl acetate layer was washed with water, dried over magnesium sulfate, treated with activated charcoal, filtered and concentrated under reduced pressure to give crystals of D,L-6-ethoxycarbonylbicyclo[2.2.2]oct-2-en-5-carboxylic acid (12.61 g.).

N.M.R. ($DMSO-d_6$) (ppm):

1.12(5H, m), 1.56(2H, m), 2.76(2H, broad s), 2.96(2H, s), 3.92(2H, quintet, J=6Hz), 6.16(2H, m).

D,L-6-ethoxycarbonylbicyclo[2.2.2]oct-2-ene-5-carboxylic acid (3.00 g.) which was prepared above was dissolved in tetrahydrofuran (30 ml.), and hydrogenated over 10% palladium charcoal catalyst under hydrogen atmosphere of ordinary pressure at ambient temperature. The reaction mixture was filtered and the filtrate was concentrated in vacuo. The oily residue was made solidified and dried to give D,L-3-ethoxycarbonylbicyclo[2.2.2]octane-2-carboxylic acid (2.86 g.).

N.M.R. ($DMSO-d_6$) (ppm):

1.03 to 1.87 (13H, m), 2.87 (2H, s), 3.97 (2H, quintet, J=6Hz).

The reaction of 3-ethoxycarbonylbicyclo[2.2.2]octane-2-carboxylic acid prepared above and diphenyl phosphoryl azide in dry pyridine provide a solution comprising 3-ethoxycarbonylbicyclo[2.2.2]oct-2-yl isocyanate [I.R.: 2270 $cm^{-1}$], which was reacted with 5-fluorouracil to provide D,L-1-[N-(3-ethoxycarbonylbicyclo[2.2.2]oct-2-yl)carbamoyl]-5-fluorouracil (1.5 g.) substantially in the similar method to that of Example 45. M.p. 145—155°C (dec.).

N.M.R. ($DMSO-d_6$) (ppm):

1.10 (3H, s), 1.35 to 1.92 (10H, m), 3.98 (1H, d, J=10Hz), 4.00 (2H, quartet, J=7Hz), 4.33 ((1H, m), 8.33 (1H, d, J=7Hz), 9.75 (1H, d, J=8Hz), 10.40 (1H, broad s).

## Example 50

1-[N-(2-Adamantylmethyl)carbamoyl]-5-fluorouracil

The reaction of 2-adamantylacetic acid and diphenyl phosphoryl azide in dry pyridine provided a solution comprising 2-adamantylmethyl isocyanate [I.R.: 2260 $cm^{-1}$]; which was reacted with 5-fluorouracil to provide 1-[N-(2-adamantylmethyl)carbamoyl]-5-fluorouracil substantially in the similar method to that of Example 45. M.p. 274—278°C.

N.M.R. ($DMSO-d_6$) (ppm):

1.4 to 1.9 (15H, m), 3.38 (2H, dd, J=7Hz, J=8Hz), 9.38 (1H, d, J=8Hz, 9.78 (1H, t, J=6Hz).

## Example 51

D,L-1-[N-(2-Methoxycarbonylnorborn-3-yl)carbamoyl]-5-fluorouracil

A solution of ethyl chlorocarbonate (60.8 g.) in tetrahydrofuran (50 ml.) was added dropwise to a solution of D,L-norbornan-2,3-dicarboxylic acid monomethyl ester (100 g.) which was prepared by the method described in Example 24 and trimethylamine (51.6 g.) with stirring at −10°C, liberating triethylamine hydrochloride as a milky precipitate. The mixture was further stirred for one hour at the same temperature. A solution of sodium azide (36.5 g.) in water (200 ml.) was added to the mixture. The clear solution was further stirred for one hour at 0°C, liberating another milky precipitate, and then evaporated in vacuo to remove tetrahydrofuran. To the residue was added water (1000 ml.) to dissolve the milky precipitate. The mixture was extracted with ethyl acetate (1000 ml x 2). The ethyl acetate layer was separated and washed with water, dried over magnesium sulfate and filtered.

The filtrate was stirred for two hours at 55°C, and evaporated in vacuo to give oily residue (93.6 g.). The residue was distilled under reduced pressure to give a colorless liquid of D,L-2-methoxycarbonylnorborn-3-yl isocyanate (70.6 g.). B.p. 90—100°C/0.2 mm Hg.

I.R. (Nujol): 1270 $cm^{-1}$.

A solution of 5-fluorouracil (26.7 g.) and D,L-2-methoxycarbonylnorborn-3-yl isocyanate (58.7 g.) in pyridine (110 ml.) was heated with stirring for 1 hour at 90°C and evaporated in vacuo. The residue was triturated with isopropyl ether (100 ml x 2) and water (100 ml x 3). The substance was filtered, dried and recrystallized with ethanol to give D,L-1-[N-(2-methoxycarbonylnorborn-3-yl)carbamoyl]-5-fluorouracil (53.5 g.). M.p. 164—165°C.

N.M.R. ($DMSO-d_6$) (ppm):

1.5(6H, broad s), 2.33 to 2.67 (2H), 2.9 to 3.4 (1H, m), 3.6(3H, s), 4.07 to 4.6 (1H, m), 8.4(1H, d, J=8Hz), 10.07 (1H, d, J=8Hz), 12.3(1H, broad s).

Example 52

D,L-1-[N-[3-(N-*tert*-Butoxycarbonylamino)norborn-2-yl]carbamoyl]-5-fluorouracil

A solution of D,L-2-methoxycarbonylnorborn-3-yl isocyanate (1.16 g.) prepared by the method described in Example 51 in ethanol (10 ml.) was heated under reflux for 3 hours, and evaporated in vacuo to give methyl D,L-3-ethoxycarbonylaminonorbornane-2-carboxvlate (1.37 g.) as a oily residue.

I.R. (Nujol): 3400, 1715 $cm^{-1}$.

Methyl D,L-3-ethoxycarbonylaminonorbornan-2-carboxylate (1.34 g.) in 40% aqueous sodium hydroxide solution (5 ml.) was heated under reflux with stirring for 1 hour, liberating precipitate. The precipitate was filtered and dissolved in a small amount of water. The solution was adjusted to pH 3.5 with concentrated hydrochloric acid, liberating precipitate. The precipitate was filtered, washed with water and dried over phosphorus pentoxide to give 3-aminonorbornane-2-carboxylic acid (0.8 g.).

I.R. (Nujol): 1620—1560 $cm^{-1}$.

To a solution of 3-aminonorbornane-2-carboxylic acid (12.6 g.) in a mixture of water (100 ml.) and dioxane (100 ml.) were added triethylamine (12.2 g) and 2-tert-butoxycarbonyloxyimino-2-phenylacetonitrile (16.2 g.). The mixture was stirred for 5 hours at ambient temperature and washed with ethyl acetate at pH 10 (each 250 ml., 2 times). The aqueous layer was adjusted to pH 2 with 10% aqueous hydrochloric acid. The acidified solution was extracted with ethyl acetate. The organic layer was dried over magnesium sulfate and filtered. The filtrate was evaporated to dryness under reduced pressure. The residue was pulverized with isopropyl ether to give 3-(N-*tert*-butoxycarbonylamino)norbornane-2-carboxylic acid (10.5 g.).

I.R. (Nujol):

3250, 2600—2500, 1710, 1690, 1650 $cm^{-1}$.

N.M.R. ($CDCl_3$) (ppm):

1.25—1.75 (15H, m), 2.43 (1H, broad s), 2.66 (1H, broad s), 3.93 (1H, m).

The reaction of 3-(N-*tert*-butoxycarbonylamino)norbornane-2-carboxylic acid (2 g.) and diphenylphosphoryl azide in pyridine provided a solution comprising D,L-N-[3-(N-*tert*-butoxycarbonylamino)norborn-2-yl]carbamoyl isocyanate, which was reacted with 5-fluorouracil to provide D,L-1-[N-[3-(N-*tert*-butoxycarbonylamino)norborn-2-yl]carbamoyl]-5-fluorouracil substantially in the similar method to that of Example 33.

I.R. (Nujol):

3300—3250, 1730, 1705, 1670 $cm^{-1}$.

N.M.R. (DMSO-$d_6$) (ppm):

1.0—1.5 (15H, m), 2.20 (1H, broad s), 2.62 (1H, broad s), 3.70 (2H, m), 8.27 (1H, d, J=8Hz), 9.23 (1H, d, J=8Hz).

Example 53

D,L-1-[N-(3-Amino-2-norbornyl)- carbamoyl]-5-fluorouracil

D,L-1-[N-[3-(N-*tert*-Butoxycarbonylamino)norborn-2-yl]carbamoyl-5-fluorouracil (2.5 g.) was dissolved in a mixture of methanol (30 ml.) and tetrahydrofuran (10 ml.).

Dry hydrogen chloride gas was passed into the above solution. The reaction mixture was evaporated to dryness at 35°C in vacuo. The residue was washed with diethyl ether and pulverized in a mixture of methanol and diethylether to give D,L-1-[N-(3-amino-2-norbornyl)carbamoyl]-5-fluorouracil (0.85 g.).

I.R. (Nujol):

3250—3150, 1730, 1690, 1660 $cm^{-1}$.

N.M.R. (DMSO-$d_6$) (ppm):

1.50 (8H, m), 3.50 (2H, m), 8.43 (1H, d, J=8Hz), 8.70 (2H, broad s), 9.25 (1H, d, J=8Hz), 13.93 (1H, broad s).

**Claims for the Contracting States: BE, CH, DE, FR, GB, IT, LU, NL, SE**

1. A compound of the formula:

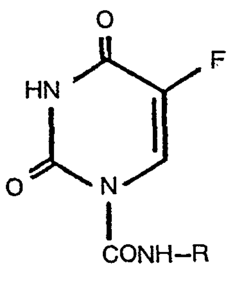

wherein R is a substituted or unsubstituted bridged alicyclic radical selected from substituted and unsubstituted norbornyl, norbornenyl, bicyclo[2.2.2]octyl, bicyclo[2.2.2]oct-2-enyl, bicyclo[3.1.1]heptyl

and adamantyl, the substituent(s) of said bridged alicyclic radical being selected from ($C_1$—$C_6$)alkyl, carboxy, esterified carboxy, ($C_1$—$C_6$)alkylidenedioxy, N,N-di-($C_1$—$C_6$)alkylcarbamoyl, amino and protected amino; or is a substituted ($C_1$—$C_6$)alkyl radical, the substituent being selected from substituted and unsubstituted bridged alicyclic radicals selected from substituted and unsubstituted norbornyl and adamantyl, the substituent of said bridged alicyclic radical being selected from ($C_1$—$C_6$)alkyl and halogen; or is a substituted ($C_1$—$C_6$)alkyl radical, the substituent being selected from amino, protected amino, ($C_1$—$C_6$)alkoxy, acyl, ($C_1$—$C_6$)alkylthio, cyano($C_1$—$C_6$)alkylthio, phenylthio, pyridyl, thienyl, 1,2-dithiolanyl, 2,5-dioxopyrrolidinyl, carboxy and esterified carboxy, with the proviso that when the substituent of the substituted ($C_1$—$C_6$)alkyl radical is carboxy or esterified carboxy, the alkyl radical contains 3 to 6 carbon atoms; or is a trans,trans-penta-1,3-dienyl radical; or is a substituted ($C_2$—$C_6$) alkenyl radical, the substituent being selected from norbornenyl, ($C_1$—$C_6$)alkoxy-substituted phenyl, furyl and thienyl; or is a tetrahydrothienyl radical; and the pharmaceutically acceptable salts thereof.

2. A compound according to claim 1, wherein R is a substituted or unsubstituted bridged alicyclic radical selected from norbornyl, norbornenyl, bicyclo[2.2.2]octyl, bicyclo[2.2.2]oct-2-enyl, bicyclo[3.1.1]heptyl and adamantyl, the substituent(s) of said bridged alicyclic radical being selected from ($C_1$—$C_6$)alkyl, carboxy, esterified carboxy, ($C_1$—$C_6$)alkylidenedioxy, N,N-di($C_1$—$C_6$)alkylcarbamoyl, amino and protected amino.

3. A compound according to claim 2, wherein R is a bridged alicyclic radical selected from norbornyl, norbornenyl and adamantyl.

4. A compound according to claim 3, wherein R is norbornyl.

5. A compound according to claim 4, wherein R is 2-norbornyl.

6. A compound according to claim 3, wherein R is adamantyl.

7. A compound according to claim 6, wherein R is 1-adamantyl.

8. A compound according to claim 2, wherein R is a bridged alicyclic radical selected from norbornyl, norbornenyl, bicyclo[2.2.2]octyl, bicyclo[2.2.2]oct-2-enyl, bicyclo[3.1.1]heptyl and adamantyl, said bridged alicyclic radical being substituted by a substituent selected from ($C_1$—$C_6$) alkyl, carboxy, esterified carboxy, ($C_1$—$C_6$) alkylidenedioxy, N,N-di-($C_1$—$C_6$)alkylcarbamoyl, amino and protected amino.

9. A compound according to claim 8, wherein R is carboxynorbornyl.

10. A compound according to claim 9, wherein R is 3-carboxy-2-norbornyl.

11. A compound according to claim 8, wherein R is esterified carboxynorbornyl.

12. A compound according to claim 11, wherein R is ($C_1$—$C_6$)alkoxycarbonylnorbornyl.

13. A compound according to claim 12, wherein R is methoxycarbonylnorbornyl.

14. A compound according to claim 13, wherein R is 3-methoxycarbonyl-2-norbornyl.

15. A compound according to claim 12, wherein R is ethoxycarbonylnorbornyl.

16. A compound according to claim 15, wherein R is 3-ethoxycarbonyl-2-norbornyl.

17. A compound according to claim 12, wherein R is isopropoxycarbonylnorbornyl.

18. A compound according to claim 17, wherein R is 3-isopropoxycarbonyl-2-norbornyl.

19. A compound according to claim 8, wherein R is norbornyl, substituted by ($C_1$—$C_6$)alkoxycarbonyl and ($C_1$—$C_6$) alkylidenedioxy.

20. A compound according to claim 8, wherein R is N,N-di-($C_1$—$C_6$)alkylcarbamoylnorbornyl.

21. A compound according to claim 8, wherein R is aminonorbornyl.

22. A compound according to claim 8, wherein R is protected aminonorbornyl.

23. A compound according to claim 8, wherein R is carboxynorbornenyl.

24. A compound according to claim 8, wherein R is esterified carboxynorbornenyl.

25. A compound according to claim 24, wherein R is ($C_1$—$C_6$)alkoxycarbonylnorbornenyl.

26. A compound according to claim 8, wherein R is bicyclo[3.1.1]heptyl substituted by at least one ($C_1$—$C_6$) alkyl radical.

27. A compound according to claim 26, wherein R is pinanyl.

28. A compound according to claim 8, wherein R is esterified carboxybicyclo[2.2.2]octyl.

29. A compound according to claim 28, wherein R is ($C_1$—$C_6$)alkoxycarbonylbicyclo[2.2.2]octyl.

30. A compound according to claim 1, wherein R is a substituted ($C_1$—$C_6$)alkyl radical, the substituent being a substituted or unsubstituted bridged alicyclic radical selected from norbornyl, and adamantyl, the substituent of said bridged alicyclic radical being selected from ($C_1$—$C_6$)alkyl and halogen.

31. A compound according to claim 30, wherein R is a substituted ($C_1$—$C_6$)alkyl radical, the substituent being a bridged alicyclic radical selected from norbornyl and adamantyl.

32. A compound according to claim 31, wherein R is norbornyl($C_1$—$C_6$)alkyl.

33. A compound according to claim 31, wherein R is adamantyl($C_1$—$C_6$)alkyl.

34. A compound according to claim 30, wherein R is a substituted ($C_1$—$C_6$)alkyl radical, the substituent being adamantyl, which is substituted by ($C_1$—$C_6$)alkyl or halogen.

35. A compound according to claim 34, wherein R is ($C_1$—$C_6$)alkyladamantyl($C_1$—$C_6$)alkyl.

36. A compound according to claim 34, wherein R is haloadamantyl($C_1$—$C_6$)alkyl.

37. A compound according to claim 1, wherein R is amino($C_1$—$C_6$)alkyl, protected

amino($C_1$—$C_6$)alkyl, ($C_1$—$C_6$)alkoxy($C_1$—$C_6$)alkyl, acyl($C_1$—$C_6$)alkyl, ($C_1$—$C_6$)alkylthio($C_1$—$C_6$)alkyl, cyano($C_1$—$C_6$)alkylthio($C_1$—$C_6$)alkyl, phenyltio($C_1$—$C_6$)alkyl, pyridyl($C_1$—$C_6$)alkyl, thienyl($C_1$—$C_6$)-alkyl, 1,2-dithiolanyl($C_1$—$C_6$)alkyl, 2,5-dioxopyrrolidinyl($C_1$—$C_6$)alkyl, carboxy($C_3$—$C_6$)alkyl or esterified carboxy($C_3$—$C_6$)alkyl.

38. A compound according to claim 1, wherein R is trans,trans-penta-1,3-dienyl.

39. A compound according to claim 1, wherein R is a ($C_2$—$C_6$)alkenyl, substituted by norbornenyl.

40. A compound according to claim 1, wherein R is ($C_1$—$C_6$)alkoxy-substituted phenyl($C_2$—$C_6$)alkenyl, furyl($C_2$—$C_6$)alkenyl or thienyl($C_2$—$C_6$)alkenyl.

41. A compound according to claim 1, wherein R is tetrahydrothienyl.

42. A process for preparing a compound according to claim 1, which comprises
(1) reacting 5-fluorouracil of the formula:—

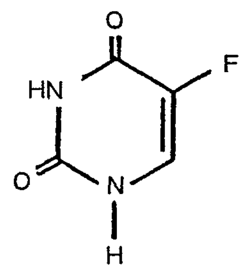

with an isocyanate of the formula:—

$$R_a—N=C=O$$

wherein $R_a$ is a substituted or unsubstituted bridged alicyclic radical selected from substituted and unsubstituted norbornyl, norbornenyl, bicyclo[2.2.2]octyl, bicyclo[2.2.2]oct-2-enyl, bicyclo[3.1.1]heptyl and adamantyl, the substituent(s) of said bridged alicyclic radical being selected from ($C_1$—$C_6$)alkyl, esterified carboxy, ($C_1$—$C_6$)alkylidenedioxy, N,N-di($C_1$—$C_6$)alkylcarbamoyl and protected amino; or is a substituted ($C_1$—$C_6$)alkyl radical, the substituent being selected from substituted and unsubstituted bridged alicyclic radicals selected from substituted and unsubstituted norbornyl, and adamantyl, the substituent of said bridged alicyclic radical being selected from ($C_1$—$C_6$)alkyl and halogen; or is a substituted ($C_1$—$C_6$)alkyl, the substituent being selected from protected amino, ($C_1$—$C_6$)alkoxy, acyl, ($C_1$—$C_6$)alkylthio, cyano($C_1$—$C_6$)alkylthio, phenylthio, pyridyl, thienyl, 1,2-dithiolanyl, 2,5-dioxo-pyrrolidinyl and esterified carboxy, with the proviso that, when the substituent of the substituted ($C_1$—$C_6$)alkyl radical is esterified carboxy, the alkyl radical contains 3 to 6 carbon atoms; or is trans, trans-penta-1,3-dienyl; or is a substituted ($C_2$—$C_6$)alkenyl radical, the substituent being selected from norbornenyl, ($C_1$—$C_6$)alkoxysubstituted phenyl, furyl and thienyl; or is tetrahydrothienyl, to give a 5-fluorouracil derivative of the formula:—

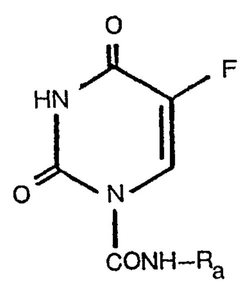

wherein $R_a$ has the same meaning as above; or
(2) hydrolysing a compound of the formula:

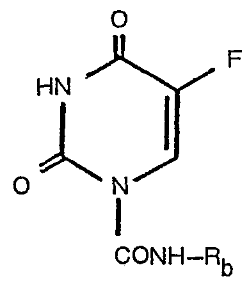

wherein $R_b$ is a bridged alicyclic radical containing a protected amino group, the bridged alicyclic radical being selected from norbornyl, norbornenyl, bicyclo[2.2.2]octyl, bicyclo[2.2.2]oct-2-enyl, bicyclo[3.1.1]heptyl and adamantyl; or is a substituted ($C_1$—$C_6$)alkyl radical, the substituent being a protected amino group; to give a 5-fluorouracil derivative of the formula:—

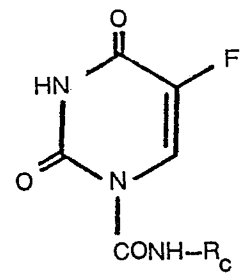

wherein $R_c$ is a bridged alicyclic radical containing an amino group, the bridged alicyclic radical being selected from norbornyl, norbornenyl, bicyclo[2.2.2]octyl, bicyclo[2.2.2]oct-2-enyl, bicyclo[3.1.1]-heptyl and adamantyl; or is a substituted ($C_1$—$C_6$)alkyl radical, the substituent being an amino group; or (3) hydrolysing a compound of the formula:—

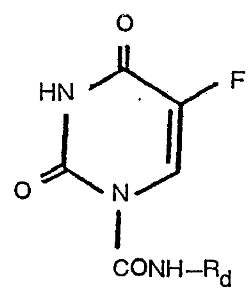

wherien $R_d$ is a bridged alicyclic radical containing an esterified carboxy group, the bridged alicyclic radical being selected from norbornyl, norbornenyl, bicyclo[2.2.2]octyl, bicyclo[2.2.2]oct-2-enyl, bicyclo[3.1.1]heptyl and adamantyl; or is a substituted ($C_1$—$C_6$)alkyl radical, the substituent being an esterified carboxy group, to give a 5-fluorouracil derivative of the formula:—

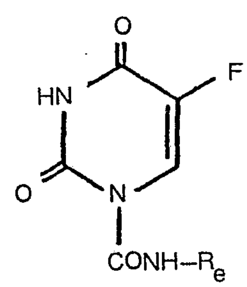

wherein $R_e$ is a bridged alicyclic radical containing a carboxy group, the bridged alicyclic radical being selected from norbornyl, norbornenyl, bicyclo[2.2.2]octyl, bicyclo[2.2.2]oct-2-enyl, bicyclo[3.1.1]heptyl and adamantyl; or is a substituted ($C_1$—$C_6$)alkyl radical, the substituent being a carboxy group.

43. A pharmaceutical composition comprising a compound according to claim 1 and/or a pharmaceutically acceptable salt thereof, in association with a pharmaceutically, substantially non-toxic carrier or excipient.

**Claims for the Contracting State AT:**

1. Process for the preparation of a compound of the formula:

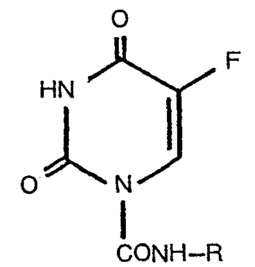

wherein R is a substituted or unsubstituted bridged alicyclic radical selected from substituted and unsubstituted norbornyl, norbornenyl, bicyclo[2.2.2]octyl, bicyclo[2.2.2]oct-2-enyl, bicyclo[3.1.1]heptyl and adamantyl, the substituent(s) of said briged alicyclic radical being selected from $(C_1—C_6)$alkyl, carboxy, esterified carboxy, $(C_1—C_6)$alkylidenedioxy, N,N-di-$(C_1—C_6)$alkylcarbamoyl, amino and protected amino; or is a substituted $(C_1—C_6)$alkyl radical, the substituent being selected from substituted and unsubstituted bridged alicyclic radicals selected from substituted and unsubstituted norbornyl and adamantyl, the substituent of said bridged alicyclic radical being selected from $(C_1—C_6)$alkyl and halogen; or is a substituted $(C_1—C_6)$alkyl radical, the substituent being selected from amino, protected amino, $(C_1—C_6)$alkoxy, acyl, $(C_1—C_6)$alkylthio, cyano$(C_1—C_6)$alkylthio, phenylthio, pyridyl, thienyl, 1,2-dithiolanyl, 2,5-dioxopyrrolidinyl, carboxy and esterified carboxy, with the proviso that when the substituent of the substituted $(C_1—C_6)$alkyl radical is carboxy or esterified carboxy, the alkyl radical contains 3 to 6 carbon atoms; or is a trans,trans-penta-1,3-dienyl radical; or is a substituted $(C_2—C_6)$ alkenyl radical, the substituent being selected from norbornenyl, $(C_1—C_6)$alkoxy-substituted phenyl, furyl and thienyl; or is a tetrahydrothienyl radical; and the pharmaceutically acceptable salts thereof which comprises

(1) reacting 5-fluorouracil of the formula:—

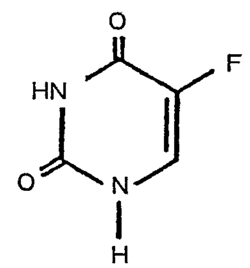

with an isocyanate of the formula:—

$R_a$—N=C=O

wherein $R_a$ is a substituted or unsubstituted bridged alicyclic radical selected from substituted and unsubstituted norbornyl, norbornenyl, bicyclo[2.2.2]octyl, bicyclo[2.2.2]oct-2-enyl, bicyclo[3.1.1]heptyl and adamantyl, the substituent(s) of said bridged alicyclic radical being selected from $(C_1—C_6)$alkyl, esterified carboxy, $(C_1—C_6)$alkylidenedioxy, N,N-di$(C_1—C_6)$alkylcarbamoyl and protected amino; or is a substituted $(C_1—C_6)$alkyl radical, the substituent being selected from substituted and unsubstituted bridged alicyclic radicals selected from substituted and unsubstituted norbornyl, and adamantyl, the substituent of said bridged alicyclic radical being selected from $(C_1—C_6)$alkyl and halogen; or is a substituted $(C_1—C)$alkyl, the substituent being selected from protected amino, $(C_1—C_6)$alkoxy, acyl, $(C_1—C_6)$alkylthio, cyano$(C_1—C_6)$alkylthio, phenylthio, pyridyl, thienyl, 1,2-dithiolanyl, 2,5-dioxo-pyrrolidinyl and esterified carboxy, with the proviso that, when the substituent of the substituted $(C_1—C_6)$alkyl radical is esterified carboxy, the alkyl radical contains 3 to 6 carbon atoms; or is trans, trans-penta-1,3-dienyl; or is a substituted $(C_2—C_6)$alkenyl radical, the substituent being selected from norbornenyl, $(C_1—C_6)$alkoxysubstituted phenyl, furyl and thienyl; or is tetrahydrothienyl, to give a 5-fluorouracil derivative of the formula:—

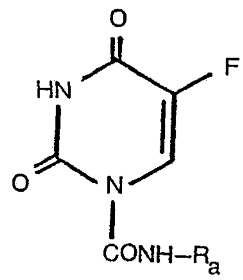

wherein $R_a$ has the same meaning as above; or
(2) hydrolysing a compound of the formula:

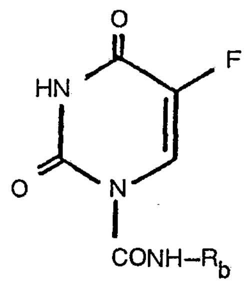

wherein $R_b$ is a bridged alicyclic radical containing a protected amino group, the bridged alicyclic radical being selected from norbornyl, norbornenyl, bicyclo[2.2.2]octyl, bicyclo[2.2.2]oct-2-enyl, bicyclo[3.1.1]heptyl and adamantyl; or is a substituted $(C_1$—$C_6)$alkyl radical, the substituent being a protected amino group; to give a 5-fluorouracil derivative of the formula:—

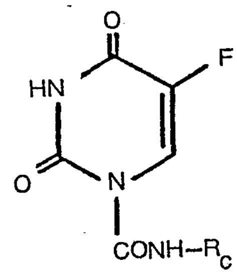

wherein $R_c$ is a bridged alicyclic radical containing an amino group, the bridged alicyclic radical being selected from norbornyl, norbornenyl, bicyclo[2.2.2]octyl, bicyclo[2.2.2]oct-2-enyl, bicyclo[3.1.1]-heptyl and adamantyl; or is a substituted $(C_1$—$C_6)$alkyl radical, the substituent being an amino group; or
(3) hydrolysing a compound of the formula:—

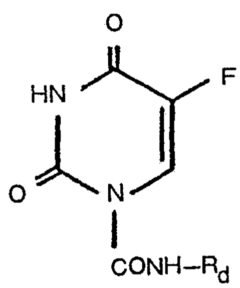

wherein $R_d$ is a bridged alicyclic radical containing an esterified carboxy group, the bridged alicyclic radical being selected from norbornyl, norbornenyl, bicyclo[2.2.2]octyl, bicyclo[2.2.2]oct-2-enyl, bicyclo[3.1.1]heptyl and adamantyl; or is a substituted $(C_1$—$C_6)$alkyl radical, the substituent being an esterified carboxy group, to give a 5-fluorouracil derivative of the formula:—

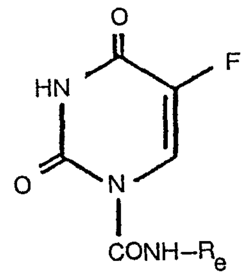

wherein $R_e$ is a bridged alicyclic radical containing a carboxy group, the bridged alicyclic radical being selected from norbornyl, norbornenyl, bicyclo[2.2.2]octyl, bicyclo[2.2.2]oct-2-enyl, bicyclo[3.1.1]heptyl and adamantyl; or is a substituted ($C_1$—$C_6$)alkyl radical, the substituent being a carboxy group.

2. Process according to claim 1, wherein R is a substituted or unsubstituted bridged alicyclic radical selected from norbornyl, norbornenyl, bicyclo[2.2.2]octyl, bicyclo[2.2.2]oct-2-enyl, bicyclo[3.1.1]heptyl and adamantyl, the substituent(s) of said bridged alicyclic radical being selected from ($C_1$—$C_6$)alkyl, carboxy, esterified carboxy, ($C_1$—$C_6$)alkylidenedioxy, N,N-di($C_1$—$C_6$)alkylcarbamoyl, amino and protected amino.

3. Process according to claim 2, wherein R is a bridged alicyclic radical selected from norbornyl, norbornenyl and adamantyl.

4. Process according to claim 3, wherein R is norbornyl.

5. Process according to claim 4, wherein R is 2-norbornyl.

6. Process according to claim 3, wherein R is adamantyl.

7. Process according to claim 6, wherein R is 1-adamantyl.

8. Process according to claim 2, wherein R is a bridged alicyclic radical selected from norbornyl, norbornenyl, bicyclo[2.2.2]octyl, bicyclo[2.2.2]oct-2-enyl, bicyclo[3.1.1]heptyl and adamantyl, said bridged alicyclic radical being substituted by a substituent selected from ($C_1$—$C_6$) alkyl, carboxy, esterified carboxy, ($C_1$—$C_6$) alkylidenedioxy, N,N-di-($C_1$—$C_6$)alkylcarbamoyl, amino and protected amino.

9. Process according to claim 8, wherein R is carboxynorbornyl.

10. Process according to claim 9, wherein R is 3-carboxy-2-norbornyl.

11. Process according to claim 8, wherein R is esterified carboxynorbornyl.

12. Process according to claim 11, wherein R is ($C_1$—$C_6$)alkoxycarbonylnorbornyl.

13. Process according to claim 12, wherein R is methoxycarbonylnorbornyl.

14. Process according to claim 13, wherein R is 3-methoxycarbonyl-2-norbornyl.

15. Process according to claim 12, wherein R is ethoxycarbonylnorbornyl.

16. Process according to claim 15, wherein R is 3-ethoxycarbonyl-2-norbornyl.

17. Process according to claim 12, wherein R is isopropoxycarbonylnorbornyl.

18. Process according to claim 17, wherein R is 3-isopropoxycarbonyl-2-norbornyl.

19. Process according to claim 8, wherein R is norbornyl, substituted by ($C_1$—$C_6$)alkoxycarbonyl and ($C_1$—$C_6$) alkylidenedioxy.

20. Process according to claim 8, wherein R is N,N-di-($C_1$—$C_6$)alkylcarbamoylnorbornyl.

21. Process according to claim 8, wherein R is aminonorbornyl.

22. Process according to claim 8, wherein R is protected aminonorbornyl.

23. Process according to claim 8, wherein R is carboxynorbornenyl.

24. Process according to claim 8, wherein R is esterified carboxynorbornenyl.

25. Process according to claim 24, wherein R is ($C_1$—$C_6$)alkoxycarbonylnorbornenyl.

26. Process according to claim 8, wherein R is bicyclo[3.1.1]heptyl substituted by at least one ($C_1$—$C_6$) alkyl radical.

27. Process according to claim 26, wherein R is pinanyl.

28. Process according to claim 8, wherein R is esterified carboxybicyclo[2.2.2]octyl.

29. Process according to claim 28, wherein R is ($C_1$—$C_6$)alkoxycarbonylbicyclo[2.2.2]octyl.

30. Process according to claim 1, wherein R is a substituted ($C_1$—$C_6$)alkyl radical, the substituent being a substituted or unsubstituted bridged alicyclic radical selected from norbornyl, and adamantyl, the substituent of said bridged alicyclic radical being selected from ($C_1$—$C_6$)alkyl and halogen.

31. Process according to claim 30, wherein R is a substituted ($C_1$—$C_6$)alkyl radical, the substituent being a bridged alicyclic radical selected from norbornyl and adamantyl.

32. Process according to claim 31, wherein R is norbornyl($C_1$—$C_6$)alkyl.

33. Process according to claim 31, wherein R is adamantyl($C_1$—$C_6$)alkyl.

34. Process according to claim 30, wherein R is a substituted ($C_1$—$C_6$)alkyl radical, the substituent being adamantyl, which is substituted by ($C_1$—$C_6$) alkyl or halogen.

35. Process according to claim 34, wherein R is ($C_1$—$C_6$)alkyladamantyl($C_1$—$C_6$)alkyl.

36. Process according to claim 34, wherein R is haloadamantyl($C_1$—$C_6$)alkyl.

37. Process according to claim 1, wherein R is amino($C_1$—$C_6$)alkyl, protected amino($C_1$—$C_6$)alkyl,

$(C_1-C_6)$alkoxy$(C_1-C_6)$alkyl, acyl$(C_1-C_6)$alkyl, $(C_1-C_6)$alkylthio$(C_1-C_6)$alkyl, cyano$(C_1-C_6)$alkylthio-$(C_1-C_6)$alkyl, phenylthio$(C_1-C_6)$alkyl, pyridyl$(C_1-C_6)$alkyl, thienyl$(C_1-C_6)$alkyl, 1,2-dithiolanyl-$(C_1-C_6)$alkyl, 2,5-dioxopyrrolidinyl$(C_1-C_6)$alkyl, carboxy$(C_1-C_6)$alkyl or esterified carboxy$(C_3-C_6)$-alkyl.

38. Process according to claim 1, wherein R is trans,trans-penta-1,3-dienyl.

39. Process according to claim 1, wherein R is a $(C_2-C_6)$alkenyl, substituted by norbornenyl.

40. Process according to claim 1, wherein R is $(C_1-C_6)$alkoxy-substituted phenyl$(C_2-C_6)$alkenyl, furyl$(C_2-C_6)$alkenyl or thienyl$(C_2-C_6)$alkenyl.

41. Process according to claim 1, wherein R is tetrahydrothienyl.

**Patentansprüche für die Vertragsstaaten: BE, CH, DE, FR, GB, IT, LU, NL, SE**

1. Verbindung der Formel

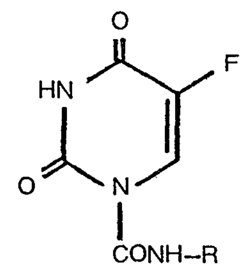

worin R darstellt einen substituierten oder unsubstituierten, über eine Brücke gebundenen alicyclischen Rest, ausgewählt aus substituiertem und unsubstituiertem Norbornyl, Norbornenyl, Bicyclo[2.2.2]octvl, Bicyclo[2.2.2]oct-2-enyl, Bicyclo[3.1.1]heptyl und Adamantyl, wobei der (die) Substituent(en) des über eine Brücke gebundenen alicyclischen Restes ausgewählt wird (werden) aus $(C_1-C_6)$Alkyl, Carboxy, verestertem Carboxy, $(C_1-C_6)$Alkylidendioxy, N,N-Di-$(C_1-C_6)$alkylcarbamoyl, Amino und geschütztem Amino; oder einen substituierten $(C_1-C_6)$Alkylrest, wobei der Substituent ausgewählt wird aus substituierten und unsubstituierten, über eine Brücke gebundenen alicyclischen Resten, ausgewählt aus substituiertem und unsubstituiertem Norbornyl und Adamantyl, wobei der Substituent des über eine Brücke gebundenen alicyclischen Restes ausgewählt wird aus $(C_1-C_6)$Alkyl und Halogen; oder einen substituierten $(C_1-C_6)$Alkylrest, wobei der Substituent ausgewählt wird aus Amino, geschütztem Amino, $(C_1-C_6)$Alkoxy, Acyl, $(C_1-C_6)$Alkylthio, Cyano-$(C_1-C_6)$alkylthio, Phenylthio, Pyridyl, Thienyl, 1,2-Di-thiolanyl, 2,5-Dioxopyrrolidinyl, Carboxy and verestertem Carboxy, mit der Maßgabe, daß dann, wenn der Substituent des substituierten $(C_1-C_6)$Alkylrestes Carboxy oder verestertes Carboxy ist, der Alkylrest 3 bis 6 Kohlenstoffatome enthält; oder einen trans, trans-Penta-1,3-dienylrest; oder einen substituierten $(C_2-C_6)$Alkenylrest, wobei der Substituent ausgewählt wird aus Norbornenyl, $(C_1-C_6)$Alkoxy-substituiertem Phenyl, Furyl und Thienyl; oder einen Tetrahydrothienylrest; sowie die pharmazeutisch akzeptablen Salze davon.

2. Verbindung nach Anspruch 1, worin R darstellt einen substituierten oder unsubstituierten, über eine Brücke gebundenen alicyclischen Rest, ausgewählt aus Norbornyl, Norbornenyl, Bicyclo[2.2.2]octyl, Bicyclo[2.2.2]oct-2-enyl, Bicyclo[3.1.1]heptyl und Adamantyl, wobei der (die) Substituent(en) des über eine Brücke gebundenen alicyclischen Restes ausgewählt wird (werden) aus $(C_1-C_6)$Alkyl, Carboxy, verestertem Carboxy, $(C_1-C_6)$Alkylidendioxy, N,N-Di-$(C_1-C_6)$alkyl-carbamoyl, Amino und geschütztem Amino.

3. Verbindung nach Anspruch 2, worin R einen über eine Brücke gebundenen alicyclischen Rest darstellt, der ausgewählt wird aus Norbornyl, Norbornenyl und Adamantyl.

4. Verbindung nach Anspruch 3, worin R Norbornyl darstellt.

5. Verbindung nach Anspruch 4, worin R 2-Norbornyl darstellt.

6. Verbindung nach Anspruch 3, worin R Adamantyl darstellt.

7. Verbindung nach Anspruch 6, worin R 1-Adamantyl darstellt.

8. Verbindung nach Anspruch 2, worin R einen über eine Brücke gebundenen alicyclischen Rest darstellt, der ausgewählt wird aus Norbornyl, Norbornenyl, Bicyclo[2.2.2]octyl, Bicyclo[2.2.2]oct-2-enyl, Bicyclo[3.1.1]heptyl und Adamantyl, wobei der über eine Brücke gebundene alicyclische Rest substituiert ist durch einen Substituenten, der ausgewählt wird aus $(C_1-C_6)$Alkyl, Carboxy, verestertem Carboxy, $(C_1-C_6)$Alkylidendioxy, N,N-Di-$(C_1-C_6)$alkylcarbamoyl, Amino und geschütztem Amino.

9. Verbindung nach Anspruch 8, worin R Carboxynorbornyl darstellt.

10. Verbindung nach Anspruch 9, worin R 3-Carboxy-2-norbornyl darstellt.

11. Verbindung nach Anspruch 8, worin R verestertes Carboxynorbornyl darstellt.

12. Verbindung nach Anspruch 11, worin R $(C_1-C_6)$Alkoxycarbonylnorbornyl darstellt.

13. Verbindung nach Anspruch 12, worin R Methoxycarbonylnorbornyl darstellt.

14. Verbindung nach Anspruch 13, worin R 3-Methoxycarbonyl-2-norbornyl darstellt.

15. Verbindung nach Anspruch 12, worin R Äthoxycarbonylnorbornyl darstellt.

16. Verbindung nach Anspruch 15, worin R 3-Äthoxycarbonyl-2-norbornyl darstellt.

17. Verbindung nach Anspruch 12, worin R Isopropoxycarbonylnorbornyl darstellt.

18. Verbindung nach Anspruch 17, worin R 3-Isopropoxycarbonyl-2-norbornyl darstellt.

19. Verbindung nach Anspruch 8, worin R Norbornyl darstellt, das substituiert ist durch $(C_1—C_6)$Alkoxycarbonyl und $(C_1—C_6)$Alkylidendioxy.

20. Verbindung nach Anspruch 8, worin R N,N-Di-$(C_1—C_6)$alkyl-carbamoylnorbornyl darstellt.

21. Verbindung nach Anspruch 8, worin R Aminonorbornyl darstellt.

22. Verbindung nach Anspruch 8, worin R geschütztes Aminonorbornyl darstellt.

23. Verbindung nach Anspruch 8, worin R Carboxynorbornenyl darstellt.

24. Verbindung nach Anspruch 8, worin R verestertes Carboxynorbornenyl darstellt.

25. Verbindung nach Anspruch 24, worin R $(C_1—C_6)$Alkoxycarbonylnorbornenyl darstellt.

26. Verbindung nach Anspruch 8, worin R Bicyclo[3.1.1]heptyl darstellt, das durch mindestens einen $(C_1—C_6)$Alkylrest substituiert ist.

27. Verbindung nach Anspruch 26, worin R Pinanyl darstellt.

28. Verbindung nach Anspruch 8, worin R verestertes Carboxybicyclo[2.2.2]octyl darstellt.

29. Verbindung nach Anspruch 28, worin R $(C_1—C_6)$Alkoxycarbonylbicyclo[2-2-2]octyl darstellt.

30. Verbindung nach Anspruch 1, worin R einen substituierten $(C_1—C_6)$Alkylrest darstellt, wobei der Substituent ein substituierter oder unsubstituierter, über eine Brücke gebundener alicyclischer Rest ist, der ausgewählt wird aus Norbornyl und Adamantyl, wobei der Substituent des über eine Brücke gebundenen alicyclischen Restes ausgewählt wird aus $(C_1—C_6)$Alkyl und Halogen.

31. Verbindung nach Anspruch 30, worin R einen substituierten $(C_1—C_6)$Alkylrest darstellt, wobei der Substituent ein über eine Brüke gebundener alicyclischer Rest ist, der ausgewählt wird aus Norbornyl und Adamantyl.

32. Verbindung nach Anspruch 31, worin R Norbornyl-$(C_1—C_6)$alkyl darstellt.

33. Verbindung nach Anspruch 31, worin R Adamantyl-$(C_1—C_6)$alkyl darstellt.

34. Verbindung nach Anspruch 30, worin R einen substituierten $(C_1—C_6)$-Alkylrest darstellt, wobei der Substituent Adamantyl ist, der substituiert ist durch $(C_1—C_6)$Alkyl oder Halogen.

35. Verbindung nach Anspruch 34, worin R $(C_1—C_6)$-Alkyladamantyl-$(C_1—C_6)$-alkyl darstellt.

36. Verbindung nach Anspruch 34, worin R Halogenadamantyl-$(C_1—C_6)$-alkyl darstellt.

37. Verbindung nach Anspruch 1, worin R Amino-$(C_1—C_6)$-alkyl, geschütztes Amino$(C_1—C_6)$-alkyl, $(C_1—C_6)$-Alkoxy-$(C_1—C_6)$alkyl, Acyl$(C_1—C_6)$-alkyl, $(C_1—C_6)$-Alkylthio-$(C_1—C_6)$alkyl, Cyano-$(C_1—C_6)$-alkylthio-$(C_1—C_6)$-alkyl, Phenylthio-$(C_1—C_6)$alkyl, Pyridyl-$(C_1—C_6)$-alkyl, Thienyl-$(C_1—C_6)$-alkyl, 1,2-Dithiolanyl-$(C_1—C_6)$-alkyl, 2,5-Dioxopyrrolidinyl-$(C_1—C_6)$-alkyl, Carboxy-$(C_3—C_6)$-alkyl oder verestertes Carboxy-$(C_3—C_6)$alkyl darstellt.

38. Verbindung nach Anspruch 1, worin R trans, trans-Penta-1,3-dienyl darstellt.

39. Verbindung nach Anspruch 1, worin R ein $(C_2—C_6)$-Alkenyl, substituiert durch Norbornenyl, darstellt.

40. Verbindung nach Anspruch 1, worin R $(C_1—C_6)$-Alkoxy-substituiertes Phenyl-$(C_2—C_6)$-alkenyl, Furyl-$(C_2—C_6)$alkenyl oder Thienyl-$(C_2—C_6)$-alkenyl darstellt.

41. Verbindung nach Anspruch 1, worin R Tetrahydrothienyl darstellt.

42. Verfahren zur Herstellung einer Verbindung nach Anspruch 1, das umfaßt

1.) die Umsetzung von 5-Fluoruracil der Formel

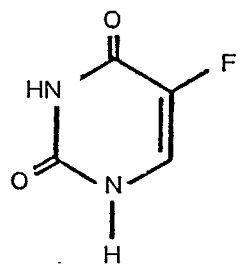

mit einem Isocyanat der Formel

$$R_a—N=C=O$$

worin $R_a$ darstellt einen substituierten oder unsubstituierten, über eine Brücke gebundenen alicyclischen Rest, ausgewählt aus substituiertem und unsubstituiertem Norbornyl, Norbornenyl, Bicyclo[2.2.2]octyl, Bicyclo[2.2.2]oct-2-enyl, Bicyclo[3.1.1]heptyl und Adamantyl, wobei der (die) Substituent(en) des über eine Brücke gebundenen alicyclischen Restes ausgewählt wird (werden) aus $(C_1—C_6)$Alkyl, verestertem Carboxy, $(C_1—C_6)$-Alkylidendioxy, N,N-Di-$(C_1—C_6)$-alkylcarbamoyl und geschütztem Amino; oder einen substituierten $(C_1—C_6)$-Alkylrest, wobei der Substituent ausgewählt wird aus substituierten und unsubstituierten, über eine Brücke gebundenen alicyclischen Resten,

ausgewählt aus substituiertem und unsubstituiertem Norbornyl und Adamantyl, wobei der Substituent des über eine Brücke gebundenen alicyclischen Restes ausgewählt wird aus ($C_1$—$C_6$)-Alkyl und Halogen; oder ein substituiertes ($C_1$—$C_6$)-Alkyl, wobei der Substituent ausgewählt wird aus geschütztem Amino, ($C_1$—$C_6$)-Alkoxy, Acyl, ($C_1$—$C_6$)-Alkylthio, Cyano-($C_1$—$C_6$)-alkylthio, Phenylthio, Pyridyl, Thienyl, 1,2-Dithiolanyl, 2,5-Dioxo-pyrrolidinyl und verestertem Carboxy, mit der Maßgabe, daß dann, wenn der Substituent des substituierten ($C_1$—$C_6$)-Alkylrestes verestertes Carboxy darstellt, der Alkylrest 3 bis 6 Kohlenstoffatome enthält; oder trans, trans-Penta-1,3-dienyl; oder einen substituierten ($C_2$—$C_6$)Alkenylrest, wobei der Substituent ausgewählt wird aus Norbornenyl, ($C_1$—$C_6$)-Alkoxy-substituiertem Phenyl, Furyl und Thienyl; oder Tetrahydrothienyl, unter Bildung eines 5-Fluoruracil-Derivats der Formel

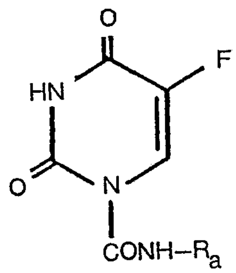

worin $R_a$ die oben angegebene Bedeutung hat; oder

2.) die Hydrolyse einer Verbindung der Formel

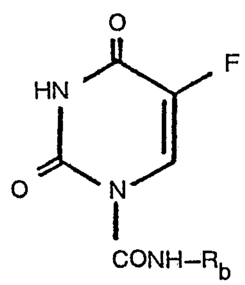

worin $R_a$ darstellt einen über eine Brücke gebundenen alicyclischen Rest, der eine geschützte Aminogruppe enthält, wobei der über eine Brücke gebundene alicyclische Rest ausgewählt wird aus Norbornyl, Norbornenyl, Bicyclo[2.2.2]octyl, Bicyclo[2.2.2]-oct-2-enyl, Bicyclo[3.1.1]heptyl und Adamantyl; oder einen substituierten ($C_1$—$C_6$)Alkylrest, wobei der Substituent eine geschützte Aminogruppe ist;

unter Bildung eines 5-Fluoruracil-Derivats der Formel

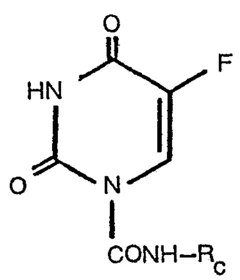

worin $R_c$ darstellt einen über eine Brücke gebundenen alicyclischen Rest, der eine Aminogruppe enthält, wobei der über eine Brücke gebundene alicyclische Rest ausgewählt wird aus Norbornyl, Norbornenyl, Bicyclo[2.2.2]octyl, Bicyclo[2.2.2]oct-2-enyl, Bicyclo[3.1.1]heptyl und Adamantyl; oder einen substituierten ($C_1$—$C_6$)Alkylrest, wobei es sich bei dem Substituenten um eine Aminogruppe handelt; oder

3.) die Hydrolyse einer Verbindung der Formel

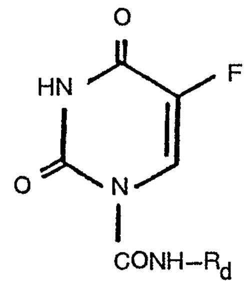

worin $R_d$ darstellt einen über eine Brücke gebundenen alicyclischen Rest, der eine veresterte Carboxygruppe enthält, wobei der über eine Brücke gebundene alicyclische Rest ausgewählt wird aus Norbornyl, Norbornenyl, Bicyclo[2.2.2]octyl, Bicyclo[2.2.2]oct-2-enyl, Bicyclo[3.1.1]heptyl und Adamantyl; oder einen substituierten ($C_1$—$C_6$)Alkylrest, wobei der Substituent eine veresterte Carboxygruppe ist, unter Bildung eines 5-Fluoruracil-Derivats der Formel

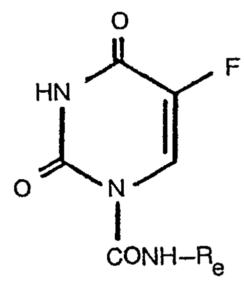

worin $R_e$ darstellt einen über eine Brücke gebundenen alicyclischen Rest, der eine Carboxygruppe enthält, wobei der über eine Brücke gebundene alicyclische Rest ausgewählt wird aus Norbornyl, Norbornenyl, Bicyclo[2.2.2]octyl, Bicyclo[2.2.2]oct-2-enyl, Bicyclo[3.1.1]heptyl und Adamantyl; oder einen substituierten-($C_1$—$C_6$)-alkylrest, wobei der Substituent eine Carboxygruppe ist.

43. Pharmazeutische Zusammensetzung, die eine Verbindung nach Anspruch 1 und/oder ein pharmazeutisch akzeptables Salz davon in Assoziation mit einem pharmazeutischen, im wesentlichen nichttoxischen Träger oder Hilfsstoff enthält.

**Patentansprüche für den Vertragsstaat AT**

1. Verfahren zur Herstellung einer Verbindung der Formel

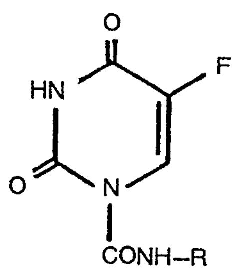

worin R darstellt einen substituierten oder unsubstituierten, über eine Brücke gebundenen alicyclischen Rest, ausgewählt aus substituiertem und unsubstituiertem Norbornyl, Norbornenyl, Bicyclo-[2.2.2]octyl, Bicyclo[2.2.2]oct-2-enyl, Bicyclo[3.1.1]heptyl und Adamantyl, wobei der (die) Substituent(en) des über eine Brücke gebundenen alicyclischen Restes ausgewählt wird (werden) aus ($C_1$—$C_6$)Alkyl, Carboxy, verestertem Carboxy, ($C_1$—$C_6$)Alkylidendioxy, N,N-Di-($C_1$—$C_6$)alkylcarbamoyl, Amino und geschütztem Amino; oder einen substituierten ($C_1$—$C_6$)Alkylrest, wobei de Substituent ausgewählt wird aus substituierten und unsubstituierten, über eine Brücke gebundenen alicyclischen Resten, ausgewählt aus substituiertem und unsubstituiertem Norbornyl und Adamantyl, wobei der Substituent des über eine Brücke gebundenen alicyclischen Restes ausgewählt wird aus ($C_1$—$C_6$)Alkyl und Halogen; oder einen substituierten ($C_1$—$C_6$)Alkylrest, wobei der Substituent ausgewählt wird aus Amino, geschütztem Amino ($C_1$—$C_6$)Alkoxy, Acyl, ($C_1$—$C_6$)Alkylthio, Cyano-($C_1$—$C_6$)alkylthio, Phenylthio, Pyridyl, Thienyl, 1,2-Dithiolanyl, 2,5-Dioxopyrrolidinyl, Carboxy und verestertem Carboxy, mit der Maßgabe, daß dann, wenn der Substituent des substituierten ($C_1$—$C_6$)Alkylrestes Carboxy oder verestertes Carboxy ist, der Alkylrest 3 bis 6 Kohlenstoffatome enthält; oder einen trans, trans-Penta-1,3-dienylrest; oder einen substituierten ($C_2$—$C_6$)Alkenylrest, wobei der Substituent ausgewählt wird aus Norbornenyl, ($C_1$—$C_6$)Alkoxy-substituiertem Phenyl, Furyl und Thienyl; oder einen Tetrahydrothienylrest; sowie die pharmazeutisch akzeptablen Salze davon, das umfaßt

1.) die Umsetzung von 5-Fluoruracil der Formel

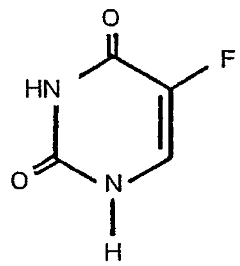

mit einem Isocyanat der Formel

$$R_a—N=C=O$$

worin $R_a$ darstellt einen substituierten oder unsubstituierten, über eine Brücke gebundenen alicyclischen Rest, ausgewählt aus substituiertem und unsubstituiertem Norbornyl, Norbornenyl, Bicyclo-[2.2.2]octyl, Bicyclo[2.2.2]oct-2-enyl, Bicyclo[3.1.1]heptyl und Adamantyl, wobei der (die) Substituent(en) des über eine Brücke gebundenen alicyclischen Restes ausgewählt wird (werden) aus ($C_1$—$C_6$)Alkyl, verestertem Carboxy, ($C_1$—$C_6$)-Alkylidendioxy, N,N-Di-($C_1$—$C_6$)-alkylcarbamoyl und geschütztem Amino; oder einen substituierten ($C_1$—$C_6$)-Alkylrest, wobei der Substituent ausgewählt wird aus substituierten und unsubstituierten, über eine Brücke gebundenen alicyclischen Resten, ausgewählt aus substituiertem und unsubstituiertem Norbornyl und Adamantyl, wobei der Substituent des über eine Brücke gebundenen alicylischen Restes ausgewählt wird aus ($C_1$—$C_6$)-Alkyl und Halogen; oder ein substituiertes ($C_1$—$C_6$)-Alkyl, wobei der Substituent ausgewählt wird aus geschütztem Amino, ($C_1$—$C_6$)-Alkoxy, Acyl, ($C_1$—$C_6$)Alkylthio, Cyano-($C_1$—$C_6$)-alkylthio, Phenylthio, Pyridyl, Thienyl, 1,2-Dithiolanyl, 2,5-Dioxo-pyrrolidinyl und verestertem Carboxy, mit der Maßgabe, daß dann, wenn der Substituent des substituierten ($C_1$—$C_6$)-Alkylrestes verestertes Carboxy darstellt, der Alkylrest 3 bis 6 Kohlenstoffatome enthält; oder trans, trans-Penta-1,3-dienyl; oder einen substituierten ($C_2$—$C_6$)-Alkenylrest, wobei der Substituent ausgewählt wird aus Norbornenyl, ($C_1$—$C_6$)-Alkoxy-substituiertem Phenyl, Furyl und Thienyl; oder Tetrahydrothienyl, unter Bildung eines 5-Fluoruracil-Derivats der Formel

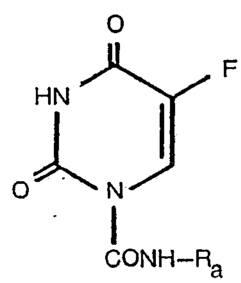

worin $R_a$ die oben angegebene Bedeutung hat; oder

2.) die Hydrolyse einer Verbindung der Formel

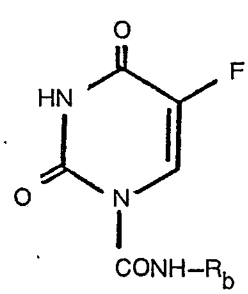

worin $R_b$ darstellt einen über eine Brücke gebundenen alicyclischen Rest, der eine geschützte Aminogruppe enthält, wobei der über eine Brücke gebundene alicyclische Rest ausgewählt wird aus Norbornyl, Norbornenyl, Bicyclo[2.2.2]octyl, Bicyclo[2.2.2]oct-2-enyl, Bicyclo[3.1.1]heptyl und Adamantyl;

oder einen substituierten ($C_1$—$C_6$)Alkylrest, wobei der Substituent eine geschützte Aminogruppe ist; unter Bildung eines 5-Fluoruracil-Derivats der Formel

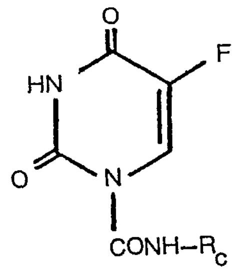

worin $R_c$ darstellt einen über eine Brücke gebundenen alicyclischen Rest, der eine Aminogruppe enthält, wobei der über eine Brücke gebundene alicyclische Rest ausgewählt wird aus Norbornyl, Norbornenyl, Bicyclo[2.2.2]octyl, Bicyclo[2.2.2]oct-2-enyl, Bicyclo[3.1.1]heptyl und Adamantyl; oder einen substituierten ($C_1$—$C_6$)Alkylrest, wobei es sich bei dem Substituenten um eine Aminogruppe handelt; oder

3.) die Hydrolyse einer Verbindung der Formel

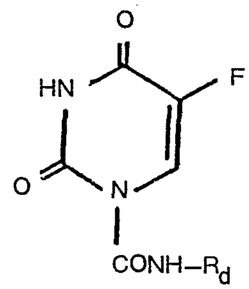

worin $R_d$ darstellt einen über eine Brücke gebundenen alicylischen Rest, der eine veresterte Carboxygruppe enthält, wobei der über eine Brücke gebundene alicyclische Rest ausgewählt wird aus Norbornyl, Norbornenyl, Bicyclo[2.2.2]octyl, Bicyclo[2.2.2]oct-2-enyl, Bicyclo[3.1.1]heptyl und Adamantyl; oder einen substituierten ($C_1$—$C_6$)Alkylrest, wobei der Substituent eine veresterte Carboxygruppe ist, unter Bildung eines 5-Fluoruracil-Derivats der Formel

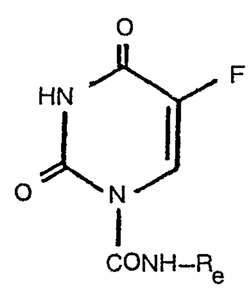

worin $R_e$ darstellt eine über eine Brücke gebundenen alicyclischen Rest, der eine Carboxygruppe enthält, wobei der über eine Brücke gebundene alicyclische Rest ausgewählt wird aus Norbornyl, Norbornenyl, Bicyclo[2.2.2]octyl, Bicyclo[2.2.2]oct-2-enyl, Bicyclo[3.1.1]heptyl und Adamantyl; oder einen substituierten-($C_1$—$C_6$)-alkylrest, wobei der Substituent eine Carboxygruppe ist.

2. Verfahren nach Anspruch 1, worin R darstellt einen substituierten oder unsubstituierten, über eine Brücke gebundenen alicyclischen Rest, ausgewählt aus Norbornyl, Norbornenyl, Bicyclo[2.2.2]-octyl, Bicyclo[2.2.2]oct-2-enyl, Bicyclo[3.1.1]heptyl und Adamantyl, wobei der (die) Substituent(en) des über eine Brücke gebundenen alicyclischen Restes ausgewählt wird (werden) aus ($C_1$—$C_6$)Alkyl, Carboxy, verestertem Carboxy, ($C_1$—$C_6$)Alkylidendioxy, N,N-Di-($C_1$—$C_6$)alkyl-carbamoyl, Amino und geschütztem Amino.

3. Verfahren nach Anspruch 2, worin R einen über eine Brücke gebundenen alicyclischen Rest darstellt, der ausgewählt wird aus Norbornyl, Norbornenyl und Adamantyl.

4. Verfahren nach Anspruch 3, worin R Norbornyl darstellt.

5. Verfahren nach Anspruch 4, worin R 2-Norbornyl darstellt.

6. Verfahren nach Anspruch 3, worin R Adamantyl darstellt.

7. Verfahren nach Anspruch 6, worin R 1-Adamantyl darstellt.

8. Verfahren nach Anspruch 2, worin R einen über eine Brücke gebundenen alicyclischen Rest darstellt, der ausgewählt wird aus Norbornyl, Norbornenyl, Bicyclo[2.2.2]octyl, Bicyclo[2.2.2]oct-2-enyl, Bicyclo[3.1.1]heptyl und Adamantyl, wobei der über iene Brücke gebundene alicyclische Rest substituiert ist durch einen Substituenten, der ausgewählt wird aus ($C_1$—$C_6$)Alkyl, Carboxy, verestertem Carboxy, ($C_1$—$C_6$)Alkylidendioxy, N,N-Di-($C_1$—$C_6$)alkylcarbamoyl, Amino und geschütztem Amino.

9. Verfahren nach Anspruch 8, worin R Carboxynorbornyl darstellt.

10. Verfahren nach Anspruch 9, worin R 3-Carboxy-2-norbonyl darstellt.

11. Verfahren nach Anspruch 8, worin R verestertes Carboxynorbornyl darstellt.

12. Verfahren nach Anspruch 11, worin R ($C_1$—$C_6$)Alkoxycarbonylnorbornyl darstellt.

13. Verfahren nach Anspruch 12, worin R Methoxycarbonylnorbornyl darstellt.

14. Verfahren nach Anspruch 13, worin R 3-Methoxycarbonyl-2-norbornyl darstellt.

15. Verfahren nach Anspruch 12, worin R Äthoxycarbonylnorbornyl darstellt.

16. Verfahren nach Anspruch 15, worin R 3-Äthoxycarbonyl-2-norbornyl darstellt.

17. Verfahren nach Anspruch 12, worin R Isopropoxycarbonylnorbornyl darstellt.

18. Verfahren nach Anspruch 17, worin R 3-Isopropoxycarbonyl-2-norbornyl darstellt.

19. Verfahren nach Anspruch 8, worin R Norbornyl darstellt das substituiert ist durch ($C_1$—$C_6$)-Alkoxycarbonyl und ($C_1$—$C_6$)Alkylidendioxy.

20. Verfahren nach Anspruch 8, worin R N,N-Di-($C_1$—$C_6$)alkylcarbamoylnorbornyl darstellt.

21. Verfahren nach Anspruch 8, worin R Aminonorbornyl darstellt.

22. Verfahren nach Anspruch 8, worin R geschütztes Aminonorbornyl darstellt.

23. Verfahren nach Anspruch 8, worin R Carboxynorbornenyl darstellt.

24. Verfahren nach Anspruch 8, worin R verestertes Carboxynorbornenyl darstellt.

25. Verfahren nach Anspruch 24, worin R ($C_1$—$C_6$)Alkoxycarbonylnorbornenyl darstellt.

26. Verfahren nach Anspruch 8, worin R Bicyclo[3.1.1]heptyl darstellt, das durch mindestens einen ($C_1$—$C_6$)Alkylrest substituiert ist.

27. Verfahren nach Anspruch 26, worin R Pinanyl darstellt.

28. Verfahren nach Anspruch 8, worin R verestertes Carboxybicyclo[2.2.2]octyl darstellt.

29. Verfahren nach Anspruch 28, worin R ($C_1$—$C_6$)Alkoxycarbonylbicyclo[2.2.2]octyl darstellt.

30. Verfahren nach Anspruch 1, worin R einen substituierten ($C_1$—$C_6$)Alkylrest darstellt, wobei der Substituent ein substituierter oder unsubstituierter, über eine Brücke gebundener alicyclischer Rest ist, der ausgewählt wird aus Norbornyl und Adamantyl, wobei der Substituent des über eine Brücke gebundenen alicyclischen Restes ausgewählt wird aus ($C_1$—$C_6$)Alkyl und Halogen.

31. Verfahren nach Anspruch 30, worin R einen substituierten ($C_1$—$C_6$)Alkylrest darstellt, wobei der Substituent ein über eine Brücke gebundener alicyclischer Rest ist, der ausgewählt wird aus Norbornyl und Adamantyl.

32. Verfahren nach Anspruch 31, worin R Norbornyl-($C_1$—$C_6$)-alkyl darstellt.

33. Verfahren nach Anspruch 31, worin R Adamantyl-($C_1$—$C_6$)-alkyl darstellt.

34. Verfahren nach Anspruch 30, worin R einen substituierten ($C_1$—$C_6$)-Alkylrest darstellt, wobei der Substituent Adamantyl ist, der substituiert ist durch ($C_1$—$C_6$)Alkyl oder Halogen.

35. Verfahren nach Anspruch 34, worin R ($C_1$—$C_6$)-Alkyladamantyl-($C_1$—$C_6$)-alkyl darstellt.

36. Verfahren nach Anspruch 34, worin R Halogenadamantyl-($C_1$—$C_6$)-alkyl darstellt.

37. Verfahren nach Anspruch 1, worin R Amino-($C_1$—$C_6$)-alkyl, geschütztes Amino($C_1$—$C_6$)-alkyl, ($C_1$—$C_6$)-Alkoxy-($C_1$—$C_6$)alkyl, Acyl($C_1$—$C_6$)-alkyl, ($C_1$—$C_6$)-Alkylthio-($C_1$—$C_6$)alkyl, Cyano-($C_1$—$C_6$)-alkylthio-($C_1$—$C_6$)-alkyl, Phenylthio-($C_1$—$C_6$)alkyl, Pyridyl-($C_1$—$C_6$)-alkyl, Thienyl-($C_1$—$C_6$)-alkyl, 1,2-Dithiolanyl-($C_1$—$C_6$)-alkyl, 2,5-Dioxopyrrolidinyl-($C_1$—$C_6$)-alkyl, Carboxy-($C_3$—$C_6$)-alkyl oder verestertes Carboxy-($C_3$—$C_6$)alkyl darstellt.

38. Verfahren nach Anspruch 1, worin R trans, trans-Penta-1,3-dienyl darstellt.

39. Verfahren nach Anspruch 1, worin R ein ($C_2$—$C_6$)-Alkenyl, substituiert durch Norbornenyl, darstellt.

40. Verfahren nach Anspruch 1, worin R ($C_1$—$C_6$)-Alkoxy-substituiertes Phenyl-($C_2$—$C_6$)alkenyl, Furyl-($C_2$—$C_6$)-alkenyl oder Thienyl-($C_2$—$C_6$)-alkenyl darstellt.

41. Verfahren nach Anspruch 1, worin R Tetrahydrothienyl darstellt.

**Revendications pour les Etats contractants: BE, CH, DE, FR, GB, IT, LU, NL, SE**

1. Composé ayant la formule:

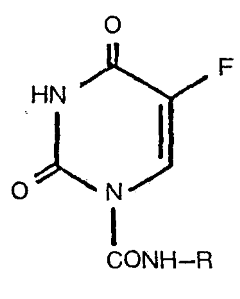

où R est un radical alicyclique ponté substitué ou non substitué choisi parmi les groupes nonbornyle, norbornényle, bicyclo[2.2.2]octyle, bicyclo[2.2.2] oct-2-ényle, bicyclo[3.1.1]heptyle et adamantyle, substitués et non substitués, le ou les substituants de ce radical alicyclique ponté étant choisis parmi les groupes alkyle en $C_1$—$C_6$, carboxy, carboxy estérifié, alkylidène (en $C_1$—$C_6$)dioxy, N,N-dialkyl(en $C_1$—$C_6$)carbamoyle, amino et amino protégé; ou est un radical alkyle en $C_1$—$C_6$ substitué, le substituant étant choisi parmi des radicaux alicycliques pontés substitués et non substitués choisis parmi les groupes norbornyle et adamantyle substitués et non substitués, le substituant de ce radical alicyclique ponté étant choisi parmi des groupes alkyles en $C_1$—$C_6$ et des halogènes; ou est un radical alkyle en $C_1$—$C_6$ substitué, le substituant étant choisi parmi les groupes amino, amino protégé, alcoxy en $C_1$—$C_6$, acyle, alkyl(en $C_1$—$C_6$)thio, cyanoalkyl(en $C_1$—$C_6$)thio, phénylthio, pyridyle, thiényle, 1,2-dithiolanyle, 2,5-dioxopyrrolydinyle, carboxyt et carboxy estérifié, en prévoyant que, quand le substituant du radical alkyle en $C_1$—$C_6$ substitué est le groupe carboxy ou carboxy estérifié, le radical alkyle contient 3 à 6 atomes de carbone, ou est un radical trans,trans-penta-1,3-diényle; ou est un radical alkényle en $C_2$—$C_6$ substitué, le substituant étant choisi parmi les groupes norbornényle, phényle à substitution alcoxy en $C_1$—$C_6$, furyle et thiényle; ou est un radical tétrahydrothiényle et leurs sels pharmaceutiquement acceptables.

2. Composé selon la revendication 1, dans lequel R est un radical alicyclique ponté substitué ou non substitué, choisi parmi les groupes norbornyle, norbornényle, bicyclo[2.2.2]octyle, bicyclo[2.2.2]-oct-2-ényle, bicyclo[3.1.1]heptyle et adamantyle, le ou les substituants de ce radical alicyclique ponté étant choisis parmi les groupes alkyle en $C_1$—$C_6$, carboxy, carboxy estérifié, alkylidène (en ($C_1$—$C_6$)-dioxy, N,N-dialkyl(en $C_1$—$C_6$)carbamoyle, amino et amino protégé.

3. Composé selon la revendication 2, dans lequel R est un radical alicyclique ponté choisi parmi les groupes norbornyle, norbornényle et adamantyle.

4. Composé selon la revendication 3, dans lequel R est le groupe norbornyle.

5. Composé selon la revendication 4, dans lequel R est le groupe 2-norbornyle.

6. Composé selon la revendication 3, dans lequel R est le groupe adamantyle.

7. Composé selon la revendication 6, dans lequel R est le groupe 1-adamantyle.

8. Composé selon la revendication 2, dans lequel R est un radical alicyclique ponté choisi parmi les groupes norbornyle, norbornényle, bicyclo[2.2.2]octyle, bicyclo[2.2.2]oct-2-ényle, bicyclo[3.1.1]-heptyle et adamantyle, ce radical alicyclique ponté étant substitué par un substituant choisi parmi les groupes alkyle en $C_1$—$C_6$, carboxy, carboxy estérifié, alkylidène(en $C_1$—$C_6$)dioxy, N,N-dialkyl(en $C_1$—$C_6$)carbamoyle, amino et amino protégé.

9. Composé selon la revendication 8, dans lequel R est le groupe carboxynorbornyle.

10. Composé selon la revendication 9, dans lequel R est le groupe 3-carboxy-2-norbornyle.

11. Composé selon la revendication 8, dans lequel R est un groupe carboxynorbornyle estérifié.

12. Composé selon la revendication 11, dans lequel R est un groupe alcoxy(en $C_1$—$C_6$)carbonyl-norbornyle.

13. Composé selon la revendication 12, dans lequel R est le groupe méthoxycarbonylnorbornyle.

14. Composé selon la revendication 13, dans lequel R est le groupe 3-méthoxycarbonyl-2-norbornyle.

15. Composé selon la revendication 12, dans lequel R est le groupe éthoxycarbonylnorbornyle.

16. Composé selon la revendication 15, dans lequel R est le groupe 3-éthoxycarbonyl-2-norbornyle.

17. Composé selon la revendication 12, dans lequel R est le groupe isopropoxycarbonyl-norbornyle.

18. Composé selon la revendication 17, dans lequel R est le groupe 3-isopropoxycarbonyl-2-norbornyle.

19. Composé selon la revendication 8, dans lequel R est le groupe norbornyle à substitution alcoxy(en $C_1$—$C_6$)carbonyle et alkylidène(en $C_1$—$C_6$)dioxy.

20 Composé selon la revendication 8, dans lequel R est un groupe N,N-dialkyl(en $C_1$—$C_6$)-carbamoylnorbornyle.

21. Composé selon la revendication 8, dans lequel R est le groupe aminonorbornyle.

22. Composé selon la revendication 8, dans lequel R est un groupe aminonorbornyle protégé.

23. Composé selon la revendication 8, dans lequel R est le groupe carboxynorbornényle.

24. Composé selon la revendication 8, dans lequel R est un groupe carboxynorbornényle estérifié.

25. Composé selon la revendication 24, dans lequel R est un groupe alcoxy(en $C_1$—$C_6$)carbonyl-norbornényle.

26. Composé selon la revendication 8, dans lequel R est le groupe bicyclo[3.1.1]heptyle substitué par au moins un radical alkyle en $C_1$—$C_6$.

27. Composé selon la revendication 26, dans lequel R est le groupe pinanyle.

28. Composé selon la revendication 8, dans lequel R est un groupe carboxybicyclo[2.2.2]octyle estérifié.

29. Composé selon la revendication 28, dans lequel R est un groupe alcoxy(en $C_1$—$C_6$)carbonyl-bicyclo[2.2.2]octyle.

30. Composé selon la revendication 1, dans lequel R est un radical alkyle en $C_1$—$C_6$ substitué, le

substituant étant un radical alicyclique ponté substitué ou non substitué, choisi parmi les groupes norbornyle et adamantyle, le substituant de ce radical alicyclique ponté étant choisi parmi les groupes alkyles en $C_1$—$C_6$ et les halogènes.

31. Composé selon la revendication 30, dans lequel R est un radical alkyle en $C_1$—$C_6$ substitué, le substituant étant un radical alicyclique ponté choisi parmi les groupes norbornyle et adamantyle.

32. Composé selon la revendication 31, dans lequel R est un radical norbornyl-alkyle en $C_1$—$C_6$.

33. Composé selon la revendication 31, dans lequel R est un groupe adamantyl-alkyle en $C_1$—$C_6$.

34. Composé selon la revendication 30, dans lequel R est un radical alkyle en $C_1$—$C_6$ substitué, le substituant étant un groupe adamantyle, qui est à substitution alkyle en $C_1$—$C_6$ ou halogénée.

35. Composé selon la revendication 34, dans lequel R est un groupe alkyle (en $C_1$—$C_6$)-adamantyl-alkyle en $C_1$—$C_6$.

36. Composé selon la revendication 34, dans lequel R est un groupe haloadamantyl-alkyle en $C_1$—$C_6$.

37. Composé selon la revendication 1, dans lequel R est un groupe aminoalkyle en $C_1$—$C_6$, amino protége-alkyle en $C_1$—$C_6$, alcoxy(en $C_1$—$C_6$)alkyle en $C_1$—$C_6$, acyl-alkyle en $C_1$—$C_6$, alkyl(en $C_1$—$C_6$)-thioalkyle en $C_1$—$C_6$, cyano-alkyl(en $C_1$—$C_6$)thioalkyle en $C_1$—$C_6$, phénylthio-alkyle en $C_1$—$C_6$, pyridyl-alkyle en $C_1$—$C_6$, thiényl-alkyle en $C_1$—$C_6$, 1,2-dithiolanyl-alkyle en $C_1$—$C_6$, 2,5-dioxopyrrolidinyl-alkyle en $C_1$—$C_6$, carboxy-alkyle en $C_3$—$C_6$ ou carboxy estérifié alkyle en $C_3$—$C_6$.

38. Composé selon la revendication 1, dans lequel R est le groupe trans, trans-penta-1,3-diényle.

39. Composé selon la revendication 1, dans lequel R est un groupe alkényle en $C_1$—$C_6$, à substitution norbornényle.

40. Composé selon la revendication 1, dans lequel R est un groupe phényl-alkényle en $C_2$—$C_6$ à substitution alcoxy en $C_1$—$C_6$, furyl-alkényle en $C_2$—$C_6$ ou thiényl-alkényle en $C_2$—$C_6$.

41. Composé selon la revendication 1, dans lequel R est le groupe tétrahydrothiényle.

42. Procédé de préparation d'un composé selon la revendication 1, qui consisté:

(1) à faire réagir du 5-fluorouracile ayant la formule:

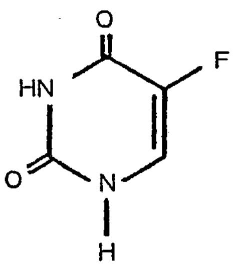

avec un isocyanate ayant la formule:

$$R_a—N{=}C{=}O$$

où $R_a$ est un radical alicyclique ponté substitué ou non substitué, choisi parmi les groupes norbornyle, norbornényle, bicyclo[2.2.2]octyle, bicyclo[2.2.2]oct-2-ényle, bicyclo[3.1.1]heptyle et adamantyle, substitués et non substitués, le ou les substituants de ce radical alicyclique ponté choisis parmi des groupes alkyle en $C_1$—$C_6$, carboxy estérifié, alkylidène (en $C_1$—$C_6$)dioxy, N,N-dialkyl(en $C_1$—$C_6$)-carbamoyle et amino protégé; ou est un radical alkyle en $C_1$—$C_6$ substitué, le substituant étant choisi parmi des radicaux alicycliques pontés substitués et non substitués, choisis parmi le groupe norbornyle et le groupe adamantyle substitués et non substitués, le substituant de ce radical alicyclique ponté étant choisi parmi des groupes alkyles en $C_1$—$C_6$ et des halogènes; ou est un radical alkyle en $C_1$—$C_6$ substitué, le substituant étant choisi parmi les groupes amino protégé, alcoxy en $C_1$—$C_6$, acyle, alkyl(en $C_1$—$C_6$) thio, cyanoalkyl(en $C_1$—$C_6$)thio, phénylthio, pyridyle, thiényle, 1,2-dithiolanyle, 2,5-dioxo-pyrrolidinyle et carboxy estérifié, en prévoyant que, quand le substituant du radical alkyle en $C_1$—$C_6$ substitué est un groupe carboxy estérifié, le radical alkyle contient 3 à 6 atomes de carbone; ou est le groupe trans, trans-penta-1,3-diényle; ou est un radical alkényle en $C_2$—$C_6$ substitué, le substituant étant choisi parmi les groupes norbornényle, phényle à substitution alcoxy en $C_1$—$C_6$, furyle et thiényle; ou est le groupe tétrahydrothiényle, pour donner un dérivé de 5-fluorouracile ayant la formule:

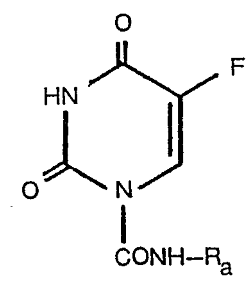

où $R_a$ est tel que désigné précédemment; ou

(2) à hydrolyser un composé ayant la formule:

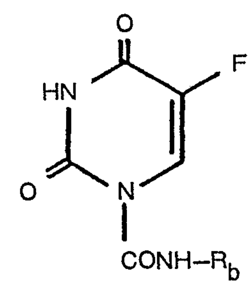

où $R_b$ est un radical alicyclique ponté contenant un groupe amino protégé, le radical alicyclique ponté étant choisi parmi les groupes norbornyle, norbornényle, bicyclo[2.2.2]octyle, bicyclo[2.2.2]oct-2-ényle, bicyclo[3.1.1]heptyle et adamantyle; ou est un radical alkyle en $C_1$—$C_6$ substitué, le substituant étant un groupe amino protégé, pour donner un dérivé de 5-fluorouracile ayant la formule:

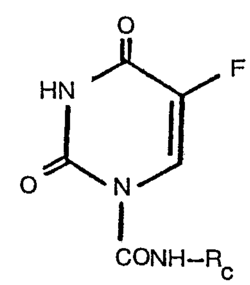

où $R_c$ est un radical alicyclique ponté contenant un groupe amino, le radical alicylique ponté étant choisi parmi les groupes norbornyle, norbornényle, bicyclo[2.2.2]octyle, bicyclo[2.2.2]oct-2-ényle, bicyclo[3.1.1]heptyle et adamantyle; ou est un radical alkyle en $C_1$—$C_6$ substitué, le substituant étant un groupe amino; ou

(3) à hydrolyser un composé ayant la formule:

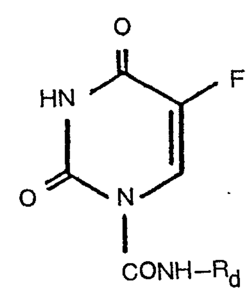

où $R_d$ est un radical alicyclique ponté contenant un groupe carboxy estérifié, le radical alicyclique ponté étant choisi parmi les groupes norbornyle, norbornényle, bicyclo[2.2.2]octyle, bicyclo[2.2.2]oct-2-ényle, bicyclo[3.1.1]heptyle et adamantyle; ou est un radical alkyle en $C_1$—$C_6$ substitué, le substituant étant un groupe carboxy estérifié, pour donner un dérivé de 5-fluorouracile ayant la formule:

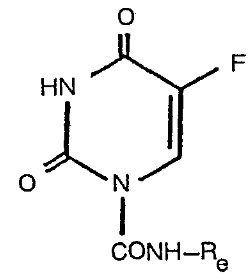

où $R_e$ est un radical alicyclique ponté contenant un groupe carboxy, le radical alicylique ponté étant choisi parmi les groupes norbornyle, norbornényle, bicyclo[2.2.2]octyle, bicyclo[2.2.2]oct-2-ényle, bicyclo[3.1.1]heptyle et adamantyle; ou est un radical alkyle en $C_1$—$C_6$ substitué, le substituant étant un groupe carboxy.

43. Composition pharmaceutique comprenant un composé selon la revendication 1 et/ou son sel pharmaceutiquement acceptable, en association avec un support ou un excipient sensiblement non toxique du point du vue pharmaceutique.

**Revendications pour l'Etat contractant: AT**

1. Procédé de préparation d'un Composé ayant la formule:

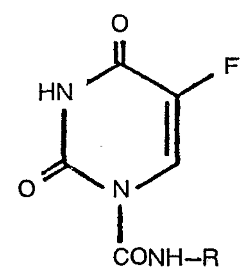

où R est un radical alicyclique ponté substitué ou non substitué choisi parmi les groupes nonbornyle, norbornényle, bicyclo[2.2.2]octyle, bicyclo[2.2.2] oct-2-ényle, bicyclo[3.1.1]heptyle et adamantyle, substitués et non substitués, le ou les substituants de ce radical alicyclique ponté étant choisis parmi les groupes alkyle en $C_1$—$C_6$, carboxy, carboxy estérifié, alkylidène (en $C_1$—$C_6$)dioxy, N,N-dialkyl(en $C_1$—$C_6$)carbamoyle, amino et amino protégé; ou est un radical alkyle en $C_1$—$C_6$ substitué, le substituant étant choisi parmi des radicaux alicycliques pontés substitués et non substitués choisis parmi les groupes norbornyle et adamantyle substitués et non substitués, le substituant de ce radical alicyclique ponté étant choisi parmi des groupes alkyles en $C_1$—$C_6$ et des halogènes; ou est un radical alkyle en $C_1$—$C_6$ substitué, le substituant étant choisi parmi les groupes amino, amino protégé, alcoxy en $C_1$—$C_6$, acyle, alkyl(en $C_1$—$C_6$)thio, cyanoalkyl(en $C_1$—$C_6$)thio, phénylthio, pyridyle, thiényle, 1,2-dithiolanyle, 2,5-dioxopyrrolydinyle, carboxy et carboxy estérifié, en prévoyant que, quand le substituant du radical alkyle en $C_1$—$C_6$ substitué est le groupe carboxy ou carboxy estérifié, le radical alkyle contient 3 à 6 atomes de carbone, ou est un radical trans,trans-penta-1,3-diényle; ou est un radical alkényle en $C_2$—$C_6$ substitué, le substituant étant choisi parmi les groupes norbornényle, phényle à substitution alcoxy en $C_1$—$C_6$, furyle et thiényle; ou est un radical tétrahydrothiényle et leurs sels pharmaceutiquement acceptables.
qui consiste:

(1) à faire réagir du 5-fluorouracile ayant la formule:

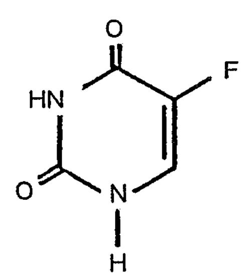

avec un isocyanate ayant la formule:

$R_a—N=C=O$

où $R_a$ est un radical alicyclique ponté substitué ou non substitué, choisi parmi les groupes norbornyle, norbornényle, bicyclo[2.2.2]octyle, bicyclo[2.2.2]oct-2-ényle, bicyclo[3.1.1]heptyle et adamantyle, substitués et non substitués, le ou les substituants de ce radical alicyclique ponté étant choisis parmi des groupes alkyle en $C_1—C_6$, carboxy estérifié, alkylidène(en $C_1—C_6$)dioxy, N,N-dialkyl(en $C_1—C_6$)-carbamoyle et amino protégé; ou est un radical alkyle en $C_1—C_6$ substitué, le substituant étant choisi parmi des radicaux alicycliques pontés substitués et non substitués, choisis parmi le groupe norbornyle et le groupe adamantyle substitués et non substitués, le substituant de ce radical alicyclique ponté étant choisi parmi des groupes alkyles en $C_1—C_6$ et des halogènes; ou est un radical alkyle en $C_1—C_6$ substitué, le substituant étant choisi parmi les groupes amino protégé, alcoxy en $C_1—C_6$, acyle, alkyl-(en $C_1—C_6$) thio, cyanoalkyl(en $C_1—C_6$)thio, phénylthio, pyridyle, thiényle, 1,2-dithiolanyle, 2,5-dioxo-pyrolidinyle et carboxy estérifié, en prévoyant que, quand le substituant du radical alkyle en $C_1—C_6$ substitué est un groupe carboxy estérifié, le radical alkyle contient 3 à 6 atomes de carbone; ou est le groupe trans, trans-penta-1,3-diényle; ou est un radical alkényle en $C_2—C_6$ substitué, le substituant étant choisi parmi les groupes norbornényle, phényle à substitution alcoxy en $C_1—C_6$, furyle et thiényle; ou est le groupe tétrahydrothiényle, pour donner un dérivé de 5-fluorouracile ayant la formule:

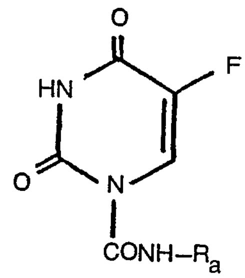

où $R_a$ est tel que désigné précédemment; ou

(2) à hydrolyser un composé ayant la formule:

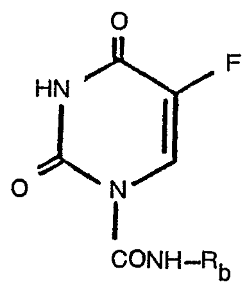

où $R_b$ est un radical alicyclique ponté contenant un groupe amino protégé, le radical alicyclique ponté étant choisi parmi les groupes norbornyle, norbornényle, bicyclo[2.2.2]octyle, bicyclo[2.2.2]oct-2-ényle, bicyclo[3.1.1]heptyle et adamantyle; ou est un radical alkyle en $C_1—C_6$ substitué, le substituant étant un groupe amino protégé, pour donner un dérivé de 5-fluorouracile ayant la formule:

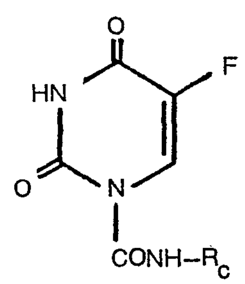

où $R_c$ est un radical alicyclique ponté contenant un groupe amino, le radical alicyclique ponté étant choisi parmi les groupes norbornyle, norbornényle, bicyclo[2.2.2]octyle, bicyclo[2.2.2]oct-2-ényle, bicyclo[3.1.1]heptyle et adamantyle; ou est un radical alkyle en $C_1—C_6$ substitué, le substituant étant un groupe amino; ou

(3) à hydrolyser un composé ayant la formule:

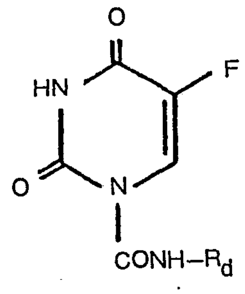

où $R_d$ est un radical alicyclique ponté contenant un groupe carboxy estérifié, le radical alicyclique ponté étant choisi parmi les groupes norbornyle, norbornényle, bicyclo[2.2.2]octyle, bicyclo[2.2.2]oct-2-ényle, bicyclo[3.1.1.]heptyle et adamantyle; ou est un radical alkyle en $C_1$—$C_6$ substitué, le substituant étant un groupe carboxy estérifié, pour donner un dérivé de 5-fluorouracile ayant la formule:

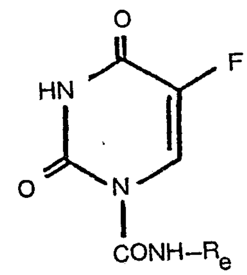

où $R_e$ est un radical alicyclique ponté contenant un groupe carboxy, le radical alicyclique ponté étant choisi parmi les groupes norbornyle, norbornényle, bicyclo[2.2.2]octyle, bicyclo[2.2.2]oct-2-ényle, bicyclo[3.1.1]heptyle et adamantyle; ou est un radical alkyle en $C_1$—$C_6$ substitué, le substituant étant un groupe carboxy.

2. Procédé selon la revendication 1, dans lequel R est un radical alicyclique ponté substitué ou non substitué, choisi parmi les groupes norbornyle, norbornényle, bicyclo[2.2.2]octyle, bicyclo[2.2.2]-oct-2-ényle, bicyclo[3.1.1.]heptyle et adamantyle, le ou les substituants de ce radical alicyclique ponté étant choisis parmi les groupes alkyle en $C_1$—$C_6$, carboxy, carboxy estérifié, alkylidène(en $C_1$—$C_6$)-dioxy, N,N-dialkyl(en $C_1$—$C_6$)carbamoyle, amino et amino protégé.

3. Procédé selon la revendication 2, dans lequel R est un radical alicyclique ponté choisi parmi les groupes norbornyle, norbornényle et adamantyle.

4. Procédé selon la revendication 3, dans lequel R est le groupe norbornyle.

5. Procédé selon la revendication 4, dans lequel R est le groupe 2-norbornyle.

6. Procédé selon la revendication 3, dans lequel R est le groupe adamantyle.

7. Procédé selon la revendication 6, dans lequel R est le groupe 1-adamantyle.

8. Procédé selon la revendication 2, dans lequel R est un radical alicyclique ponté choisi parmi les groupes norbornyle, norbornényle, bicyclo[2.2.2]octyle, bicyclo[2.2.2]oct-2-ényle, bicyclo[3.1.1]-heptyle et adamantyle, ce radical alicyclique ponté étant substitué par un substituant choisi parmi les groupes alkyle en $C_1$—$C_6$, carboxy, carboxy estérifié, alkylidène(en $C_1$—$C_6$)dioxy, N,N-dialkyl(en $C_1$—$C_6$)carbamoyle, amino et amino protégé.

9. Procédé selon la revendication 8, dans lequel R est le groupe carboxynorbornyle.

10. Procédé selon la revendication 9, dans lequel R est le groupe 3-carboxy-2-norbornyle.

11. Procédé selon la revendication 8, dans lequel R est un groupe carboxynorbornyle estérifié.

12. Procédé selon la revendication 11, dans lequel R est un groupe alcoxy(en $C_1$—$C_6$)carbonyl-norbornyle.

13. Procédé selon la revendication 12, dans lequel R est le groupe méthoxycarbonylnorbornyle.

14. Procédé selon la revendication 13, dans lequel R est le groupe 3-méthoxycarbonyl-2-norbornyle.

15. Procédé selon la revendication 12, dans lequel R est le groupe éthoxycarbonylnorbornyle.

16. Procédé selon la revendication 15, dans lequel R est le groupe 3-éthoxycarbonyl-2-norbornyle.

17. Procédé selon la revendication 12, dans lequel R est le groupe isopropoxycarbonyl-norbornyle.

18. Procédé selon la revendication 17, dans lequel R est le groupe 3-isopropoxycarbonyl-2-norbornyle.

19. Procédé selon la revendication 8, dans lequel R est le groupe norbornyle à substitution alcoxy-(en $C_1$—$C_6$)carbonyle et alkylidène(en $C_1$—$C_6$)dioxy.

20. Procédé selon la revendication 8, dans lequel R est un groupe N,N-dialkyl(en $C_1$—$C_6$)-carbamoylnorbornyle.

21. Procédé selon la revendication 8, dans lequel R est le groupe aminonorbornyle.

22. Procédé selon la revendication 8, dans lequel R est un groupe aminonorbornyle protégé.

23. Procédé selon la revendication 8, dans lequel R est le groupe carboxynorbornényle.

24. Procédé selon la revendication 8, dans lequel R est un groupe carboxynorbornényle estérifié.

25. Procédé selon la revendication 24, dans lequel R est un groupe alcoxy(en $C_1$—$C_6$)carbonylnorbornényle.

26. Procédé selon la revendication 8, dans lequel R est le groupe bicyclo[3.1.1]heptyle substitué par au moins un radical alkyle en $C_1$—$C_6$.

27. Procédé selon la revendication 26, dans lequel R est le groupe pinanyle.

28. Procédé selon la revendication 8, dans lequel R est un groupe carboxybicyclo[2.2.2]octyle estérifié.

29. Procédé selon la revendication 28, dans lequel R est un groupe alcoxy(en $C_1$—$C_6$)carbonylbicyclo[2.2.2]octyle.

30. Procédé selon la revendication 1, dans lequel R est un radical alkyle en $C_1$—$C_6$ substitué, le substituant étant un radical alicyclique ponté substitué ou non substitué, choisi parmi les groupes norbornyle et adamantyle, le substituant de ce radical alicyclique ponté étant choisi parmi les groupes alkyles en $C_1$—$C_6$ et les halogènes.

31. Procédé selon la revendication 30, dans lequel R est un radical alkyle en $C_1$—$C_6$ substitué, le substituant étant un radical alicyclique ponté choisi parmi les groupes norbornyle et adamantyle.

32. Procédé selon la revendication 31, dans lequel R est un radical norbornyl-alkyle en $C_1$—$C_6$.

33. Procédé selon la revendication 31, dans lequel R est un groupe adamantyl-alkyl en $C_1$—$C_6$.

34. Procédé selon la revendication 30, dans lequel R est un radical alkyle en $C_1$—$C_6$ substitué, le substituant étant un groupe adamantyle, qui est à substitution alkyle en $C_1$—$C_6$ ou halogénée.

35. Procédé selon la revendication 34, dans lequel R est un groupe alkyle (en $C_1$—$C_6$)adamantyl-alkyle en $C_1$—$C_6$.

36. Procédé selon la revendication 34, dans lequel R est un groupe haloadamantyl-alkyle en $C_1$—$C_6$.

37. Procédé selon la revendication 1, dans lequel R est un groupe aminoalkyle en $C_1$—$C_6$, amino protégé-akyle en $C_1$—$C_6$, alcoxy(en $C_1$—$C_6$)alkyle en $C_1$—$C_6$, acyl-alkyle en $C_1$—$C_6$, alkyl(en $C_1$—$C_6$)-thioalkyle en $C_1$—$C_6$, cyano-alkyl(en $C_1$—$C_6$)thioaklyle en $C_1$—$C_6$, phénylthio-alkyle en $C_1$—$C_6$, pyridyl-alkyle en $C_1$—$C_6$, thiényl-alkyle en $C_1$—$C_6$, 1,2-dithiolanyl-alkyle en $C_1$—$C_6$, 2,5-dioxopyrrolidinyl-alkyle en $C_1$—$C_6$, carboxy-alkyle en $C_3$—$C_6$ ou carboxy estérifié alkyle en $C_3$—$C_6$.

38. Procédé selon la revendication 1, dans lequel R est le groupe trans, trans-penta-1,3-diényle.

39. Procédé selon la revendication 1, dans lequel R est un groupe alkényle en $C_2$—$C_6$, à substitution norbornényle.

40. Procédé selon la revendication 1, dans lequel R est un groupe phényl-alkényle en $C_2$—$C_6$ à substitution alcoxy en $C_1$—$C_6$, furyl-aklényle en $C_2$—$C_6$ ou thiényl-alkényle en $C_2$—$C_6$.

41. Procédé selon la revendication 1, dans lequel est le groupe tétrahydrothiényle.